# EUROPÄISCHE PATENTANMELDUNG

(11) **EP 2 708 539 A1**
(43) Veröffentlichungstag der Anmeldung: **19.03.2014**
(21) Anmeldenummer: 13180879.2
(22) Anmeldetag: 05.07.2011
(51) Int. Cl.: C07D 487/04, A61K 31/519, A61K 31/522, A61P 9/00

(54) **ANNELLIERTE PYRIMIDINE UND TRIAZINE UND IHRE VERWENDUNG ZUR BEHANDLUNG BZW. PROPHYLAXE VON HERZ-KREISLAUF-ERKRANKUNGEN**

(30) Priorität: 09.07.2010 DE 102010031149; 28.01.2011 DE 102011003315
(62) Teilanmeldung aus: 11732409.5
(71) Anmelder: Bayer Intellectual Property GmbH, 40789 Monheim (DE)
(72) Erfinder: Follmann, Markus, 50859 Köln (DE); Stasch, Johannes-Peter, 42651 Solingen (DE); Redlich, Gorden, 44799 Bochum (DE); Ackerstaff, Jens, 40225 Düssseldorf (DE); Griebenow, Nils, 41541 Dormagen (DE); Knorr, Andreas, 40699 Erkart (DE); Wunder, Frank, 42117 Wuppertal (DE); Li, Volkhart, Min-Jian, 42553 Velbert (DE); Kroh, Walter, 42115 Wuppertal (DE); Bärfacker, Lars, 46047 Oberhausen (DE)
(74) Vertreter: BIP Patents

(57) **Zusammenfassung**

Die vorliegende Anmeldung betrifft neue annellierte Pyrimidine und Triazine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

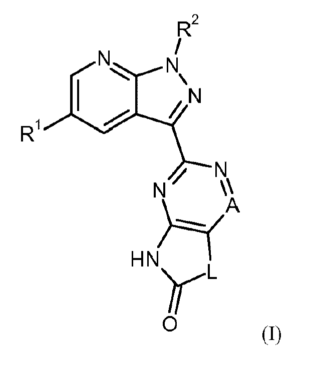

## Beschreibung

Die vorliegende Anmeldung betrifft neue annellierte Pyrimidine und Triazine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 03/095451 Carbamat-substitutierte 3-Pyrimidinyl-Pyrazolopyridine offenbart. WO 2010/065275 offenbart substituierte Pyrrolo- und Dihydropyridopyrimidine als sGC Aktivatoren.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften und/oder ihres pharmakokinetischem Verhaltens.

Gegenstand der vorliegenden Erfindung sind Verbindungen der allgemeinen Formel (I) 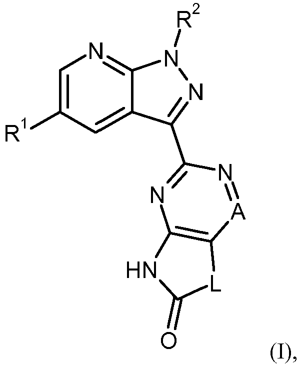 in welcher
- A: für Stickstoff oder CR³ steht,
wobei
R³ für Wasserstoff, Deuterium, Fluor, Difluormethyl, Trifluormethyl, (C₁-C₄)-Alkyl, Cyclopropyl oder Cyclobutyl steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0, 1 oder 2 steht,
R^{4A} für Wasserstoff, Fluor, (C₁-C₄)-Alkyl oder Hydroxy steht,
R^{4B} für Wasserstoff, Fluor, (C₁-C₄)-Alkyl, Trifluormethyl, (C₁-C₄)-Alkoxycarbonyl-amino oder Phenyl steht,
worin (C₁-C₄)-Alkyl mit 1 oder 2 Substituenten unabhängig voneinander ausgewählt aus der Gruppe Fluor, Trifluormethyl, Hydroxy, Hydroxycarbonyl und (C₁-C₄)-Alkoxycarbonyl substituiert sein kann,
oder
R^{4A} und R^{4B} zusammen mit dem Kohlenstoffatom, an das sie gebunden sind, eine Oxo-Gruppe, einen 3- bis 6-gliedrigen Carbocyclus oder einen 4- bis 6-gliedrigen Heterocyclus bilden,
worin der 3- bis 6-gliedrigen Carbocyclus und der 4- bis 6-gliedrigen Heterocyclus mit 1 oder 2 Substituenten unabhängig voneinander ausgewählt aus der Gruppe Fluor und (C₁-C₄)-Alkyl substituiert sein können,
oder
R^{4A} und R^{4B} zusammen mit dem Kohlenstoffatom, an das sie gebunden sind, eine (C₂-C₄)-Alkenyl-Gruppe bilden,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
- R¹: für Wasserstoff oder Fluor steht,
- R²: für Benzyl steht,
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl.
Carbocyclus steht im Rahmen der Erfindung für einen monocyclischen, gesättigten Carbocyclus mit 3 bis 6 Ring-Kohlenstoffatomen. Beispielhaft und vorzugsweise seien genannt: Cyclopropyl, Cyclobutyl, Cyclopentyl und Cyclohexyl.
Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkoxycarbonyl steht im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Alkoxycarbonylamino steht im Rahmen der Erfindung für eine Amino-Gruppe mit einem linearen oder verzweigten Alkoxycarbonyl-Substituenten, der 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.
Heterocyclus steht im Rahmen der Erfindung für einen gesättigten Heterocyclus mit insgesamt 4 bis 6 Ringatomen, der ein oder zwei Ring-Heteroatome aus der Reihe N, O, S, SO und/oder SO₂ enthält und über ein Ring-Kohlenstoffatom verknüpft ist. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl und Tetrahydropyranyl.
Halogen steht im Rahmen der Erfindung für Fluor, Chlor, Brom und Iod. Bevorzugt sind Brom und Iod.

In der Formel der Gruppe, für die L bzw. R² stehen kann, steht der Endpunkt der Linie, an dem das Zeichen *, # und ## steht, nicht für ein Kohlenstoffatom beziehungsweise eine CH₂-Gruppe, sondern ist Bestandteil der Bindung zu dem jeweils bezeichneten Atom, an das L bzw. R² gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bevorzugt sind im Rahmen der vorliegenden Erfindung Verbindungen der Formel (I), in welcher
- A: für Stickstoff oder CR³ steht,
wobei
R³ für Wasserstoff, Deuterium, Fluor, Difluormethyl, Trifluormethyl, (C₁-C₄)-Alkyl, Cyclopropyl oder Cyclobutyl steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0, 1 oder 2 steht,
R^{4A} für Wasserstoff, Fluor, (C₁-C₄)-Alkyl, Hydroxy steht,
R^{4B} für Wasserstoff, Fluor, (C₁-C₄)-Alkyl oder Trifluormethyl steht,
oder
R^{4A} und R^{4B} zusammen mit dem Kohlenstoffatom, an das sie gebunden sind, eine Oxo-Gruppe, einen 3- bis 6-gliedrigen Carbocyclus oder einen 4- bis 6-gliedrigen Heterocyclus bilden,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
- R¹: für Wasserstoff oder Fluor steht,
- R²: für Benzyl steht,
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- A: für Stickstoff oder CR³ steht,
wobei
R³ für Wasserstoff, Fluor, Difluormethyl, Trifluormethyl, Methyl, Ethyl oder Cyclopropyl steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 oder 1 steht,
R^{4A} für Wasserstoff, Fluor, Methyl, Ethyl oder Hydroxy steht,
R^{4B} für Wasserstoff, Fluor, Methyl, Ethyl, Trifluormethyl, Methoxycarbonylamino oder Phenyl steht,
worin Methyl und Ethyl mit 1 oder 2 Substituenten unabhängig voneinander ausgewählt aus der Gruppe Fluor, Trifluormethyl und Hydroxy substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
worin der Cyclopropyl-, Cyclobutyl-, Cyclopentyl-, Azetidinyl-, Pyrrolidinyl-, Tetrahydrofuranyl-, Piperidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für Benzyl steht,
wobei Benzyl mit 1 bis 3 Substituenten Fluor substituiert ist,
sowie ihre Salze, Solvate und Solvate der Salze.

Bevorzugt sind im Rahmen der vorliegenden Erfindung auch Verbindungen der Formel (I), in welcher
- A: für Stickstoff oder CR³ steht,
wobei
R³ für Wasserstoff, Difluormethyl, Trifluormethyl, Methyl, Ethyl oder Cyclopropyl steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0, 1 oder 2 steht,
R^{4A} für Wasserstoff, Fluor, Methyl, Ethyl oder Hydroxy steht,
R^{4B} für Wasserstoff, Fluor, Methyl, Ethyl oder Trifluormethyl steht,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für Benzyl steht, wobei Benzyl mit 1 oder 2 Substituenten Fluor substituiert ist,
sowie ihre Salze, Solvate und Solvate der Salze.

Besonders bevorzugt sind im Rahmen der vorliegenden Erfindung auch Verbindungen der Formel (I), in welcher
- A: für CR³ steht,
wobei
R³ für Wasserstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
- R¹: für Wasserstoff oder Fluor steht,
- R²: für Benzyl steht,
wobei Benzyl mit 1 oder 2 Substituenten Fluor substituiert ist,
sowie ihre Salze, Solvate und Solvate der Salze.

Besonders bevorzugt sind im Rahmen der vorliegenden Erfindung auch Verbindungen der Formel (I), in welcher
- A: für Stickstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0 steht,
R^{4A} für Wasserstoff, Fluor oder Methyl steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
- R¹: für Wasserstoff oder Fluor steht,
- R²: für Benzyl steht,
wobei Benzyl mit 1 oder 2 Substituenten Fluor substituiert ist,
sowie ihre Salze, Solvate und Solvate der Salze.

Besonders bevorzugt sind im Rahmen der vorliegenden Erfindung auch Verbindungen der Formel (I), in welcher
- A: für Stickstoff oder CR³ steht,
wobei
R³ für Wasserstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropylring bilden,
- R¹: für Wasserstoff oder Fluor steht,
- R²: für eine Gruppe der Formel 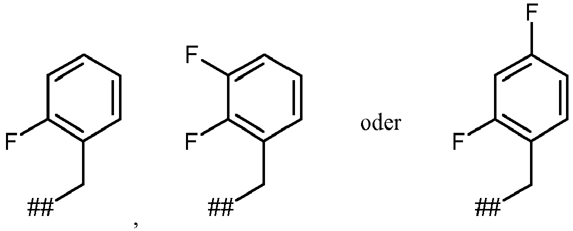
steht, wobei
## für die Anknüpfstelle an den Pyrazolopyridin-Ring steht,
sowie ihre Salze, Solvate und Solvate der Salze.

Bevorzugt sind im Rahmen der vorliegenden Erfindung auch Verbindungen der Formel (I), in welcher R¹ für H steht, sowie ihre Salze, Solvate und Solvate der Salze.

Bevorzugt sind im Rahmen der vorliegenden Erfindung auch Verbindungen der Formel (I), in welcher R¹ für Fluor steht, sowie ihre Salze, Solvate und Solvate der Salze.

Bevorzugt sind im Rahmen der vorliegenden Erfindung auch Verbindungen der Formel (I), in welcher A für N oder CH steht, sowie ihre Salze, Solvate und Solvate der Salze.

Bevorzugt sind im Rahmen der vorliegenden Erfindung auch Verbindungen der Formel (I), in welcher A für CH steht, sowie ihre Salze, Solvate und Solvate der Salze.

Bevorzugt sind im Rahmen der vorliegenden Erfindung auch Verbindungen der Formel (I), in welcher A für N steht, sowie ihre Salze, Solvate und Solvate der Salze.

Bevorzugt sind im Rahmen der vorliegenden Erfindung auch Verbindungen der Formel (I), in welcher
- L: für eine Gruppe *-CR^{4A}R^{4B}-(CR^{5A}R^{5B})ₚ-# steht
wobei
* für die Anknüpfstelle an die Carbonylgruppe steht,
# für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
sowie ihre Salze, Solvate und Solvate der Salze.

Bevorzugt sind im Rahmen der vorliegenden Erfindung auch Verbindungen der Formel (I), in welcher
- L: für eine Gruppe *-CR^{4A}R^{4B}-(CR^{5A}R^{5B})ₚ-# steht
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
sowie ihre Salze, Solvate und Solvate der Salze.

Bevorzugt sind im Rahmen der vorliegenden Erfindung auch Verbindungen der Formel (I), in welcher
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Methyl steht,
R^{4B} für Methyl steht,
sowie ihre Salze, Solvate und Solvate der Salze.

Bevorzugt sind im Rahmen der vorliegenden Erfindung auch Verbindungen der Formel (I), in welcher
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
sowie ihre Salze, Solvate und Solvate der Salze.

Bevorzugt sind im Rahmen der vorliegenden Erfindung auch Verbindungen der Formel (I), in welcher
- R²: für Benzyl steht,
wobei Benzyl mit 1 oder 2 Substituenten Fluor substituiert ist,
sowie ihre Salze, Solvate und Solvate der Salze.

Bevorzugt sind im Rahmen der vorliegenden Erfindung auch Verbindungen der Formel (I), in welcher
- R²: für eine Gruppe der Formel 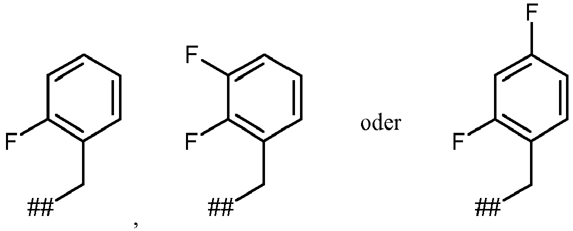 steht, wobei
## für die Anknüpfstelle an den Pyrazolopyridin-Ring steht,
sowie ihre Salze, Solvate und Solvate der Salze.

Weiterer Gegenstand der vorliegenden Erfindung sind Verbindungen der Formel (XV), 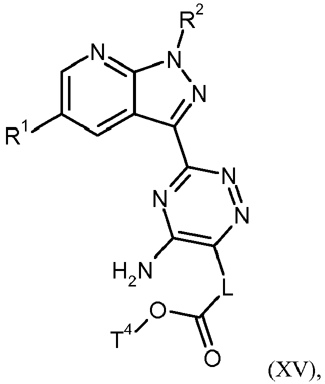 in welcher
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0, 1 oder 2 steht,
R^{4A} für Wasserstoff, Fluor, (C₁-C₄)-Alkyl oder Hydroxy steht,
R^{4B} für Wasserstoff, Fluor, (C₁-C₄)-Alkyl, Trifluormethyl, (C₁-C₄)-Alkoxycarbonylamino oder Phenyl steht,
worin (C₁-C₄)-Alkyl mit 1 oder 2 Substituenten unabhängig voneinander ausgewählt aus der Gruppe Fluor, Trifluormethyl, Hydroxy, Hydroxycarbonyl und (C₁-C₄)-Alkoxycarbonyl substituiert sein kann,
oder
R^{4A} und R^{4B} zusammen mit dem Kohlenstoffatom, an das sie gebunden sind, einen 3- bis 6-gliedrigen Carbocyclus oder einen 4- bis 6-gliedrigen Heterocyclus bilden,
worin der 3- bis 6-gliedrigen Carbocyclus und der 4- bis 6-gliedrigen Heterocyclus mit 1 oder 2 Substituenten unabhängig voneinander ausgewählt aus der Gruppe Fluor und (C₁-C₄)-Alkyl substituiert sein können,
R^{5A} für Wasserstoff, Fluor, (C₁-C₄)-Alkyl oder Hydroxy steht,
- R^{5B}: für Wasserstoff, Fluor, (C₁-C₄)-Alkyl oder Trifluormethyl steht,
- R¹: für Wasserstoff oder Fluor steht,
- R²: für Benzyl steht,
wobei Benzyl mit 1 bis 3 Substituenten Fluor substituiert ist,
- T⁴: für (C₁-C₄)-Alkyl steht,
sowie ihre Salze, Solvate und Solvate der Salze.

Weiterer Gegenstand der vorliegenden Erfindung sind Verbindungen der Formel (XIII), 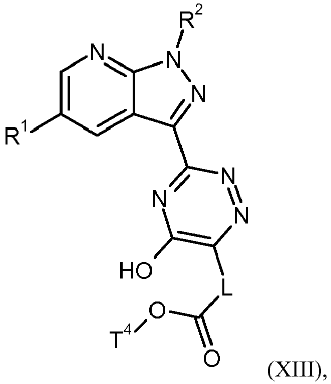 in welcher
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0, 1 oder 2 steht,
R^{4A} für Wasserstoff, Fluor, (C₁-C₄)-Alkyl oder Hydroxy steht,
R^{4B} für Wasserstoff, Fluor, (C₁-C₄)-Alkyl, Trifluormethyl, (C₁-C₄)-Alkoxycarbonylamino oder Phenyl steht,
worin (C₁-C₄)-Alkyl mit 1 oder 2 Substituenten unabhängig voneinander ausgewählt aus der Gruppe Fluor, Trifluormethyl, Hydroxy, Hydroxycarbonyl und (C₁-C₄)-Alkoxycarbonyl substituiert sein kann,
oder
R^{4A} und R^{4B} zusammen mit dem Kohlenstoffatom, an das sie gebunden sind, einen 3- bis 6-gliedrigen Carbocyclus oder einen 4- bis 6-gliedrigen Heterocyclus bilden,
worin der 3- bis 6-gliedrigen Carbocyclus und der 4- bis 6-gliedrigen Heterocyclus mit 1 oder 2 Substituenten unabhängig voneinander ausgewählt aus der Gruppe Fluor und (C₁-C₄)-Alkyl substituiert sein können,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
- R¹: für Wasserstoff oder Fluor steht,
- R²: für Benzyl steht,
wobei Benzyl mit 1 bis 3 Substituenten Fluor substituiert ist,
- T⁴: für (C₁-C₄)-Alkyl steht,
sowie ihre Salze, Solvate und Solvate der Salze.

Bevorzugt sind im Rahmen der vorliegenden Erfindung auch Verbindungen der Formeln (XII) und (XV), in welchen
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0, 1 oder 2 steht,
R^{4A} für Wasserstoff, Fluor, Methyl, Ethyl oder Hydroxy steht,
R^{4B} für Wasserstoff, Fluor, Methyl, Ethyl oder Trifluormethyl steht,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für Benzyl steht,
wobei Benzyl mit 1 oder 2 Substituenten Fluor substituiert ist,
- T⁴: für Methyl oder Ethyl steht,
sowie ihre Salze, Solvate und Solvate der Salze.

Bevorzugt sind im Rahmen der vorliegenden Erfindung auch Verbindungen der Formeln (XIII) und (XV), in welchen
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0 steht,
R^{4A} für Methyl steht,
R^{4B} für Methyl steht,
sowie ihre Salze, Solvate und Solvate der Salze.

Bevorzugt sind im Rahmen der vorliegenden Erfindung auch Verbindungen der Formeln (XIII) und (XV), in welchen
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0 steht,
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man eine Verbindung der Formel (II) 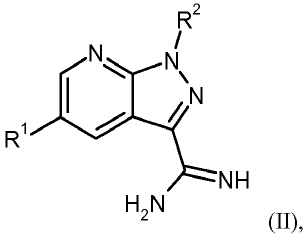 in welcher R¹ und R² jeweils die oben genannten Bedeutungen haben,
[A] in einem inerten Lösungsmittel in Gegenwart einer geeigneten Base mit einer Verbindung der Formel (III) 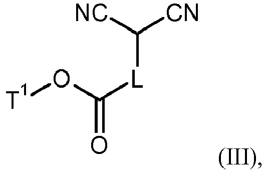 in welcher L die oben genannte Bedeutung hat und
   T¹ für (C₁-C₄)-Alkyl steht,
   zu einer Verbindung der Formel (IV) 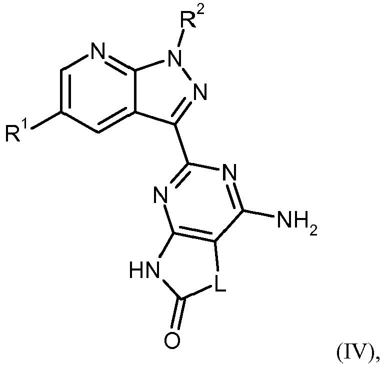 in welcher L, R¹ und R² jeweils die oben angegebenen Bedeutungen haben,
   umsetzt, diese dann mit iso-Pentylnitrit und einem Iod-Äquivalent in eine Verbindung der Formel (V) 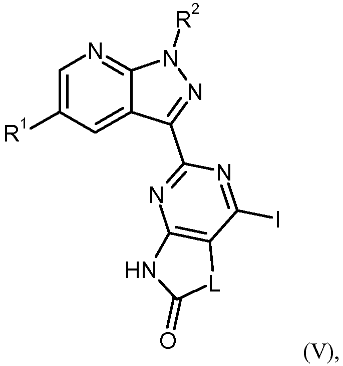 in welcher L, R¹ und R² jeweils die oben angegebenen Bedeutungen haben, überführt, und diese im Anschluss in einen inerten Lösungsmittel in Gegenwart eines geeigneten Übergangsmetallkatalysators zu einer Verbindung der Formel (I-A) 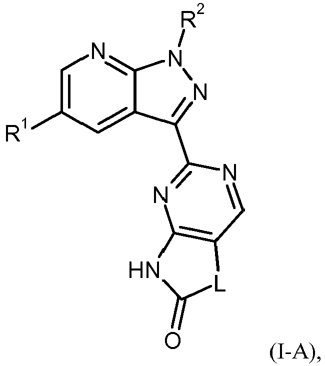 in welcher L, R¹ und R² jeweils die oben angegebenen Bedeutungen haben,
   umsetzt,
   oder
[B] in einem inerten Lösungsmittel in Gegenwart einer geeigneten Base mit einer Verbindung der Formel (VI) 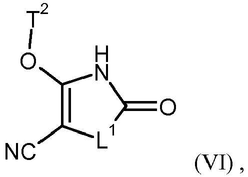 in welcher
   - L¹: für eine Gruppe *-CR^{4A}R^{4B}-(CR^{5A}R^{5B})ₚ-# steht,
   wobei
   * für die Anknüpfstelle an die Carbonylgruppe steht,
   # für die Anknüpfstelle an den Pyrimidin- bzw. Triazinring steht,
   p für eine Zahl 1 oder 2 steht,
   R^{4A} für Wasserstoff, Fluor, (C₁-C₄)-Alkyl oder Hydroxy steht,
   R^{4B} für Wasserstoff, Fluor, (C₁-C₄)-Alkyl oder Trifluormethyl steht,
   oder
   R^{4A} und R^{4B} zusammen mit dem Kohlenstoffatom, an das sie gebunden sind, eine Oxo-Gruppe, einen 3- bis 6-gliedrigen Carbocyclus oder einen 4-bis 6-gliedrigen Heterocyclus bilden,
   R^{5A} für Wasserstoff, Fluor, (C₁-C₄)-Alkyl oder Hydroxy steht,
   R^{5B} für Wasserstoff, Fluor, (C₁-C₄)-Alkyl oder Trifluormethyl steht, und
   - T²: für (C₁-C₄)-Alkyl steht,
   zu einer Verbindung der Formel (IV-B) 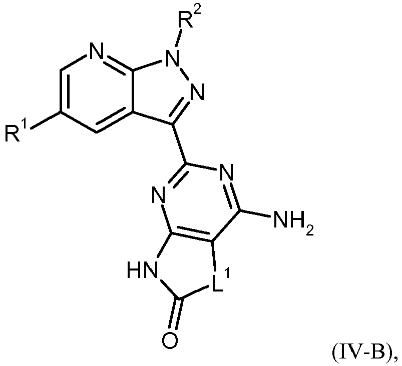 in welcher L¹, R¹ und R² jeweils die oben angegebenen Bedeutungen haben,
   umsetzt, und diese dann analog zum Verfahren [A] weiter zu einer Verbindung der Formel (I-B) 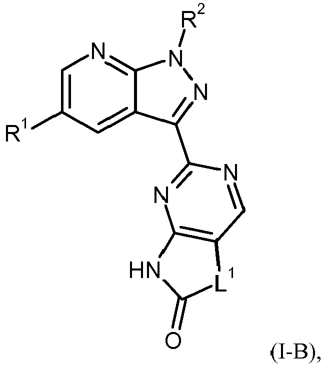 in welcher L¹, R¹ und R² jeweils die oben angegebenen Bedeutungen haben, umsetzt,
   oder
[C] in einem inerten Lösungsmittel in Gegenwart einer geeigneten Base mit einer Verbindung der Formel (VII) 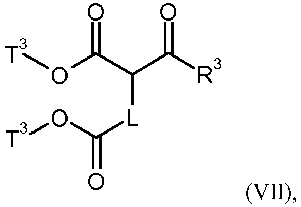 in welcher L und R³ jeweils die oben angegebenen Bedeutungen haben und
   - T³: für (C₁-C₄)-Alkyl steht,
   zu einer Verbindung der Formel (VIII) 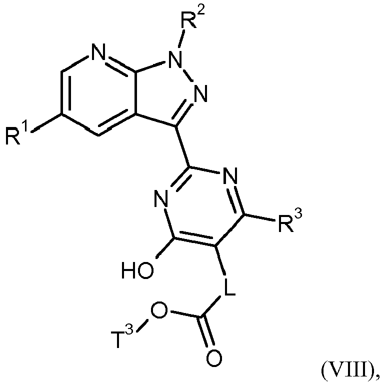 in welcher L, R¹, R², R³ und T³ jeweils die oben angegebenen Bedeutungen haben,
   umsetzt, diese anschliessend mit Phosphorylchlorid in eine Verbindung der Formel (IX) 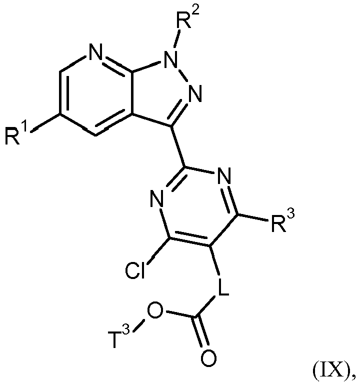 in welcher L, R¹, R², R³ und T³ jeweils die oben angegebenen Bedeutungen haben,
   überführt, diese im folgenden in einem inerten Lösungsmittel in eine entsprechende Azid-Verbindung überführt und diese direkt zu einer Verbindung der Formel (X) 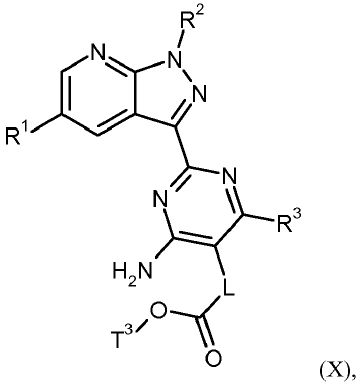 in welcher L, R¹, R², R³ und T³ jeweils die oben angegebenen Bedeutungen haben,
   reduziert, und diese dann in einem inerten Lösungsmittel in Gegenwart einer geeigneten Base zu einer Verbindung der Formel (I-C) 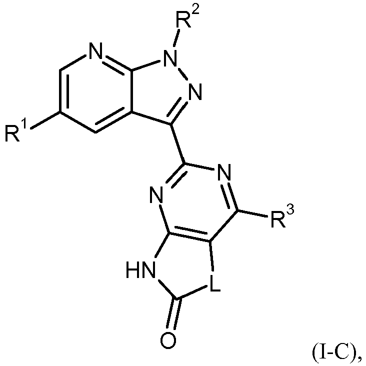 in welcher L, R¹, R² und R³ jeweils die oben angegebenen Bedeutungen haben,
   umsetzt,
   oder
[D] in einem inerten Lösungsmittel in Gegenwart einer geeigneten Base mit Hydrazinhydrat zu einer Verbindung der Formel (XI) 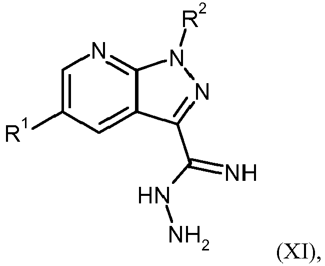 in welcher R¹ und R² jeweils die oben genannten Bedeutungen haben,
   umsetzt, diese dann in einem inerten Lösungsmittel mit einer Verbindung der Formel (XII) 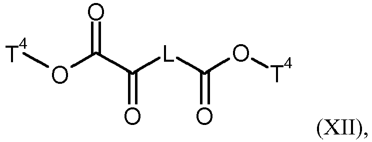 in welcher L die oben angegebene Bedeutung hat und
   T⁴ für (C₁-C₄)-Alkyl steht,
   zu einer Verbindung der Formel (XIII) 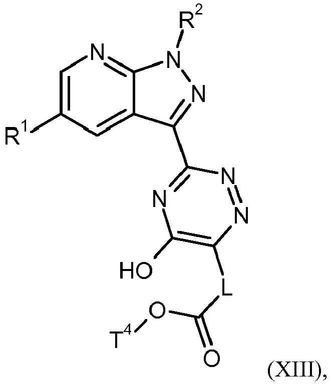 in welcher L, R¹, R² und T⁴ jeweils die oben angegebenen Bedeutungen haben,
   reagiert, diese anschliessend mit Phosphorylchlorid in eine Verbindung der Formel (XIV) 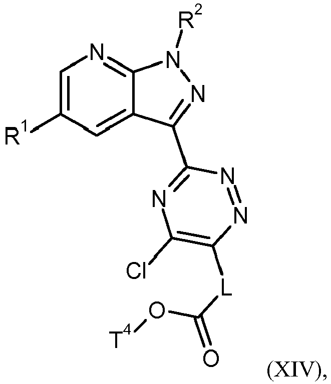 in welcher L, R¹, R² und T⁴ jeweils die oben angegebenen Bedeutungen haben,
   überführt, und diese direkt mit Ammoniak zu einer Verbindung der Formel (XV) 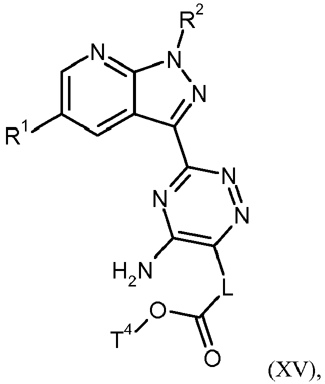 in welcher L, R¹, R² und T⁴ jeweils die oben angegebenen Bedeutungen haben,
   umsetzt, und schliesslich in einem inerten Lösungsmittel in Gegenwart einer geeigneten Base zu einer Verbindung der Formel (I-D) 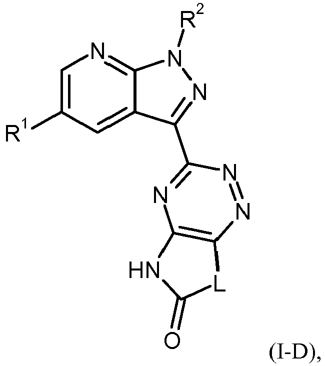 in welcher L, R¹ und R² jeweils die oben angegebenen Bedeutungen haben,
   cyclisiert,
und gegebenenfalls die resultierenden Verbindungen der Formeln (I-A), (I-B), (I-C) und (I-D) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A), (I-B), (I-C) und (I-D) bilden zusammen die Gruppe der erfindungsgemäßen Verbindungen der Formel (I).

Inerte Lösungsmittel für den Verfahrensschritt (II) + (III) bzw. (VI) → (IV) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol oder Methanol.

Geeignete Basen für den Verfahrensschritt (II) + (III) bzw. (VI) → (IV) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat oder Natriummethanolat.

Die Reaktion (II) + (III) bzw. (VI) → (IV) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (IV) → (V) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (IV) → (V) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (IV) → (V) erfolgt im Allgemeinen mit einem Molverhältnis von 10 bis 30 Mol Isopentylnitrit und 10 bis 30 Mol des Iod-Äquivalents bezogen auf 1 Mol der Verbindung der Formel (IV).

Als Iod-Quelle bei der Umsetzung (IV) → (V) eignen sich beispielsweise Diiodmethan oder eine Mischung aus Cäsiumiodid, Iod und Kupfer-(I)-iodid.

Inerte Lösungsmittel für den Verfahrensschritt (V) → (I-A) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion (V) → (I-A) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (V) → (I-A) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (II) + (VII) → (VIII) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Geeignete Basen für den Verfahrensschritt (II) + (VII) → (VIII) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Natriummethanolat oder Natriumethanolat.

Die Reaktion (II) + (VII) → (VIII) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzungen (VIII) → (IX) und (XIII) → (XIV) können in einem unter den Reaktionsbedingungen inerten Lösungsmittel oder ohne Lösungsmittel erfolgen. Bevorzugtes Lösungsmittel ist Sulfolan.

Die Reaktionen (VIII) → (IX) und (XIII) → (XIV) erfolgen im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (XIII) → (XIV) ohne Lösungsmittel in einem Temperaturbereich von 0°C bis +50°C bei Normaldruck.

Der Verfahrensschritt (IX) → (X) erfolgt durch Reaktion mit Natriumazid unter intermediärer Bildung der Azid-Derivate, die direkt weiter zu den entsprechenden Aminen reduziert werden. Inerte Lösungsmittel für die Azidbildung sind beispielsweise Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder Sulfolan. Ebenso ist es möglich, Gemische der genannten Lösungsmittel einzusetzen. Bevorzugt ist DMF.

Die Azidbildung erfolgt erfolgt im Allgemeinen in einem Temperaturbereich von +50°C bis +100°C, bevorzugt von +60°C bis +80°C, bei Normaldruck.

Die Reduktion erfolgt in einem inerten Lösungsmittel wie beispielsweise Alkoholen wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion erfolgt bei +10°C bis +30°C mit Wasserstoff in Verbindung mit Übergangsmetallkatalysatoren wie beispielsweise Palladium (10% auf Aktivkohle), Platindioxid oder Palladiumhydroxid, oder ohne Wasserstoff mit Zinn(II)chlorid und Salzsäure.

Alternativ kann die Umsetzung (IX) → (X) auch in einer Stufe analog zum Verfahrensschritt (XIV) → (XV) durchgeführt werden.

Der Verfahrensschritt (XIV) → (XV) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Die Reaktion (XIV) → (XV) erfolgt im Allgemeinen in einem Temperaturbereich von +20°C bis +100°C, bevorzugt von +40°C bis +70°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Cyclisierungen (X) → (I-C) und (XV) → (I-D) erfolgen in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran (THF), Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethyl-propylenharnstoff (DMPU), *N*-Methylpyrrolidon (NMP), Pyridin, Acetonitril oder Sulfolan. Ebenso ist es möglich, Gemische der genannten Lösungsmittel einzusetzen. Bevorzugt ist THF.

Geeignete Basen für die Verfahrensschritte (X) → (I-C) und (XV) → (I-D) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktionen (X) → (I-C) und (XV) → (I-D) erfolgen im Allgemeinen in einem Temperaturbereich von 0°C bis +50°C, bevorzugt von +10°C bis +30°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Alternativ erfolgt die Cyclisierung zu (I-C) bzw. (I-D) direkt bei der Reduktion des Azids zum entsprechenden Amin (X) bzw. bei der Umsetzung (XIV) → (XV) ohne Zugabe weiterer Reagentien.

In einer alternativen Durchführung der Verfahren [C] bzw. [D] werden die Umwandlungen (IX) → (X) → (I-C) bzw. (XI) + (XII) → (XIII) → (XIV) → (XV) → (I-D) simultan in einer Eintopfreaktion ohne Isolierung der Zwischenstufen durchgeführt.

Bevorzugt erfolgen die Umsetzungen (XIII) → (XIV) → (XV) → (I-D) simultan in einer Eintopfreaktion ohne Isolierung der Zwischenstufen.

Inerte Lösungsmittel für den Verfahrensschritt (XI) + (XII) → (XIII) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Die Reaktion (XI) + (XII) → (XIII) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (II) → (XI) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Ethanol.

Geeignete Basen für den Verfahrensschritt (II) → (XI) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt Triethylamin.

Die Reaktion (II) → (XI) erfolgt im Allgemeinen in einem Temperaturbereich von 0°C bis +60°C, bevorzugt von +10°C bis +30°C. Die Umsetzung kann bei normalem oder erhöhtem Druck durchgeführt werden (z.B. im Bereich von 0.5 bis 5 bar). Im Allgemeinen arbeitet man bei Normaldruck.

Die beschriebenen Herstellverfahren können durch die folgenden Syntheseschemata (Schema 1 bis 4) beispielhaft verdeutlicht werden:

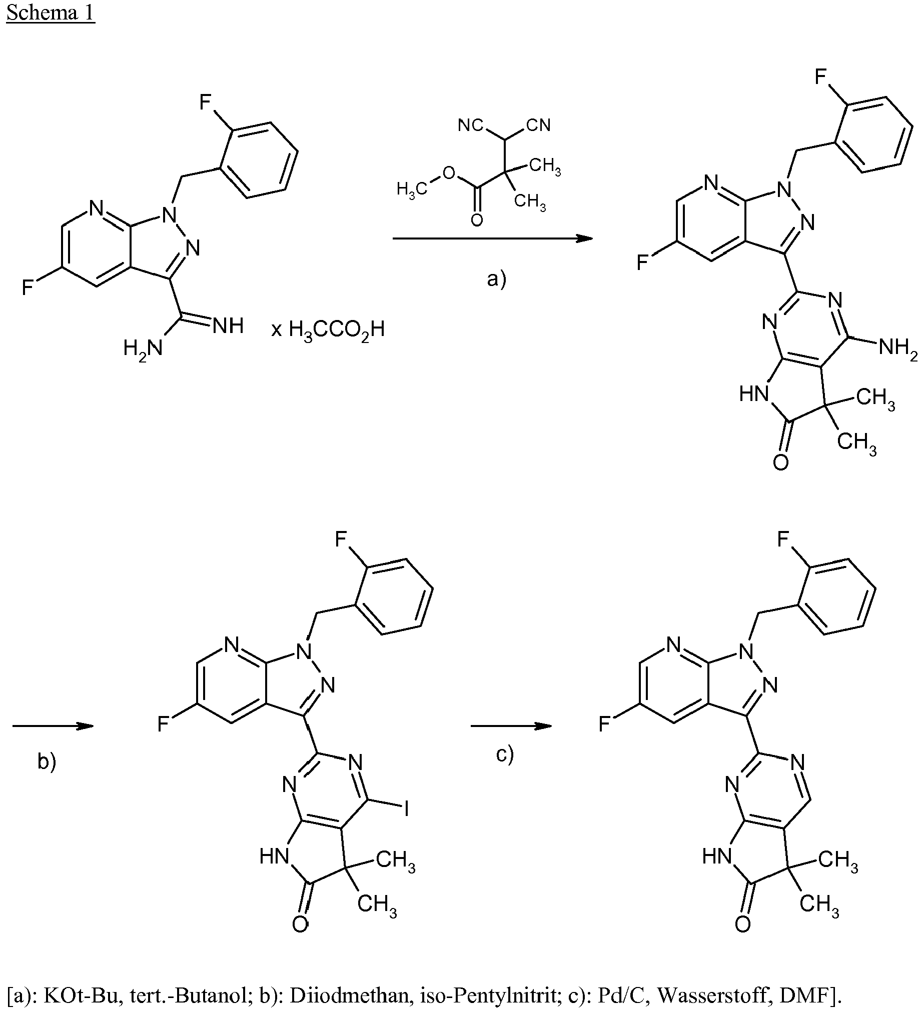

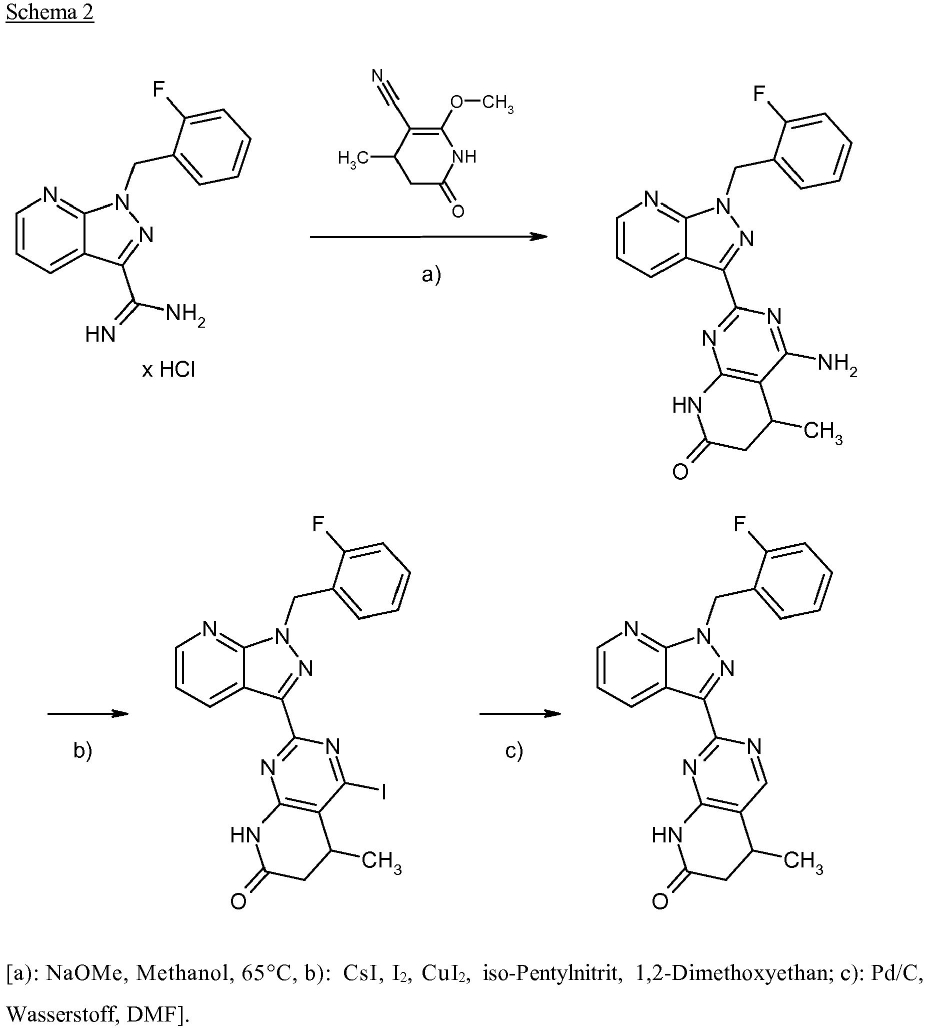

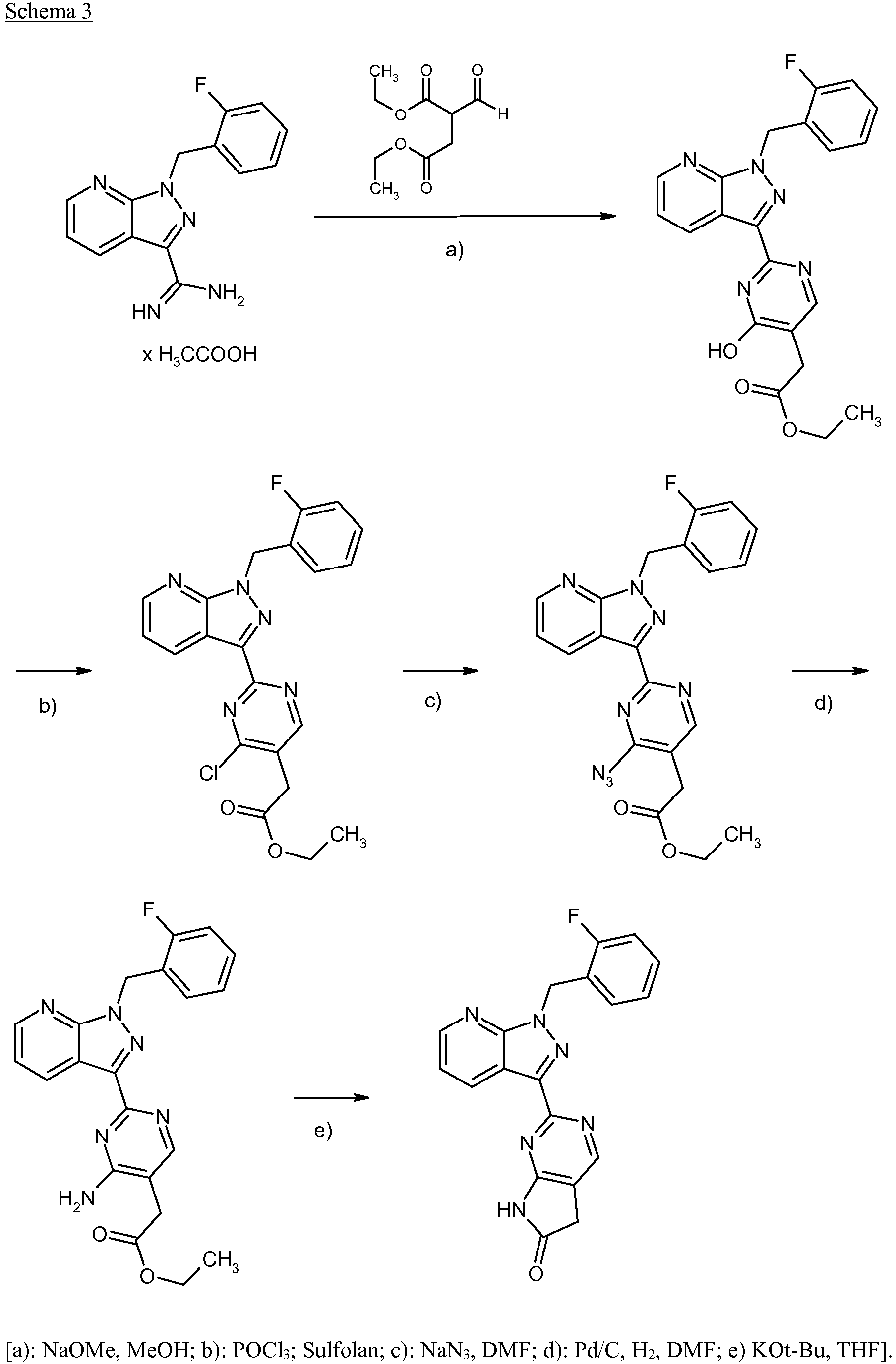

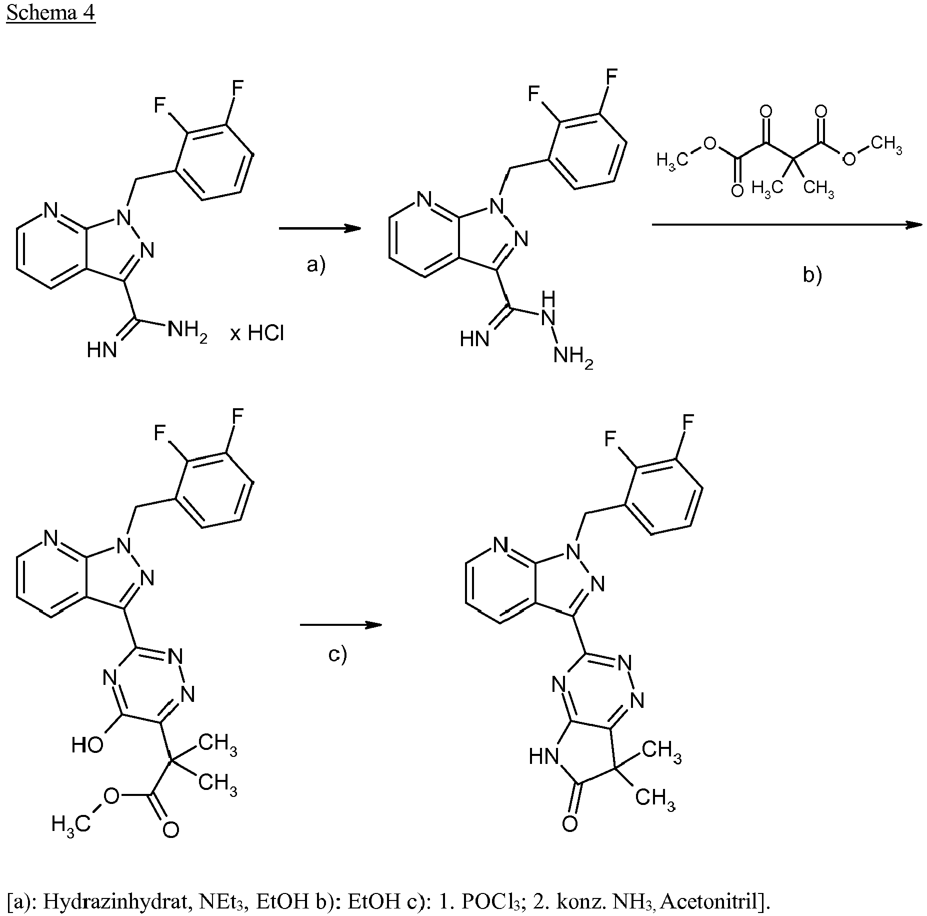

Weitere erfindungsgemäße Verbindungen können gegebenenfalls auch hergestellt werden durch Umwandlungen von funktionellen Gruppen einzelner Substituenten, insbesondere den unter L und R³ aufgeführten, ausgehend von den nach obigen Verfahren erhaltenen Verbindungen der Formel (I), (IX) bzw. (XIV). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen. Anhand der folgenden Syntheseschemata (Schema 5, 6, 11 und 12) werden bevorzugte Umwandlungen beispielhaft verdeutlicht:

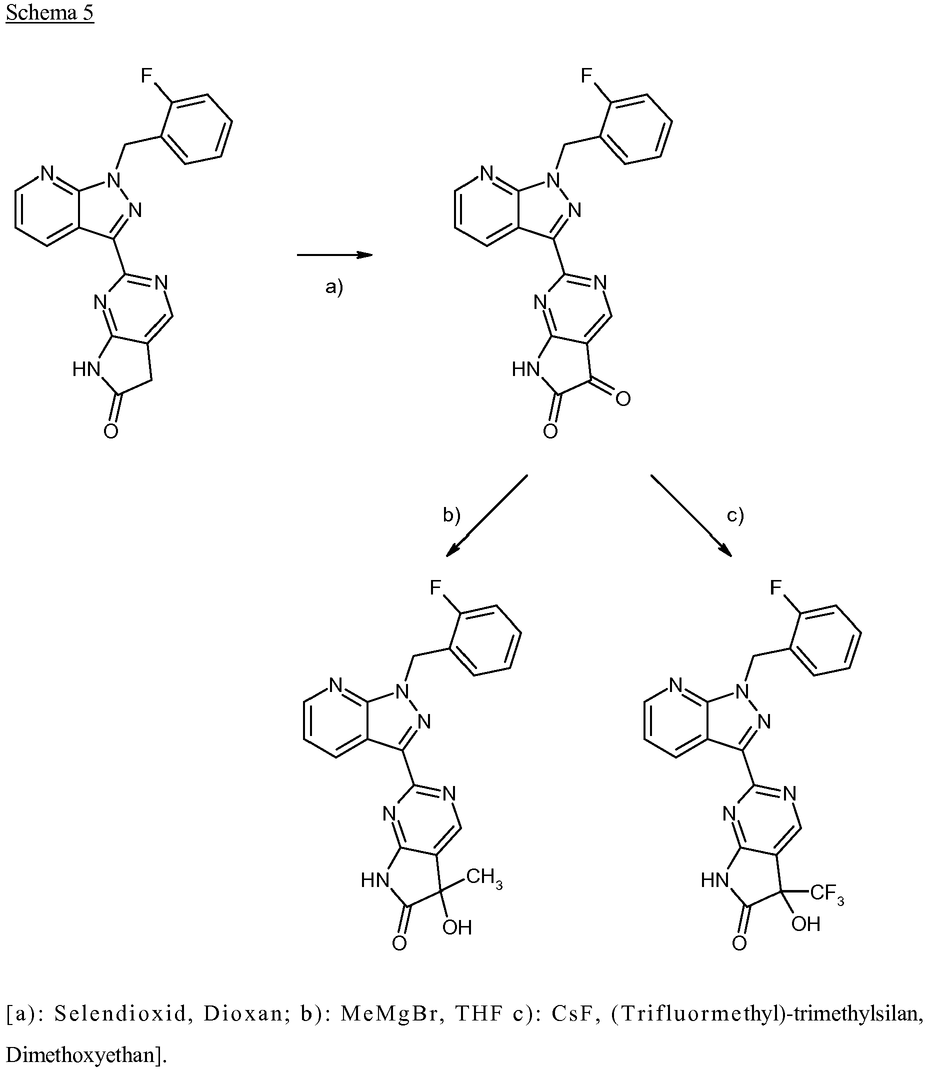

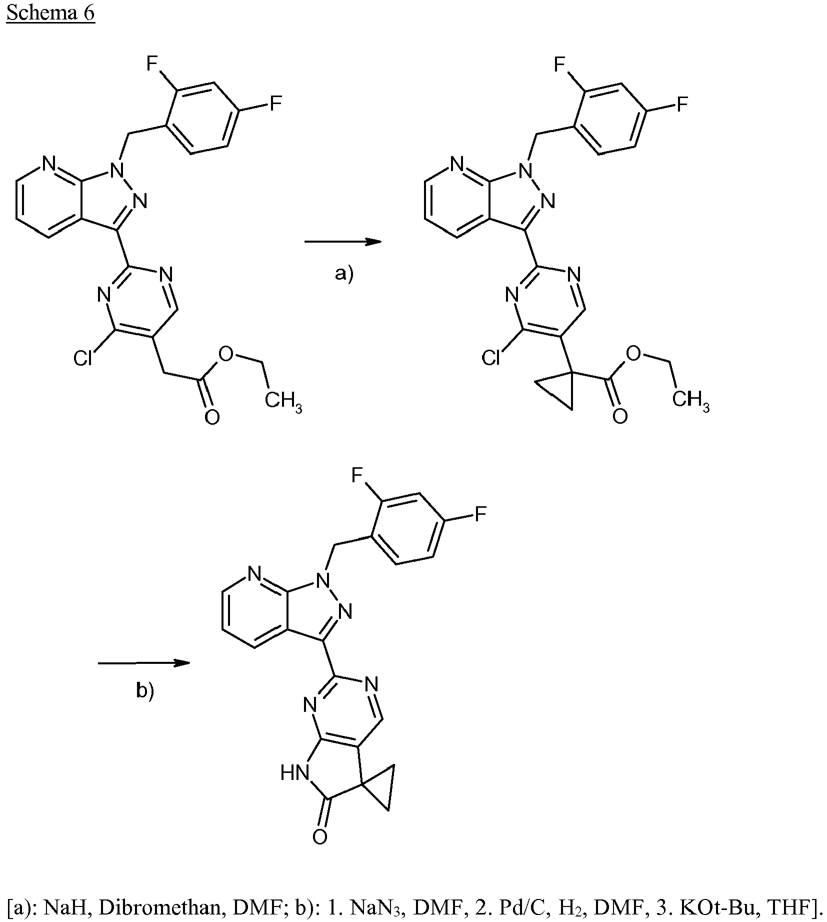

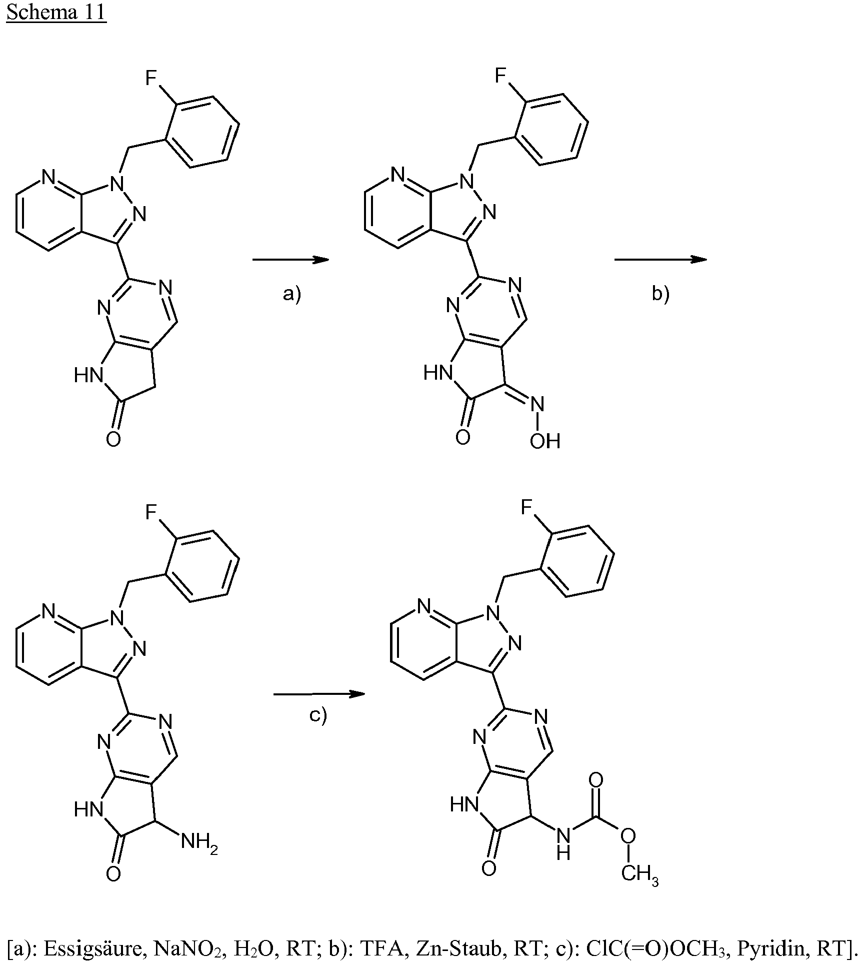

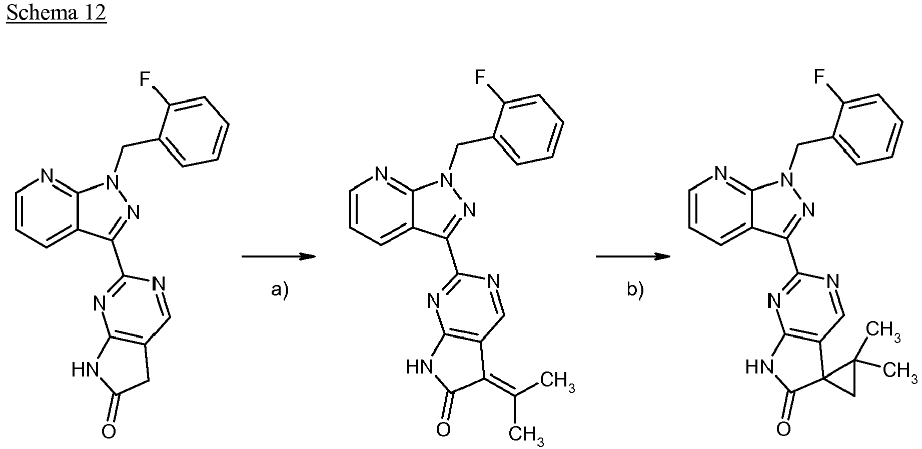

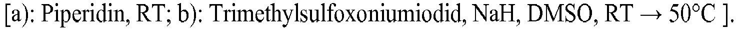

Die Verbindungen der Formel (II) sind literaturbekannt (siehe z.B. WO 03/095451, Beispiel 6A) oder können hergestellt werden, indem man eine Verbindung der Formel (XVI) 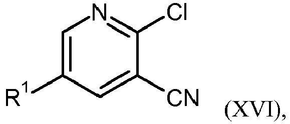 in welcher R¹ die oben angegebene Bedeutung hat,
in einem inerten Lösungsmittel mit Hydrazinhydrat zur Verbindung der Formel (XVII) 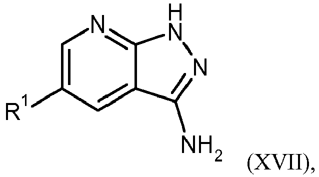 in welcher R¹ die oben angegebene Bedeutung hat,
zyklisiert, diese anschliessend in einem inerten Lösungsmittel in Gegenwart einer geeigneten LewisSäure zunächst mit Isopentylnitrit zum entsprechenden Diazoniumsalz umsetzt, und dieses dann direkt mit Natriumiodid in die Verbindung der Formel (XVIII) 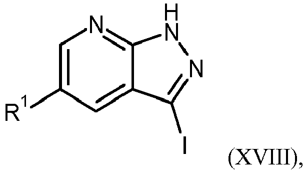 in welcher R¹ die oben angegebene Bedeutung hat,
überführt, diese im Folgenden in einem inerten Lösungsmittel in Gegenwart einer geeigneten Base mit der Verbindung der Formel (XIX)

R²-X¹ (XIX),

in welcher R² die oben angegebene Bedeutung hat und
X¹ für eine geeignete Abgangsgruppe, wie beispielsweise Halogen, Tosylat oder Mesylat, steht,
in eine Verbindung der Formel (XX) 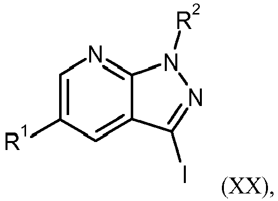 in welcher R¹ und R² jeweils die oben angegebenen Bedeutungen haben,
überführt, diese anschließend in einem inerten Lösungsmittel mit Kupfercyanid zu einer Verbindung der Formel (XXI) 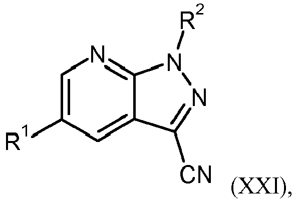 in welcher R¹ und R² jeweils die oben angegebenen Bedeutungen haben,
reagiert, und diese schließlich unter sauren Bedingungen mit einem Ammoniak-Äquivalent umsetzt.

Inerte Lösungsmittel für den Verfahrensschritt (XVI) → (XVII) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylen-harnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist 1,2-Ethandiol.

Die Reaktion (XVI) → (XVII) wird im Allgemeinen in einem Temperaturbereich von +60°C bis +200°C, bevorzugt bei +120°C bis +180°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XVII) → (XVIII) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Bevorzugt ist DMF.

Als Lewis-Säuren für den Verfahrensschritt (XVII) → (XVIII) eignen sich Bortrifluorid-Diethylether-Komplex, Cer(IV)ammoniumnitrat (CAN), Zinn(II)chlorid, Lithiumperchlorat, Zink(II)chlorid, Indium(III)chlorid oder Indium(III)bromid. Bevorzugt ist Bortrifluorid-Diethylether-Komplex.

Die Reaktion (XVII) → (XVIII) wird im Allgemeinen in einem Temperaturbereich von -78°C bis +40°C, bevorzugt bei 0°C bis +20°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XVIII) + (XIX) → (XX) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril. Bevorzugt ist DMF.

Geeignete Basen für den Verfahrensschritt (XVIII) + (XIX) → (XX) sind Alkalihydride wie Kaliumhydrid oder Natriumhydrid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Cäsiumcarbonat.

Die Reaktion (XVIII) + (XIX) → (XX) wird im Allgemeinen in einem Temperaturbereich von 0°C bis +60°C, bevorzugt bei +10°C bis +25°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XX) → (XXI) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist DMSO.

Die Reaktion (XX) → (XXI) wird im Allgemeinen in einem Temperaturbereich von +20°C bis +180°C, bevorzugt bei +100°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzung (XXI) → (II) erfolgt nach den dem Fachmann bekannten Methoden in einem zweistufigen Prozess zunächst unter Bildung des Iminoesters mit Natriummethanolat in Methanol bei 0°C bis +40°C und anschliessender nucleophiler Addition eines Ammoniak-Äquivalents wie beispielsweise Ammoniak oder Ammoniumchlorid in einer geeigneten Säure unter Bildung des Amidins (III) bei +50 bis +150°C.

Geeignete Säure für die Bildung des Amidins (II) sind anorganische Säuren wie beispielsweise Chlorwasserstoff/ Salzsäure, Schwefelsäure, Polyphosphorsäure oder Phosphorsäure oder organische Säuren wie beispielsweise Essigsäure, Trifluoressigsäure oder Ameisensäure. Bervorzugt werden Salzsäure oder Essigsäure verwendet.

Das beschriebene Herstellverfahren kann durch das folgende Syntheseschema (Schema 7) beispielhaft verdeutlicht werden:

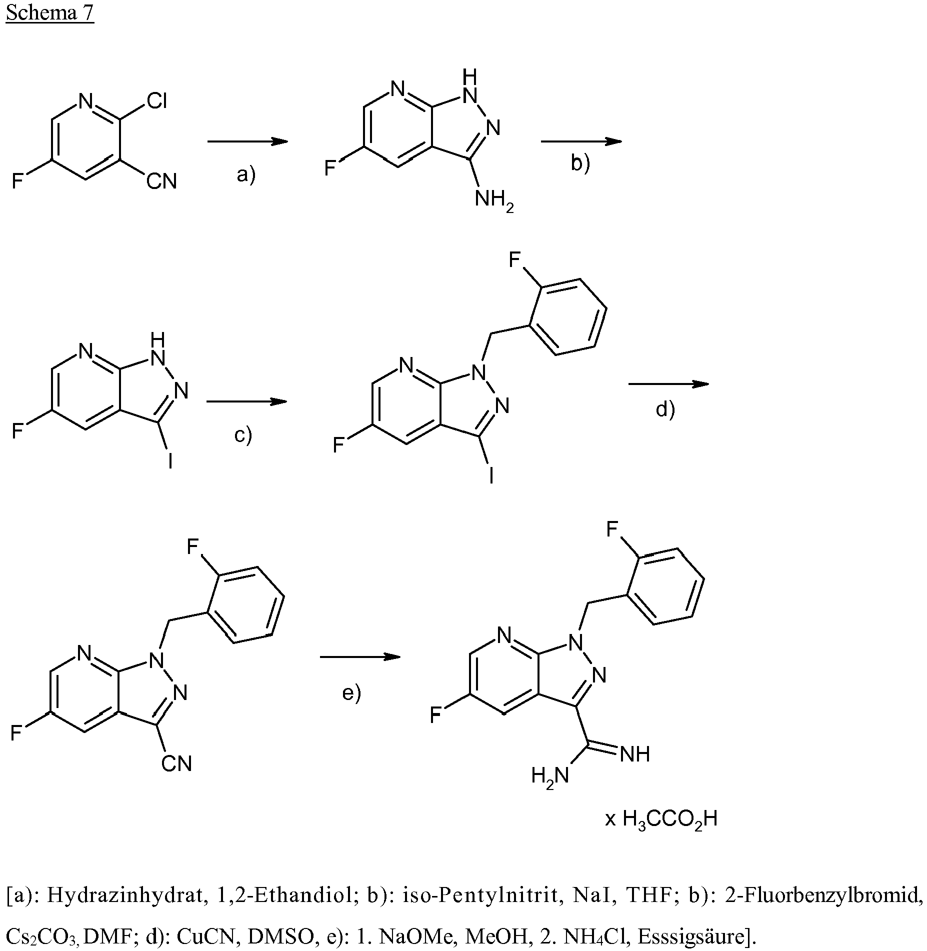

Alternativ erfolgt die Herstellung der Verbindungen der Formel (II) wie im nachfolgenden Syntheseschema (Schema 13) beispielhaft gezeigt:

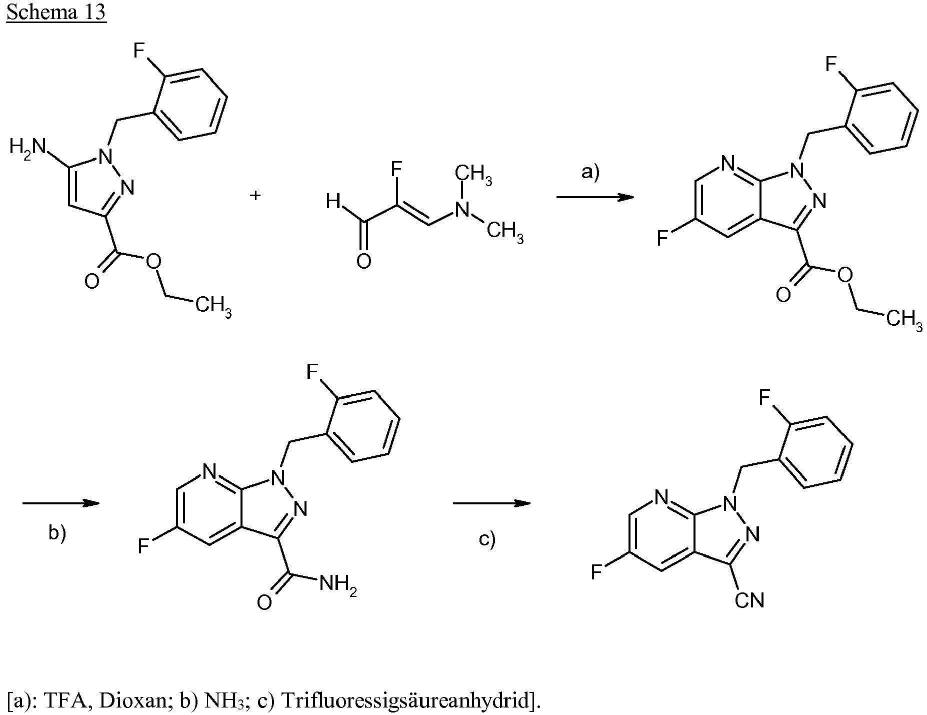

Die Verbindung der Formel (XVI) ist literaturbekannt [vgl. z.B. Winn M., J. Med. Chem. 1993, 36, 2676-7688; EP 634 413-A1; CN 1613849-A; EP 1626045-A1; WO 2009/018415], kann in Analogie zu literaturbekannten Verfahren oder wie im nachstehenden Syntheseschema gezeigt (Schema 8) hergestellt werden:

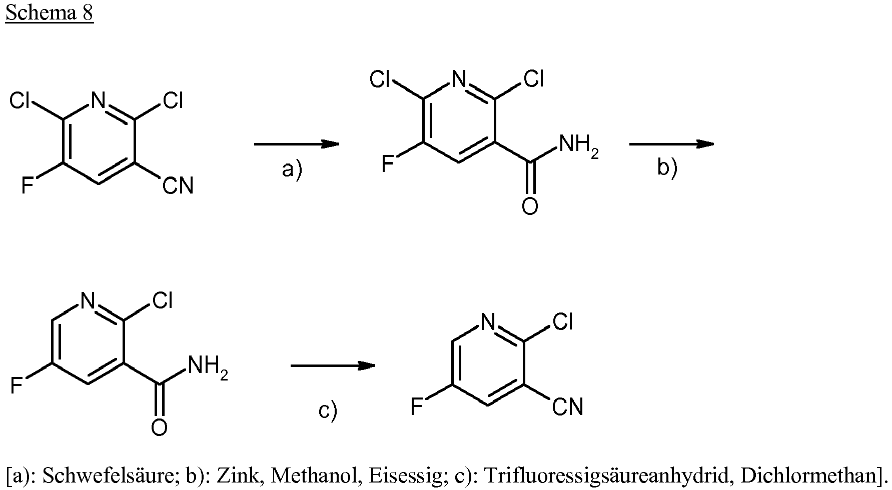

Die Verbindungen der Formeln (III) und (VI) sind kommerziell erhältlich, literaturbekannt, köännen in Analogie zu literaturbekannten Verfahren oder wie in den nachfolgenden Syntheseschemata (Schemata 9 und 10) beispielhaft gezeigt, hergestellt werden:

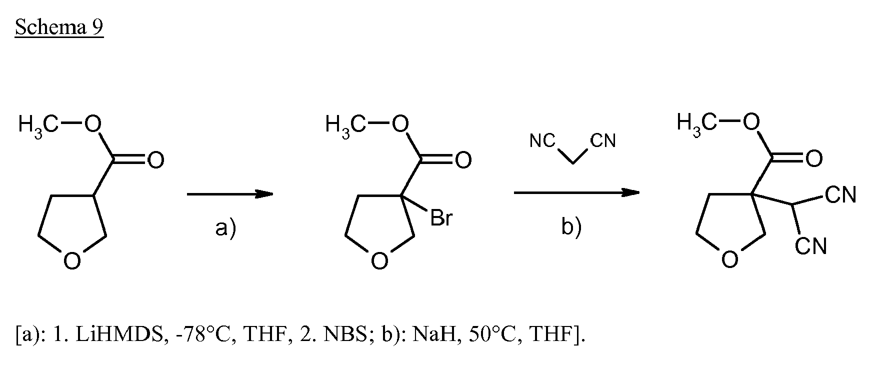

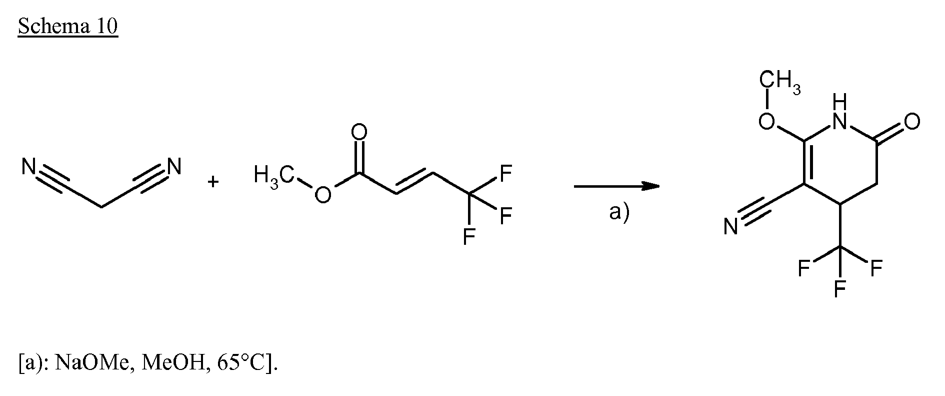

Die Verbindungen der Formel (VII) und (XII) sind kommerziell erhältlich, literaturbekannt oder können in Analogie zu literaturbekannten Vorschriften hergestellt werden.

Die erfindungsgemäßen Verbindungen sind potente Stimulatoren der löslichen Guanylatcyclase, besitzen wertvolle pharmakologische Eigenschaften, und weisen ein verbessertes therapeutisches Profil auf, wie beispielsweise hinsichtlich ihrer *in-vivo* Eigenschaften und/oder ihres pharmakokinetischen Verhaltens. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd/C: Palladium auf Aktivkohle (10%-ig)
- Ph: Phenyl
- RT: Raumtemperatur
- Rₜ: Retentionszeit (bei HPLC)
- t-Bu: tert.-Butyl
- TFA: Trifluoressigsäure
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-triisopropylbiphenyl-2-yl)-phosphin

### LC/MS-Methoden:

### Methode 1 (LC-MS):

Instrument: Waters ACQUITY SQD UPLC System; Säule: Waters Acquity UPLC HSS T3 1,8µ 50 x 1mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 100% A → 3.0 min 10% A → 4.0 min 10% A, Ofen: 55°C; Fluss 2ml/min; UV-Detektion: 210 nm.

### Methode 3 (LC-MS):

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.

### Methode 4 (LC-MS):

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Ausgangsverbindungen und Intermediate:

### Beispiel 1A

### 1-(2-Fluorbenzyl)-1H-pyrazolo[3,4-b]pyridin-3-carboximidamid-Hydrochlorid

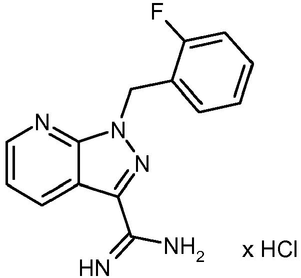

Die Synthese dieser Verbindung ist beschrieben in WO 03/095451, Beispiel 6A.

### Beispiel 2A

### 2,6-Dichlor-5-fluornicotinamid

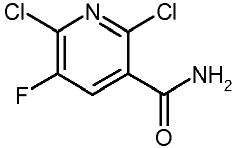

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.

Ausbeute: 24.5 g (90% d. Theorie)

¹H-NMR (400 MHz, DMSO-d₆): δ = 7.95 (br s, 1H), 8.11 (br s, 1H), 8.24 (d, 1H).

### Beispiel 3A

### 2-Chlor-5-fluornicotinamid

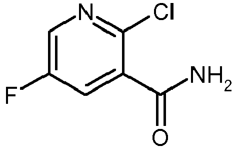

Zu einer Suspension von 21.9 g (335.35 mmol) Zink in Methanol (207 ml) wurden bei RT 44 g (210.58 mmol) 2,6-Dichlor-5-fluornicotinamid gegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigter wässriger Natriumhydrogencarbonat-Lösung(414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min. ausgerührt. Es wurde erneut abgesaugt und mit Diethylether nachgewaschen und trockengesaugt.

Ausbeute: 20.2 g (53% d. Theorie)

¹H-NMR (400 MHz, DMSO-d₆): δ = 7.87 (br s, 1H), 7.99 (dd, 1H), 8.10 (br s, 1H), 8.52 (d, 1H).

### Beispiel 4A

### 2-Chlor-5-fluornicotinonitril

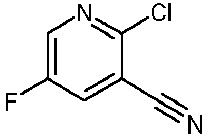

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.

Ausbeute: 42.1 g (90% d. Theorie).

¹H-NMR (400 MHz, DMSO-d₆): δ = 8.66 (dd, 1H), 8.82 (d, 1H).

### Beispiel 5A

### 5-Fluor-1H-pyrazolo[3,4-b]pyridin-3-amin

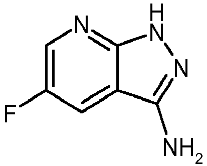

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml) versetzt. Es wurde unter Rühren für 4 h zum Rückfluss erhitzt. Beim Abkühlen fiel das Produkt aus. Die Kristalle wurden mit Wasser (380 ml) versetzt und 10 min bei RT ausgerührt. Anschliessend wurde die Suspension über eine Fritte abgesaugt, mit Wasser (200 ml) und mit -10°C-kaltem THF (200 ml) nachgewaschen. Trocknung im Hochvakuum über Phosphorpentoxid.

Ausbeute: 22.8 g (61 % d. Theorie)

¹H-NMR (400 MHz, DMSO-d₆): δ = 5.54 (s, 2H), 7.96 (dd, 1H), 8.38 (m, 1H), 12.07(m, 1H).

### Beispiel 6A

### 5-Fluor-3-iod-1H-pyrazolo[3,4-b]pyridin

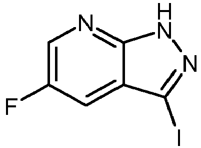

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.81) gegeben und zweimal mit Essigsäureethylester (je 487 ml) extrahiert. Die gesammelten organischen Phasen wurden mit gesättigter wässriger Natriumchlorid-Lösung (244 ml) gewaschen, getrocknet, filtriert und eingeengt. Man erhielt 12.1 g (86%-ige Reinheit, 60 % d. Th.) der Titelverbindung als Feststoff. Das Rohprodukt wurde ohne weitere Reinigung umgesetzt.

LC-MS (Methode 2): Rₜ = 1.68 min

MS (ESIpos): m/z = 264 (M+H)+

### Beispiel 7A

### 5-Fluor-1-(2-fluorbenzyl)-3-iod-1H-pyrazolo[3,4-b]pyridin

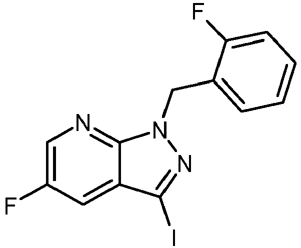

In DMF (217 ml) wurden 12.1 g (ca. 39.65 mmol) der Verbindung aus Beispiel 6A vorgelegt und anschließend 8.25 g (43.62 mmol) 2-Fluorbenzylbromid sowie 14.21 g (43.62 mmol) Cäsiumcarbonat zugefügt. Die Mischung wurde zwei Stunden bei RT gerührt. Anschließend wurde die Reaktionsmischung auf Wasser (1.17 1) gegeben und zweimal mit Essigsäureethylester (502 ml) extrahiert. Die gesammelten organischen Phasen wurden mit gesättigter wässriger Natriumchlorid-Lösung (335 ml) gewaschen, getrocknet, filtriert und eingeengt. Der Rückstand wurde an Kieselgel (Laufinittel: Petrolether/ Essigsäureethylester 97:3) chromatographiert und die Produktfraktionen eingeengt. Man erhielt 9.0 g (61 % d. Th.) der Titelverbindung als Feststoff. Der Feststoff wird in Essigsäureethylester aufgenommen und mit 10%-iger wässriger Natriumthiosulfatlösung und danach mit gesättigter wässriger Natriumchlorid-Lösung gewaschen, getrocknet und eingeengt.

LC-MS (Methode 2): Rₜ = 2.57 min

MS (ESIpos): m/z = 372 (M+H)⁺

1H-NMR (400 MHz, DMSO-d6): δ = 5.73 (s, 2H), 7.13 - 7.26 (m, 3H), 7.33 - 7.41 (m, 1H), 7.94 (dd, 1H), 8.69 - 8.73 (m, 1H).

### Beispiel 8A

### 5-Fluor-1-(2-fluorbenzyl)-1H-pyrazolo[3,4-b]pyridin-3-carbonitril

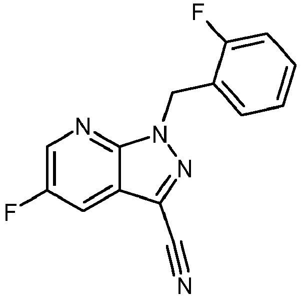

Eine Suspension aus 16.03 g (43.19 mmol) 5-Fluor-1-(2-fluorbenzyl)-3-iod-1H-pyrazolo[3,4-b]pyridin (Beispiel 7A) und 4.25 g (47.51 mmol) Kupfercyanid wurden in DMSO (120 ml) vorgelegt und 2 h bei 150°C gerührt. Nach Abkühlen wurde der Kolbeninhalt auf ca. 40°C abgekühlt, auf eine Lösung aus konz. Ammoniakwasser (90 ml) und Wasser (500 ml) gegossen, mit Essigsäureethylester (200 ml) versetzt und kurz ausgerührt. Die wässrige Phase wurde abgetrennt und noch zweimal mit Essigsäureethylester (je 200 ml) extrahiert. Die vereinigten organischen Phasen wurden zweimal mit 10%-iger wässriger Natriumchlorid-Lösung (je 100 ml) gewaschen, getrocknet und im Vakuum eingeengt. Das Rohprodukt wurde ohne weitere Reinigung umgesetzt.

Ausbeute: 11.1 g (91 % d. Theorie)

¹H-NMR (400 MHz, DMSO-d₆): δ = 5.87 (s, 2H), 7.17 - 7.42 (m, 4H), 8.52 (dd, 1H), 8.87 (dd, 1H).

### Beispiel 9A

### 5-Fluor-1-(2-fluorbenzyl)-1H-pyrazolo[3,4-b]pyridin-3-carboximidamid-Acetat

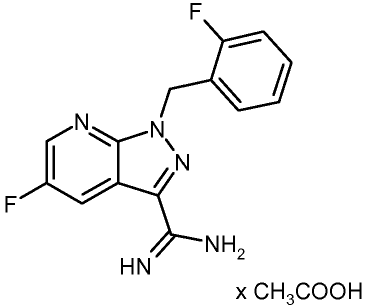

Zu 2.22 g (41.07 mmol) Natriummethanolat in Methanol (270 ml) wurden 11.1 g (41.07 mmol) 5-Fluor-1-(2-fluorbenzyl)-1H-pyrazolo[3,4-b]pyridin-3-carbonitril (Beispiel 8A) gegeben und 2 h bei RT gerührt. Danach wurden 2.64 g (49.29 mmol) Ammoniumchlorid und Essigsäure (9.17 ml) zugegeben und über Nacht zum Rückfluss erhitzt. Danach wurde bis zur Trockene eingeengt und der Rückstand in Wasser (100 ml) und Essigsäureethylester (100 ml) aufgenommen und mit 2N Natronlauge auf pH 10 gestellt. Es wurde für ca. 1 h bei RT intensiv gerührt. Die erhaltene Suspension wurde abgesaugt und mit Essigsäureethylester (100 ml), Wasser (100 ml) und nochmals Essigsäureethylester (100 ml) nachgewaschen. Der Rückstand wird über Pphosphorpentoxid im Hochvakuum getrocknet.

### Ausbeute: 9.6 g (78 % d. Theorie)

MS (ESIpos): m/z = 288 (M+H)⁺

1H-NMR (400 MHz, DMSO-d₆): δ = 1.85 (s, 3H), 5.80 (s, 2H), 7.14 - 7.25 (m, 3H), 7.36 (m, 1H), 8.42 (dd, 1H), 8.72 (dd, 1H).

### Bespiel 10A

### Methyl-3,3-dicyan-2,2-dimethylpropanoat

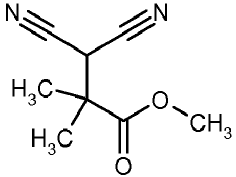

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben und es wurde über Nacht bei RT gerührt. Danach wurden nochmals 5.876 ml (45.411 mmol) Methyl-2-brom-2-methylpropanoat zugegeben und es wurde über Nacht auf 50°C erhitzt. Dann wurden abermals 1.762 ml (13.623) mmol) Methyl-2-brom-2-methylpropanoat zugegeben und es wurde weitere 4h auf 50°C erhitzt. Der Ansatz wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, mit Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.

Ausbeute: 6.47 g (85% d. Th.)

1H-NMR (400 MHz, DMSO-d6): δ [ppm] = 1.40 (s, 6H), 3.74 (s, 3H), 5.27 (s, 1H).

### Beispiel 11A

### Methyl-3-bromtetrahydrofuran-3-carboxylat

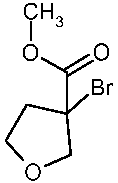

Es wurden 5.0 g (38.419 mmol) Methyltetrahydrofuran-3-carboxylat (Journal of Organic Chemistry; 1996; 2690) in 200 ml THF gelöst und auf -78°C abgekühlt und anschliessend mit 76.83 ml (76.83 mmol) Bis-(trimethylsilyl)-lithiumamid, (1M in THF), versetzt. Nach 30 min bei -78°C wurden 10.26 g (57.63 mmol) N-Bromsuccinimid in 50 ml THF suspendiert langsam zugegeben. Danach wurde liess man über Nacht auf RT erwärmen. Der Ansatz wurde mit Wasser versetzt und mit Essigsäureethylester extrahiert. Die Phasen wurden getrennt und die wässrige Phase wurde noch zweimal mit Essigsäureethylester extrahiert. Die vereinigten organischen Phasen wurden mit gesättigter wässriger Natriumchlorid-Lösung gewaschen und anschliessend mit Natriumsulfat getrocknet, filtriert und eingeengt. Das Rohprodukt wurde mittels Chromatographie an Kieselgel (Laufinittel: Dichlormethan) gereinigt. Man erhielt 491 mg (6 % d. Th.) der Titelverbindung.

1H-NMR (400 MHz, CDCl₃): δ [ppm] = 2.49 (ddd, 1H), 2.74 (ddd, 1H), 3.83 (s, 3H), 4.03-4.10 (m, 1H), 4.11-4.17 (m, 2H), 4.31 (d, 1H).

### Beispiel 12A

### Methyl-3-(dicyanmethyl)tetrahydrofuran-3-carboxylat

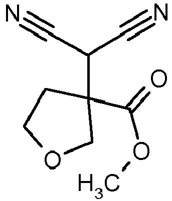

440 mg (11.00 mmol) Natriumhydrid (60%-ig in Mineralöl) wurden in 30 ml THF vorgelegt und portionsweise mit 726 mg (11.00 mmol) Malonsäuredinitril versetzt. Danach wurden 2.3 g (11.00 mmol) der unter Beispiel 69A erhaltenen Verbindung in THF (50 ml) zugegeben. Es wurde 6 h bei RT gerührt und anschliessend über Nacht auf 50°C erhitzt. Nach Abkühlen wurde der Ansatz mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen und anschliessend mit Natriumsulfat getrocknet, filtriert und eingeengt. Der Rückstand (2.66 g) wurde 1 h am Hochvakuum getrocknet und anschließend ohne weitere Reinigung umgesetzt.

### Beispiel 13A

### 4-Amino-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

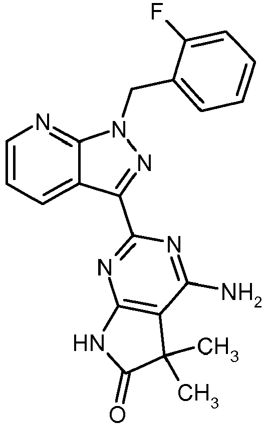

5.887 g (19.256 mmol) Beispiel 1A wurden in tert.-Butanol (50 ml) vorgelegt und mit 2.593 g (23.107 mmol) Kalium-tert.-butylat versetzt. Anschliessend wurden 3.2 g (19.256 mmol) Beispiel 10A in tert.-Butanol (25 ml) zugetropft und die Mischung über Nacht zum Rückfluss erhitzt. Am nächsten Tag wurden nochmal 0.64 g (3.851 mmol) Beispiel 10A zugegeben und es wurde für einen weiteren Tag zum Rückfluss erhitzt. Nach Abkühlen wurde ein Niederschlag abfiltriert, welcher mit Diethylether nachgewaschen wurde. Anschliessend wurde in Wasser aufgeschlämmt und ein weiteres Mal abfiltriert und mit Diethylether nachgewaschen. Nach Trocknung im Hochvakuum konnten 6.65 g der Titelverbindung erhalten (85 % d. Th.) werden.

LC-MS (Methode 1): Rₜ = 0.90 min; MS (ESIpos): m/z = 404 (M+H)⁺

¹H-NMR (400 MHz, DMSO-d₆): δ [ppm] = 1.35 (s, 6H), 5.82 (s, 2H), 6.82 (br s, 2H), 7.14-7.25 (m, 3H), 7.33-7.40 (m, 2H), 8.63 (dd, 1H), 9.03 (dd, 1H), 10.98 (s br, 1H).

### Beispiel 14A

### 4-Amino-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

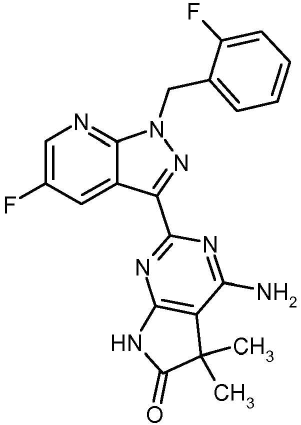

In Analogie zur Darstellung von Beispiel 2 wurden 4.18 g (12.035 mmol) Beispiel 9A mit 2.20 g (13.239 mmol) Beispiel 10A umgesetzt. Es wurden 3.72 g der Titelverbindung erhalten (73 % d. Th.).

LC-MS (Methode 1): Rₜ = 0.98 min; MS (ESIpos): m/z = 422 (M+H)⁺

¹H-NMR (400 MHz, DMSO-d₆): δ [ppm] = 1.34 (s, 6H), 5.81 (s, 2H), 6.85 (br s, 2H), 7.13-7.25 (m, 3H), 7.36 (m, 1H), 8.69 (dd, 1H), 8.84 (dd, 1H), 10.96 (s br, 1H).

### Beispiel 15A

### 4'-Amino-2'-[1-(2-fluorbenzyl)-1H-pyrazolo[3,4-b]pyridin-3-yl]-4,5-dihydrospiro[furan-3,5'-pyrrolo[2,3-d]pyrimidin]-6'(7'H)-on

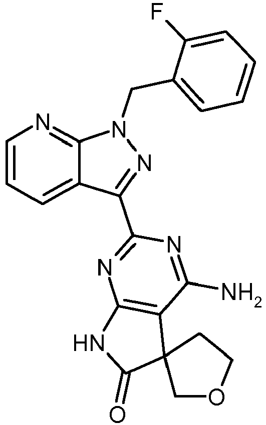

In Analogie zur Darstellung von Beispiel 13A wurden 2.257 g (7.382 mmol) Beispiel 1A mit 1.434 g (7.382 mmol) Beispiel 12A umgesetzt. Es wurden 566 mg der Titelverbindung erhalten (17 % d. Th.).

LC-MS (Methode 1): Rₜ = 0.84 min; MS (ESIpos): m/z = 432 (M+H)⁺

¹H-NMR (400 MHz, DMSO-d₆): δ [ppm] = 2.20-2.37 (m, 2H), 3.71 (d, 1H), 3.90 (q, 1H), 4.10 (d, 1H), 4.25-4.31 (m, 1H), 5.82 (s, 2H), 6.57 (br s, 2H), 7.12-7.25 (m, 3H), 7.33-7.41 (m, 2H), 8.64 (dd, 1H), 9.02 (dd, 1H), 11.96 (s br, 1H).

### Beispiel 16A

### Ethyl- {2-[1-(2-fluorbenzyl)-1H-pyrazolo[3,4-b]pyridin-3-yl]-4-hydroxypyrimidin-5-yl}acetat

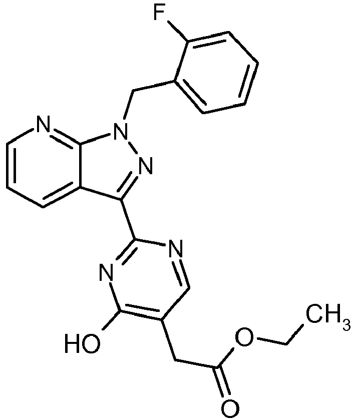

7.519 g (327 mmol) Natrium wurden in Ethanol (660 ml) gegeben und unter Argon vollständig umgesetzt. Danach wurden 50.00 g (163.53 mmol) Beispiel 1A und nach 5 min 40.45 g (188.01 mmol) Diethyl-2-formylbutandioat (Synthese beschrieben in WO 2005/73234, Seite 43) zugegeben. Anschliessend wurde für 12 h zum Rückfluss erhitzt. Nach dem Abkühlen wurde das Reaktionsgemisch mit Wasser und danach mit 1N Salzsäure versetzt. Der sich bildende Niederschlag wurde abgesaugt und nacheinander mit Wasser-Ethanol (1:1, 200 ml), Ethanol (100 ml) und abschliessend mit Diethylether gewaschen. Nach Trocknung im Hochvakuum wurden 58.0 g der Titelverbindung erhalten (83 % d. Th.).

LC-MS (Methode 1): Rₜ = 1.00 min; MS (ESIpos): m/z = 408 (M+H)⁺

¹H-NMR (400 MHz, DMSO-d₆): δ [ppm] = 1.19 (t, 3H), 3.48 (s, 2H), 4.09 (q, 2H), 5.87 (s, 2H), 7.15 (t, 1H), 7.24 (t, 1H), 7.34-7.39 (m, 2H), 7.46 (dd, 1H), 8.10 (s br, 1H), 8.71 (dd, 1H), 8.74 (d, 1H), 12.83 (s br, 1H).

### Beispiel 17A

### Ethyl-{4-chlor-2-[1-(2-fluorbenzyl)-1H-pyrazolo[3,4-b]pyridin-3-yl]pyrimidin-5-yl}acetat

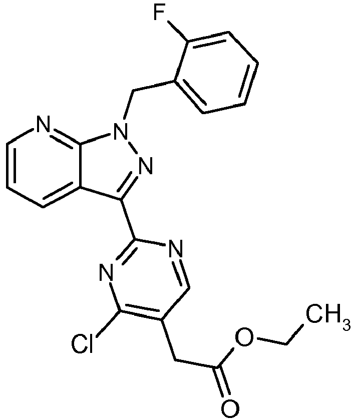

55.00 g (135 mmol) Beispiel 16A wurden in Sulfolan (220 ml) vorgelegt und mit 41.40 g (270 mmol) Phosphorylchlorid versetzt. Danach wurde für 1 h auf 120°C erhitzt. Nach Abkühlen wurde auf warmes Wasser (1500 ml) gegeben und danach mit festem Natriumhydrogencarbonat neutralisiert. Der sich bildende Niederschlag wurde abgesaugt und mit Wasser gewaschen. Danach wurde mittels Chromatographie an Kieselgel (Laufinittel: Cyclohexan/Essigsäureethylester 3:2) weiter gereinigt. Nach Trocknung im Hochvakuum wurden 43.0 g der Titelverbindung erhalten (73 % d. Th.).

LC-MS (Methode 1): Rₜ = 1.20 min; MS (ESIpos): m/z = 426 (M+H)⁺

¹H-NMR (400 MHz, DMSO-d₆): δ [ppm] = 1.21 (t, 3H), 3.96 (s, 2H), 4.15 (q, 2H), 5.90 (s, 2H), 7.16 (t, 1H), 7.22-7.27 (m, 2H), 7.36-7.39 (m, 1H), 7.49 (dd, 1H), 8.71 (dd, 1H), 8.84 (dd, 1H), 8.96 (s, 1H).

### Beispiel 18A

### Ethyl- {4-azido-2-[1-(2-fluorbenzyl)-1H-pyrazolo[3,4-b]pyridin-3-yl]pyrimidin-5-yl} acetat

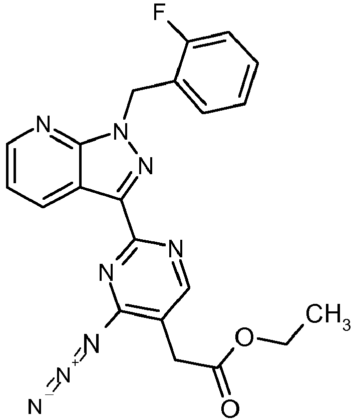

10.00 g (23.482 mmol) Beispiel 17A wurden in DMF (200 ml) vorgelegt und mit 2.290 g (35.223 mmol) Natriumazid versetzt. Danach wurde für 1 h auf 60°C erhitzt. Nach dem Abkühlen wurde das Reaktionsgemisch auf Wasser gegeben und dreimal mit Essigsäureethylester extrahiert. Die organischen Phasen wurden vereinigt und einmal mit gesättigter wässriger Natriumchlorid-Lösung gewaschen, dann über Natriumsulfat getrocknet, filtriert und eingeengt. Der Rückstand wurde ohne weitere Reinigung in der nächsten Stufe eingesetzt.

LC-MS (Methode 1): Rₜ = 1.16 min; MS (ESIpos): m/z = 433 (M+H)⁺

### Beispiel 19A

### Ethyl- {4-amino-2-[1-(2-fluorbenzyl)-1H-pyrazolo[3,4-b]pyridin-3-yl]pyrimidin-5-yl} acetat

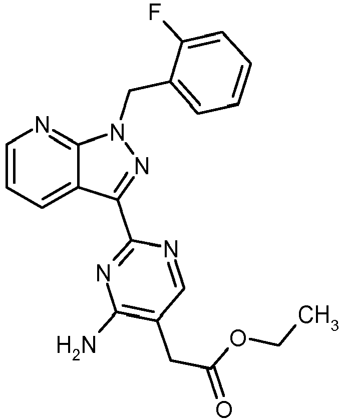

10.15 g (23.482 mmol) Rohprodukt aus Beispiel 18A wurden in DMF (400 ml) mit Palladium auf Kohle (10 %) über Nacht bei Wasserstoff-Normaldruck hydriert. Danach wurde über Celite filtriert und eingeengt. Der Rückstand wurde ohne weitere Reinigung in der nächsten Stufe eingesetzt.

LC-MS (Methode 1): Rₜ = 0.83 min; MS (ESIpos): m/z = 407 (M+H)⁺

### Beispiel 20A

### Ethyl-1- {4-chlor-2-[1-(2-fluorbenzyl)-1H-pyrazolo[3,4-b]pyridin-3-yl]pyrimidin-5-yl}cyclopropan-carboxylat

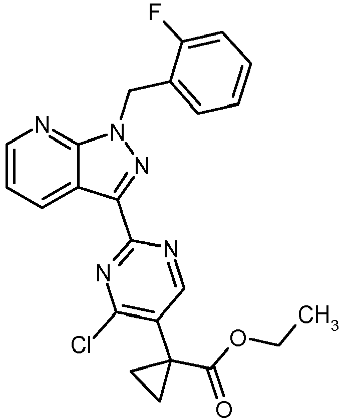

1.00 g (2.348 mmol) Beispiel 17A wurden in THF (15 ml) und DMF (15 ml) vorgelegt und mit 469 mg (11.741 mmol) Natriumhydrid (60 %) versetzt und für 15 min bei RT gerührt. Danach wurden 0.607 ml (7.054 mmol) 1,2-Dibromethan zugegeben und für weitere 30 min bei RT gerührt. Der Ansatz wurde mit Wasser und Essigsäureethylester versetzt, die Phasen getrennt und die organische Phase zweimal mit Essigsäureethylester extrahiert. Die vereinigten organischen Phasen wurden einmal mit gesättigter wässriger Natriumchlorid-Lösung gewaschen und mit Natriumsulfat getrocknet, filtriert und eingeengt. Die so erhaltene Titelverbindung (1.38 g, 75%-ige Reinheit) wurde ohne weitere Reinigung in der nächsten Stufe eingesetzt.

LC-MS (Methode 1): Rₜ = 1.26 min; MS (ESIpos): m/z = 452 (M+H)⁺

### Beispiel 21A

### Ethyl-1- {4-azido-2-[1-(2-fluorbenzyl)-1H-pyrazolo[3,4-b]pyridin-3-yl]pyrimidin-5-yl} cyclopropancarboxylat

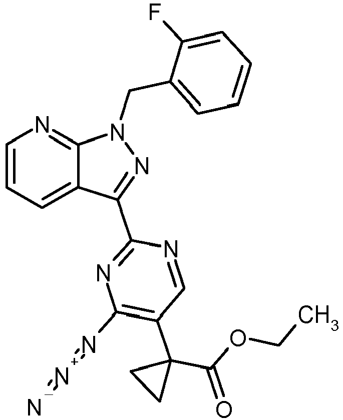

550 mg (ca. 0.913 mmol) von Beispiel 20A wurden in Analogie zur Vorschrift unter Beispiel 18A umgesetzt. Die so erhaltene Titelverbindung wurde ohne weitere Reinigung in der nächsten Stufe eingesetzt.

LC-MS (Methode 1): Rₜ = 1.23 min; MS (ESIpos): m/z = 458 (M+H)⁺

### Beispiel 22A

### Ethyl-1- {4-amino-2-[1-(2-fluorbenzyl)-1H-pyrazolo[3,4-b]pyridin-3-yl]pyrimidin-5-yl} cyclopropancarboxylat

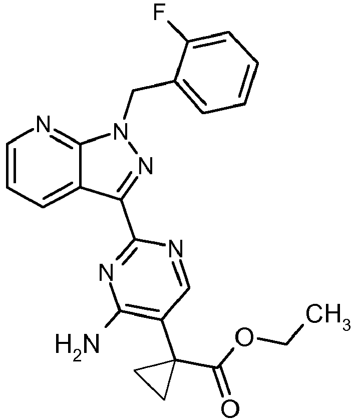

418 mg (0.913 mmol) von Beispiel 21A wurden in Analogie zur Vorschrift unter Beispiel 19A hydriert. Die so erhaltene Titelbindung wurde ohne weitere Reinigung in der nächsten Stufe eingesetzt.

LC-MS (Methode 1): Rₜ = 0.86 min; MS (ESIpos): m/z = 433 (M+H)⁺

### Beispiel 23A

### 1-(2-Fluorbenzyl)-1H-pyrazolo[3,4-b]pyridin-3-carboximidohydrazid

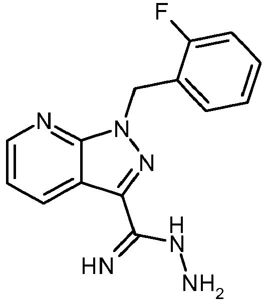

20.000 g (65.414 mmol) der Verbindung aus Beispiel 1A wurden in 320 ml Ethanol gelöst und bei 0°C mit 26.477 g (261.656 mmol) Triethylamin sowie 4.093 g (65.414 mmol) Hydrazinhydrat (80%-ige Lösung in Wasser) versetzt. Das Gemisch wurde über Nacht bei RT gerührt und anschließend am Rotationsverdampfer eingeengt. Es wurden 26.84 g (100 % d. Th., 69%-ige Reinheit) der Titelverbindung erhalten.

LC-MS (Methode 3): Rₜ = 0.64 min; MS (ESIpos): m/z = 285 (M+H)⁺

### Beispiel 24A

### Diethyl-2-(difluoracetyl)butandioat

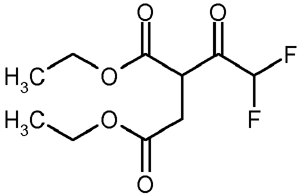

0.52 g (14.296 mmol) Natriumhydrid (60 % in Mineralöl) wurden in THF (30 ml) vorgelegt und anschliessend wurde bei 0°C eine Lösung von 2.00 g (11.437 mmol) Ethyl-4,4-difluor-3-oxobutyrat in THF (20 ml) zugetropft. Nach Erwärmen auf RT und weiteren 30 min bei dieser Temperatur wurden 2.865 g (17.156 mmol) Bromessigsäureethylester in THF (15 ml) zugegeben und anschliessend über Nacht zum Rückfluss erhitzt. Nach Abkühlen wurde der Ansatz mit gesättigter wässriger Ammoniumchlorid-Lösung versetzt und dreimal mit Essigsäureethylester extrahiert. Die vereinigten organischen Phasen wurden mit Natriumsulfat getrocknet, filtriert und eingeengt. Nach 5 min am Hochvakuum wurde die so erhaltene Titelverbindung (3.00 g, ca. 50%-ige Reinheit) ohne weitere Reinigung in der nächsten Stufe eingesetzt.

### Beispiel 25A

### Ethyl- {4-(difluormethyl)-2-[1-(2-fluorbenzyl)-1H-pyrazolo[3,4-b]pyridin-3-yl]-6-hydroxypyrimidin-5-yl} acetat

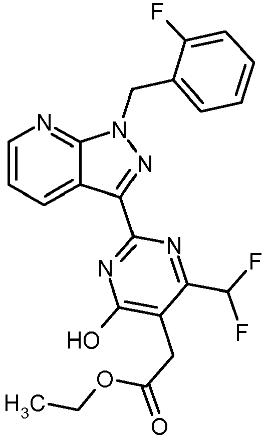

In Analogie zu Beispiel 16A wurden 1.521 g (4.973 mmol) Beispiel 1A und 2.885 g (ca. 5.719 mmol) Beispiel 24A umgesetzt. Nach Aufarbeitung erfolgte eine Reinigung mittels präparativer HPLC (Eluent: Acetonitril/ Wasser, Gradient). Es wurden 600 mg (26% d. Th.) der Titelverbindung erhalten.

LC-MS (Methode 1): Rₜ = 1.08 min; MS (ESIpos): m/z = 458 (M+H)⁺

### Beispiel 26A

### Ethyl- {4-chlor-6-(difluormethyl)-2-[1-(2-fluorbenzyl)-1H-pyrazolo[3,4-b]pyridin-3-yl]pyrimidin-5-yl} acetat

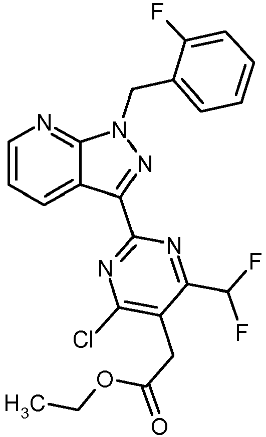

In Analogie zu Beispiel 17A wurden 600 mg (1.312 mmol) Beispiel 25A umgesetzt. Es wurden 1.7 g der Titelverbindung (ca. 36%-ige Reinheit, verunreinigt mit Sulfolan) erhalten.

LC-MS (Methode 1): Rₜ = 1.25 min; MS (ESIpos): m/z = 476 (M+H)⁺

### Beispiel 27A

### Ethyl-2- {4-chlor-6-(difluormethyl)-2-[1-(2-fluorbenzyl)-1H-pyrazolo[3,4-b]pyridin-3-yl]pyrimidin-5-yl}-2-methylpropanoat

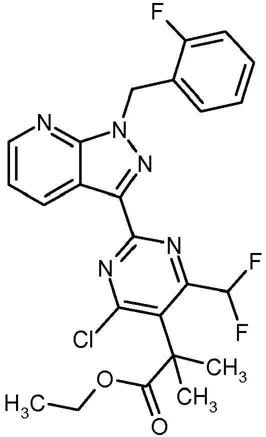

In Analogie zu 20A wurden 1.7 g (ca. 1.31 mmol) von Beispiel 26A mit Methyliodid umgesetzt. Es wurden 1.24 g der Titelverbindung (ca. 36%-ige Reinheit, verunreinigt mit Sulfolan) erhalten.

LC-MS (Methode 1): Rₜ = 1.33 min; MS (ESIpos): m/z = 504 (M+H)⁺

### Beispiel 28A

### Ethyl-2- {4-azido-6-(difluormethyl)-2-[1-(2-fluorbenzyl)-1H-pyrazolo[3,4-b]pyridin-3-yl]pyrimidin-5-yl} -2-methylpropanoat

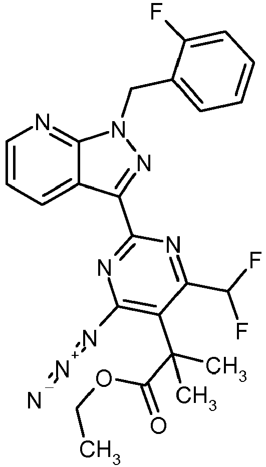

In Analogie zu Beispiel 18A wurden 1.24 g (ca. 1.312 mmol) von Beispiel 27A umgesetzt. Es wurde die Titelverbindung (verunreinigt mit Sulfolan) erhalten, die ohne Bestimmung der Ausbeute weiter umgesetzt wurde, da die Azid-enthaltende Lösung nicht weiter eingeengt wurde.

LC-MS (Methode 1): Rₜ = 1.27 min; MS (ESIpos): m/z = 511 (M+H)⁺

### Beispiel 29A

### 1-(2,4-Difluorbenzyl)-3-iod-1H-pyrazolo[3,4-b]pyridin

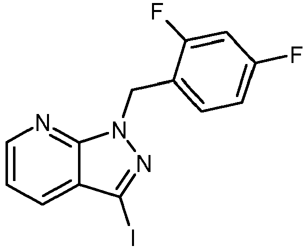

In Analogie zur Synthese von Beispiel 7A wurden 54.00 g (215.98 mmol) 3-Iod-1H-pyrazolo[3,4-b]pyridin (Synthese beschrieben in WO 2006/130673, Beispiel 4) mit 50.18 g (237.578) 2,4-Difluorbenzylbromid umgesetzt. Das Rohprodukt wurde mit Eiswasser versetzt, abgesaugt und der Niederschlag mit Isopropanol und Pentan gewaschen und anschliessend im Hochvakuum getrocknet. Es wurden 76.7 g der Titelverbindung erhalten (89 % d. Th.).

LC-MS (Methode 1): Rₜ = 1.17 min; MS (ESIpos): m/z = 372 (M+H)⁺

¹H-NMR (400 MHz, DMSO-d₆): δ = 5.71 (s, 2H), 7.04-7.08 (m, 1H), 7.25 - 7.36 (m, 3H), 7.96 (dd, 1H), 8.65 (dd, 1H).

### Beispiel 30A

### 1-(2,4-Difluorbenzyl)-1H-pyrazolo[3,4-b]pyridin-3-carbonitril

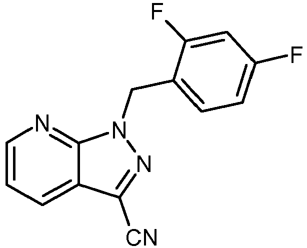

In Analogie zu Beispiel 8A wurden 76.00 g (204.78 mmol) von Beispiel 29A umgesetzt. Es wurden 57.00 g (94 %-ige Reinheit, 97 % d. Th.) der Titelverbindung erhalten.

LC-MS (Methode 1): Rₜ = 1.07 min; MS (ESIpos): m/z = 271 (M+H)^{+ 1}H-NMR (400 MHz, DMSO-d₆): δ = 5.86 (s, 2H), 7.07-7.12 (m, 1H), 7.27 - 7.32 (m, 1H), 7.44-7.50 (m, 1H), 7.55 (dd, 1H), 8.49 (dd, 1H), 8.81 (dd, 1H).

### Beispiel 31A

### 1-(2,4-Difluorbenzyl)-1H-pyrazolo[3,4-b]pyridin-3-carboximidamid-Acetat

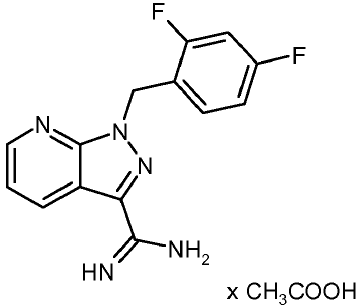

In Analogie zu Beispiel 9A wurden 56.00 g (207.221 mmol) von Beispiel 30A umgesetzt. Es wurden 19.00 g der Titelverbindung erhalten (26 % d. Th.).

LC-MS (Methode 1): Rₜ = 0.60 min; MS (ESIpos): m/z = 288 (M+H)⁺

¹H-NMR (400 MHz, DMSO-d₆): δ = 1.81 (s, 3H), 5.80 (s, 2H), 7.03-7.08 (m, 1H), 7.26 - 7.37 (m, 2H), 7.41-7.44 (m, 1H), 8.61 (dd, 1H), 8.69 (dd, 1H).

### Beispiel 32A

### Ethyl- {2-[1-(2,4-difluorbenzyl)-1H-pyrazolo[3,4-b]pyridin-3-yl]-4-hydroxypyrimidin-5-yl}acetat

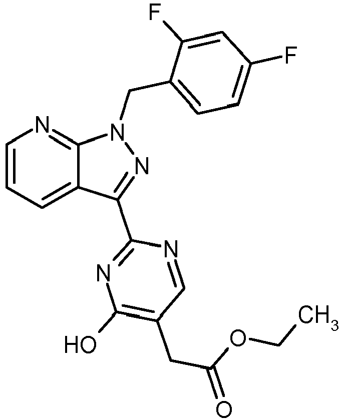

15 g (43.187 mmol) Beispiel 31A wurden analog der Vorschrift von Beispiel 16A umgesetzt. Man erhielt 14.30 g der Titelverbindung (75 % d. Th.).

LC-MS (Methode 1): Rₜ = 1.03 min; MS (ESIpos): m/z = 426 (M+H)⁺

¹H-NMR (400 MHz, DMSO-d₆): δ [ppm] = 1.17 (t, 3H), 3.43 (s, 2H), 4.08 (q, 2H), 5.81 (s, 2H), 7.04 (ddd, 1H), 7.28 (ddd, 1H), 7.40-7.46 (m, 2H), 8.00 (s, 1H), 8.67 (dd, 1H), 8.77 (d, 1H), 12.73 (s br, 1H).

### Beispiel 33A

### Ethyl- {4-chlor-2-[1-(2,4-difluorbenzyl)-1H-pyrazolo[3,4-b]pyridin-3-yl]pyrimidin-5-yl} acetat

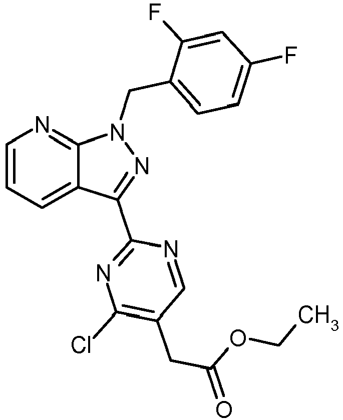

14.20 g (33.380 mmol) Beispiel 32A wurden analog der Vorschrift von Beispiel 17A umgesetzt. Man erhielt 13.20 g der Titelverbindung (88 % d. Th.).

LC-MS (Methode 1): Rₜ = 1.23 min; MS (ESIpos): m/z = 444 (M+H)⁺

¹H-NMR (400 MHz, DMSO-d₆): δ [ppm] = 1.21 (t, 3H), 3.95 (s, 2H), 4.15 (q, 2H), 5.87 (s, 2H), 7.06 (ddd, 1H), 7.29 (ddd, 1H), 7.36 (ddd, 1H), 7.48 (dd, 1H), 8.71 (dd, 1H), 8.83 (dd, 1H), 8.96 (s, 1H).

### Beispiel 34A

### 1-(2,3-Difluorbenzyl)-3-iod-1H-pyrazolo[3,4-b]pyridin

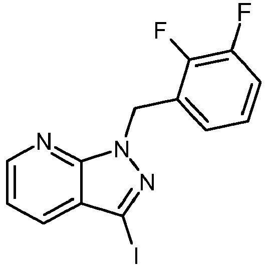

In Analogie zur Synthese von Beispiel 7A wurden 54.00 g (215.98 mmol) 3-Iod-1H-pyrazolo[3,4-b]pyridin (Synthese beschrieben in WO 2006/130673 Beispiel 4) mit 50.18 g (237.578) 2,3-Difluorbenzylbromid umgesetzt. Das Rohprodukt wurde mit Eiswasser versetzt, abgesaugt und der Niederschlag mit Isopropanol und Pentan gewaschen und anschliessend im Hochvakuum getrocknet. Es wurden 73.00 g der Titelverbindung erhalten (85 % d. Th.).

LC-MS (Methode 1): Rₜ = 1.13 min; MS (ESIpos): m/z = 372 (M+H)⁺

¹H-NMR (400 MHz, DMSO-d₆): δ = 5.79 (s, 2H), 7.04 (t, 1H), 7.14 - 7.20 (m, 1H), 7.33-7.43 (m, 2H), 7.98 (dd, 1H), 8.66 (dd, 1H).

### Beispiel 35A

### 1-(2,3-Difluorbenzyl)-1H-pyrazolo[3,4-b]pyridin-3-carbonitril

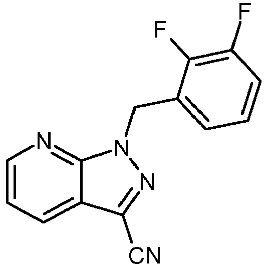

In Analogie zur Synthese von Beispiel 8A wurden 72.00 g (194.002 mmol) Beispiel 34A umgesetzt. Es wurden 50.00 g (93 %ige Reinheit, 88 % d. Th.) der Titelverbindung erhalten.

LC-MS (Methode 3): Rₜ = 1.24 min; MS (ESIpos): m/z = 271 (M+H)⁺

¹H-NMR (400 MHz, DMSO-d₆): δ = 5.94 (s, 2H), 7.14-7.21 (m, 2H), 7.39 - 7.46 (m, 1H), 7.55 (dd, 1H), 8.51 (dd, 1H), 8.81 (dd, 1H).

### Beispiel 36A

### 1-(2,3-Difluorbenzyl)-1H-pyrazolo[3,4-b]pyridin-3-carboximidamid-Acetat

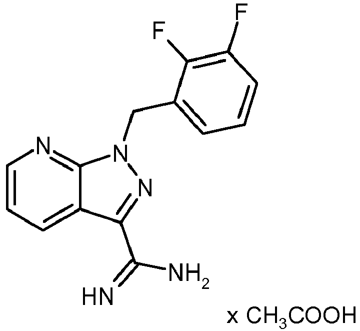

In Analogie zur Synthese von Beispiel 9A wurden 49.00 g (181.318 mmol) Beispiel 35A umgesetzt. Es wurden 29.00 g der Titelverbindung erhalten (46 % d. Th.).

LC-MS (Methode 1): Rₜ = 0.62 min; MS (ESIpos): m/z = 288 (M+H)⁺

¹H-NMR (400 MHz, DMSO-d₆): δ = 1.81 (s, 3H), 5.88 (s, 2H), 7.04 (t, 1H), 7.13 - 7.19 (m, 1H), 7.36-7.45 (m, 2H), 8.63 (dd, 1H), 8.69 (dd, 1H).

### Beispiel 37A

### Ethyl- {2-[1-(2,3-difluorbenzyl)-1H-pyrazolo[3,4-b]pyridin-3-yl]-4-hydroxypyrimidin-5-yl}acetat

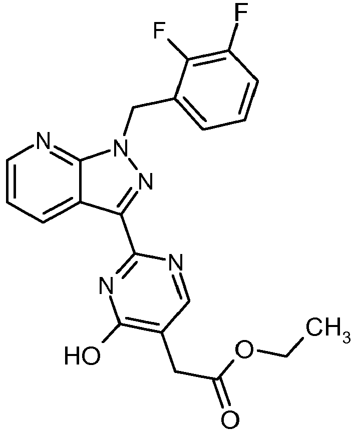

15 g (43.187 mmol) Beispiel 36A wurden analog der Vorschrift von Beispiel 16A umgesetzt. Man erhielt 13.20 g der Titelverbindung (69 % d. Th.).

LC-MS (Methode 1): Rₜ = 1.03 min; MS (ESIpos): m/z = 426 (M+H)⁺

¹H-NMR (400 MHz, DMSO-d₆): δ [ppm] = 1.19 (t, 3H), 3.48 (s, 2H), 4.09 (q, 2H), 5.90 (s, 2H), 7.13-7.18 (m, 2H), 7.36-7.43 (m, 1H), 7.47 (dd, 1H), 8.11 (s, 1H), 8.72-8.75 (m, 2H), 12.83 (s br, 1H).

### Beispiel 38A

### Ethyl- {4-chlor-2-[1-(2,3-difluorbenzyl)-1H-pyrazolo[3,4-b]pyridin-3-yl]pyrimidin-5-yl} acetat

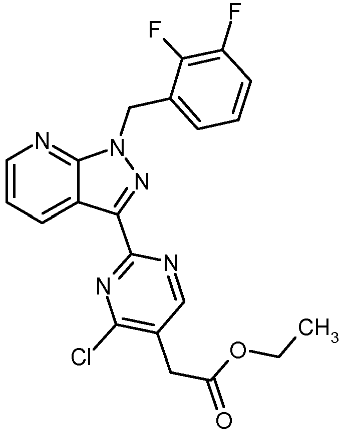

13.10 g (30.795 mmol) Beispiel 37A wurden analog der Vorschrift beschrieben unter Beispiel 17A umgesetzt. Man erhielt 12.10 g der Titelverbindung (88 % d. Th.).

LC-MS (Methode 1): Rₜ = 1.23 min; MS (ESIpos): m/z = 444 (M+H)⁺

¹H-NMR (400 MHz, DMSO-d₆): δ [ppm] = 1.21 (t, 3H), 3.96 (s, 2H), 4.15 (q, 2H), 5.95 (s, 2H), 7.07-7.09 (t, 1H), 7.15-7.19 (m, 1H), 7.37-7.43 (m, 1H), 7.49 (dd, 1H), 8.71 (dd, 1H), 8.84 (dd, 1H), 8.96 (s, 1H).

### Beispiel 39A

### Ethyl-2- {4-chlor-2-[1-(2,3-difluorbenzyl)-1H-pyrazolo[3,4-b]pyridin-3-yl]pyrimidin-5-yl}-2-methylpropanoat

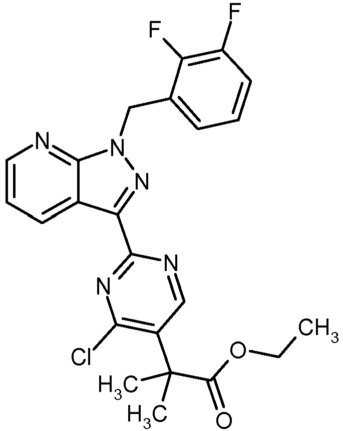

In Analogie zu Beispiel 20A wurden 800 mg (1.802 mmol) von Beispiel 38A mit Methyliodid umgesetzt. Es wurden 1.00 g (Reinheit 84%) der Titelverbindung erhalten, die ohne weitere Reinigung weiter umgesetzt wurden.

LC-MS (Methode 1): Rₜ = 1.30 min; MS (ESIpos): m/z = 472 (M+H)⁺

¹H-NMR (400 MHz, DMSO-d₆): δ [ppm] = 1.14 (t, 3H), 1.65 (s, 6H), 4.13 (q, 2H), 5.95 (s, 2H), 7.07 (t, 1H), 7.15-7.20 (m, 1H), 7.37-7.44 (m, 1H), 7.49 (dd, 1H), 8.72 (dd, 1H), 8.83 (dd, 1H), 9.06 (s, 1H).

### Beispiel 40A

### Ethyl-2- {4-azido-2-[1-(2,3-difluorbenzyl)-1H-pyrazolo[3,4-b]pyridin-3-yl]pyrimidin-5-yl}-2-methylpropanoat

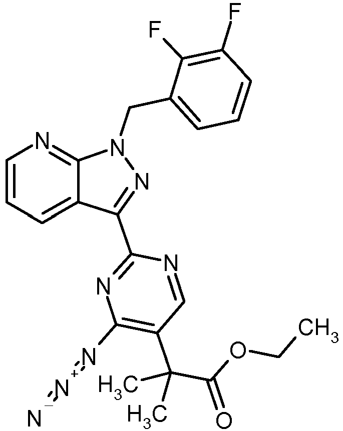

1.00 g (ca. 1.80 mmol, 84 % Reinheit) von Beispiel 39A wurden in Analogie zu Beispiel 18A umgesetzt. Die so erhaltene Titelverbindung wurde ohne weitere Reinigung weiter umgesetzt. Eine Ausbeute konnte nicht bestimmt werden, da die Azid enthaltende Lösung nicht bis zur Trockene eingeengt wurde.

LC-MS (Methode 1): Rₜ = 1.27 min; MS (ESIpos): m/z = 479 (M+H)⁺

### Beispiel 41A

### Ethyl-2- {4-amino-2-[1-(2,3-difluorbenzyl)-1H-pyrazolo[3,4-b]pyridin-3-yl]pyrimidin-5-yl}-2-methylpropanoat

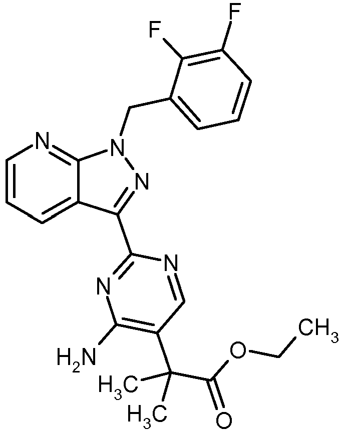

Die unter Beispiel 40A erhaltene Lösung wurde in Analogie zu Beispiel 19A hydriert. Die so erhaltene Rohverbindung (0.863 g, ca. 94%-ige Reinheit) wurde ohne weitere Reinigung in der nächsten Stufe eingesetzt.

LC-MS (Methode 1): Rₜ = 0.90 min; MS (ESIpos): m/z = 453 (M+H)⁺

### Beispiel 42A

### 4-Ethyl-1-methyl-2-(cyclopropylcarbonyl)butandioat

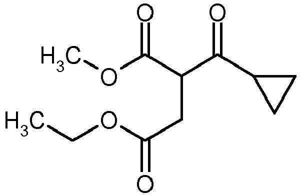

In Analogie zu Beispiel 24A wurden 2.00 g (14.069 mmol) Methyl-3-cyclopropyl-3-oxopropionat umgesetzt. Es wurden 3.62 g (ca. 80%-ige Reinheit) der Titelverbindung erhalten, welche ohne weitere Reinigung in der nächsten Stufe eingesetzt wurde.

LC-MS (Methode 1): Rₜ = 0.78 min; MS (ESIpos): m/z = 229 (M+H)⁺

### Beispiel 43A

### {4-Cyclopropyl-2-[1-(2-fluorbenzyl)-1H-pyrazolo[3,4-b]pyridin-3-yl]-6-hydroxypyrimidin-5-yl} essigsäure

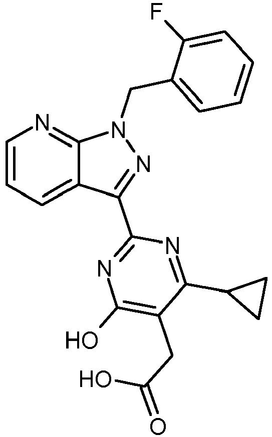

In Analogie zu Beispiel 16A wurden 3.74 g (12.234 mmol) von Beispiel 1A und 3.211 g von Beispiel 42A umgesetzt. Nach Aufarbeitung wurden 763 mg (14% d. Th.) der Titelverbindung erhalten.

LC-MS (Methode 1): Rₜ = 0.95 min; MS (ESIpos): m/z = 420 (M+H)⁺

### Beispiel 44A

### Methyl- {4-cyclopropyl-2-[1-(2-fluorbenzyl)-1H-pyrazolo[3,4-b]pyridin-3-yl]-6-hydroxypyrimidin-5-yl} acetat

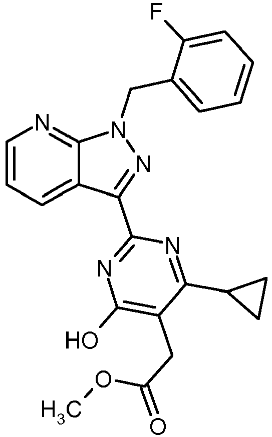

762 mg (1.817 mmol) von Beispiel 43A wurden in Methanol (20 ml) vorgelegt und mit 3 Tropfen konz. Schwefelsäure versetzt. Man erhielt einen Brei, welcher durch weitere Zugabe von Methanol (15 ml) wieder gerührt werden konnte. Es wurde 1 h zum Rückfluss erhitzt. Nach Abkühlen wurde abgesaugt und mit Methanol nachgewaschen und am Hochvakuum getrocknet. Man erhielt 685 mg (87% d. Th.) der Titelverbindung.

LC-MS (Methode 1): Rₜ = 1.07 min; MS (ESIpos): m/z = 434 (M+H)⁺

¹H-NMR (400 MHz, DMSO-d₆): δ [ppm] = 1.01-1.05 (m, 2H), 1.16-1.19 (m, 2H), 2.12-2.16 (m, 1H), 3.63 (s, 3H), 3.72 (s, 2H), 5.85 (s, 2H), 7.14 (t, 1H), 7.20-7.25 (m, 1H), 7.31-7.39 (m, 2H), 7.49 (dd, 1H), 8.55 (dd, 1H), 8.70 (dd, 1H), 12.57 (s br, 1H).

### Beispiel 45A

### Methyl-{4-chlor-6-cyclopropyl-2-[1-(2-fluorbenzyl)-1H-pyrazolo[3,4-b]pyridin-3-yl]pyrimidin-5-yl} acetat

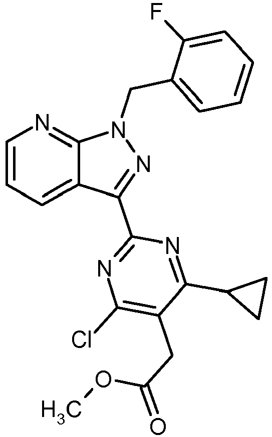

683 mg (1.576 mmol) von Beispiel 44A wurden in Phosphorylchlorid (2.423 ml) vorgelegt, mit 470 mg (3.151 mmol) Diethylanilin versetzt und für 2 Tage auf 90°C erhitzt. Nach Abkühlen wurde auf warmes Wasser gegeben, der enstandene Niederschlag abgesaugt und mit Wasser nachgewaschen und am Hochvakuum getrocknet. Man erhielt 724 mg (100 % d. Th.) der Titelverbindung.

LC-MS (Methode 1): Rₜ = 1.25 min; MS (ESIpos): m/z = 452 (M+H)⁺

### Beispiel 46A

### Methyl-2-{4-chlor-6-cyclopropyl-2-[1-(2-fluorbenzyl)-1H-pyrazolo[3,4-b]pyridin-3-yl]pyrimidin-5-yl}-2-methylpropanoat

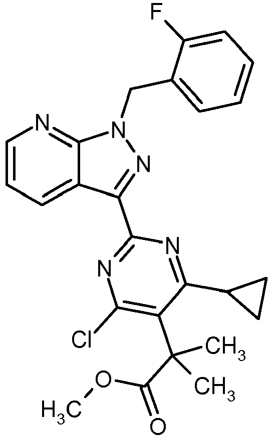

In Analogie zu Beispiel 20A wurden 712 mg (1.576 mmol) von Beispiel 45A mit Methyliodid umgesetzt. Es wurden 867 mg (ca. 75%-ige Reinheit) der Titelverbindung erhalten, die ohne weitere Reinigung im nächsten Schritt eingesetzt wurden.

LC-MS (Methode 1): Rₜ = 1.39 min; MS (ESIpos): m/z = 480 (M+H)⁺

### Beispiel 47A

### Methyl-2-{4-azido-6-cyclopropyl-2-[1-(2-fluorbenzyl)-1H-pyrazolo[3,4-b]pyridin-3-yl]pyrimidin-5-yl}-2-methylpropanoat

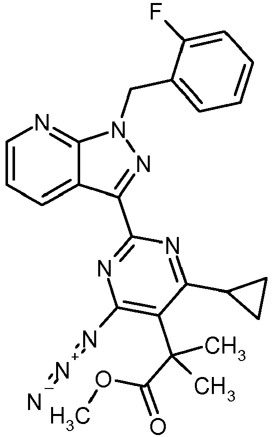

867 mg (ca. 1.355 mmol) von Beispiel 46A wurden in Analogie zur Vorschrift unter Beispiel 18A umgesetzt. Die so erhaltene Titelverbindung wurde ohne weitere Reinigung in der nächsten Stufe eingesetzt. Eine Ausbeute wurde nicht bestimmt, da die Azid enthaltende Lösung nicht bis zur Trockene eingeengt wurde.

LC-MS (Methode 1): Rₜ = 1.31 min; MS (ESIpos): m/z = 487 (M+H)⁺

### Beispiel 48A

### Ethyl-2-{4-chlor-2-[1-(2,4-difluorbenzyl)-1H-pyrazolo[3,4-b]pyridin-3-yl]pyrimidin-5-yl}-2-methylpropanoat

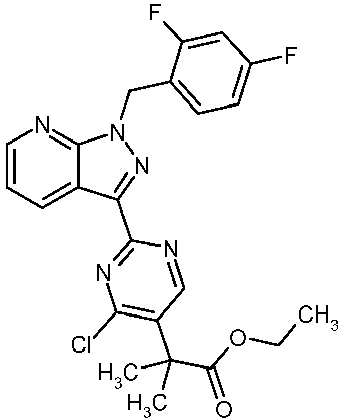

In Analogie zu Beispiel 20A wurden 800 mg (1.802 mmol) von Beispiel 33A mit Methyliodid umgesetzt. Es wurden 1.05 g (78%-ige Reinheit) der Titelverbindung erhalten, die ohne weitere Reinigung im nächsten Schritt eingesetzt wurden.

LC-MS (Methode 1): Rₜ = 1.31 min; MS (ESIpos): m/z = 472 (M+H)⁺

### Beispiel 49A

### Ethyl-2-{4-azido-2-[1-(2,4-difluorbenzyl)-1H-pyrazolo[3,4-b]pyridin-3-yl]pyrimidin-5-yl}-2-methylpropanoat

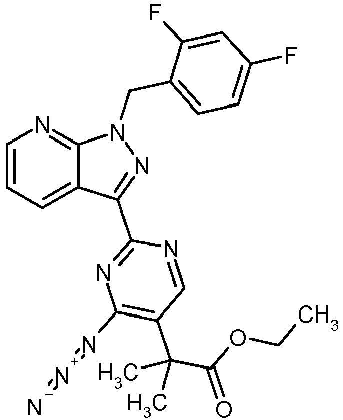

1.05 g (ca. 1.736 mmol) von Beispiel 48A wurden in Analogie zur Vorschrift unter Beispiel 18A umgesetzt. Die so erhaltene Titelverbindung wurde ohne weitere Reinigung in der nächsten Stufe eingesetzt. Eine Ausbeute wurde nicht bestimmt, da die Azid enthaltende Lösung nicht bis zur Trockene eingeengt wurde.

LC-MS (Methode 1): Rₜ = 1.27 min; MS (ESIpos): m/z = 479 (M+H)⁺

### Beispiel 50A

### Ethyl-2-{4-amino-2-[1-(2,4-difluorbenzyl)-1H-pyrazolo[3,4-b]pyridin-3-yl]pyrimidin-5-yl}-2-methylpropanoat

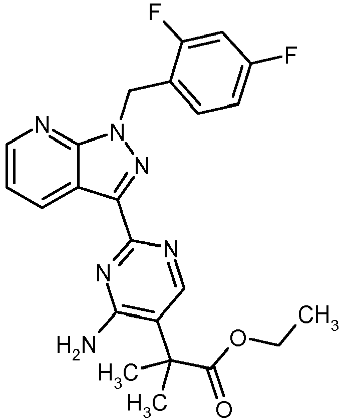

Die unter Beispiel 49A erhaltene Lösung wurde in Analogie zur Vorschrift unter Beispiel 19A hydriert. Die so erhaltene Titelverbindung (0.755 g, ca. 76%-ige Reinheit) wurde ohne weitere Reinigung in der nächsten Stufe eingesetzt.

LC-MS (Methode 1): Rₜ = 0.91 min; MS (ESIpos): m/z = 453 (M+H)⁺

### Beispiel 51A

### 2-Methoxy-4-methyl-6-oxo-1,4,5,6-tetrahydropyridin-3-carbonitril

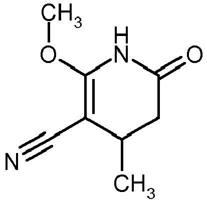

Die Synthese der Verbindung ist beschrieben: Heterocycles, 1985; 1135 - 1141.

### Beispiel 52A

### 2-Methoxy-6-oxo-4-(trifluormethyl)-1,4,5,6-tetrahydropyridin-3-carbonitril

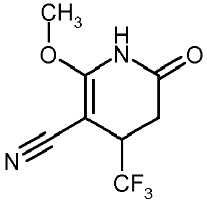

7.47 g (138.39 mmol) Natriummethanolat in Methanol (85 ml) wurden unter Eiskühlung vorgelegt und portionsweise mit 6.04 g (91.44 mmol) Malonodinitril versetzt. Anschliessend wurden unter Rühren 11.84 g (76.84 mmol) Methyl 4,4,4-trifluorocrotonat zugetropft, 30 min bei RT gerührt und danach 1 h zum Rückfluss erhitzt. Im Anschluss wurde im Vakuum bis zur Trockene eingeengt. Der Rückstand wurde mit Wasser versetzt und viermal mit Essigsäureethylester extrahiert. Die vereinigten organischen Phasen wurden mit Natriumsulfat getrocknet, filtriert und eingeengt. Weitere Reinigung erfolgte durch Chromatographie an Kieselgel (Cyclohexan/ Essigsäureethylester 3:1). Man erhielt 1.95 g der Titelverbindung (11 % d. Th.).

LC-MS (Methode 1): Rₜ = 0.61 min; MS (ESIpos): m/z = 221 (M+H)⁺

### Beispiel 53A

### 4-Amino-2-[1-(2-fluorbenzyl)-1H-pyrazolo[3,4-b]pyridin-3-yl]-5-methyl-5,8-dihydropyrido[2,3-d]pyrimidin-7(6H)-on

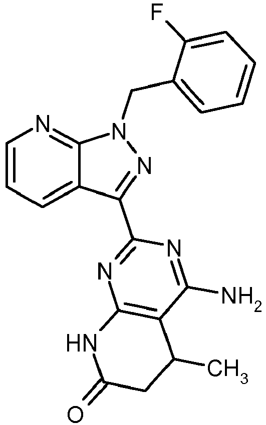

2.174 g (7.112 mmol) Beispiel 1A und 1.3 g (7.823 mmol) Beispiel 51A wurden in 20 ml Methanol vorgelegt und anschliessend bei RT portionsweise mit 422 mg (7.823 mmol) Natriummethanolat versetzt. Man rührte für 10 min bei RT und erhitzte anschliessend über Nacht zum Rückfluss. Nach Abkühlen wurde der Ansatz mit Essigsäure (0,5 ml) und Wasser (20 ml) versetzt und im Eisbad gekühlt. Der Niederschlag wurde abgesaugt, mit Wasser und Methanol gewaschen und anschliessend im Hochvakuum getrocknet. Es wurden 2.51 g der Titelverbindung erhalten (87 % d. Th.).

LC-MS (Methode 1): Rₜ = 0.85 min; MS (ESIpos): m/z = 404 (M+H)⁺

¹H-NMR (400 MHz, DMSO-d₆): δ [ppm] = 1.04 (d, 3H), 2.31 (d, 1H), 2.79 (dd, 1H), 3.13-3.19 (m, 1H), 5.81 (s, 2H), 6.93 (br s, 2H), 7.12-7.25 (m, 3H), 7.34-7.37 (m, 2H), 8.62 (dd, 1H), 9.14 (dd, 1H), 10.56 (s, 1H).

### Beispiel 54A

### 4-Amino-2-[1-(2-fluorbenzyl)-1H-pyrazolo[3,4-b]pyridin-3-yl]-5-(trifluormethyl)-5,8-dihydropyrido[2,3-d]pyrimidin-7(6H)-on

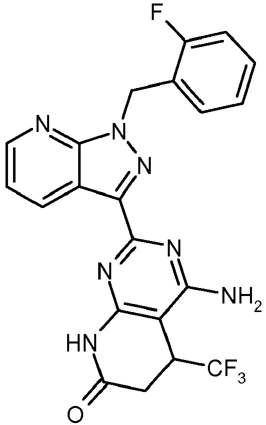

694 mg (2.271 mmol) Beispiel 1A und 500 mg (2.271 mmol) Beispiel 14A wurden in 10 ml tert.-Butanol vorgelegt und anschliessend bei RT portionsweise mit 305 mg (2.725 mmol) Kalium-tert.-butylat versetzt. Man rührte für 10 min bei RT und erhitzte anschliessend für 2 Tage zum Rückfluss. Nach Abkühlen wurde das Reaktionsgemisch mit Wasser und Essigsäureethylester versetzt. Der Niederschlag wurde abgesaugt. Das Filtrat wurde eingeengt, mit wenig Essigsäureethylester und Diethylether versetzt und der entstandene Niederschlag abgesaugt. Die vereinigten Feststofffraktionen wurden anschliessend im Hochvakuum getrocknet. Es wurden 588 mg der Titelverbindung erhalten (53 % d. Th.).

LC-MS (Methode 1): Rₜ = 0.92 min; MS (ESIpos): m/z = 458 (M+H)⁺

1H-NMR (400 MHz, DMSO-d6): δ [ppm] = 2.63 (d, 1H), 3.19 (dd, 1H), 4.16-4.20 (m, 1H), 5.83 (s, 2H), 7.13-7.40 (m, 7H), 8.63 (dd, 1H), 9.15 (dd, 1H), 10.85 (s, 1H).

### Beispiel 55A

### 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on

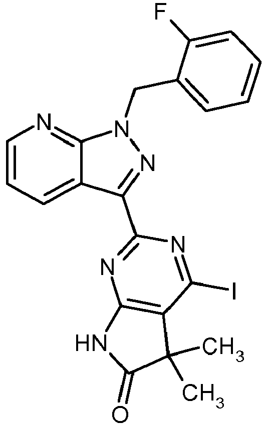

5.00 g (12.394 mmol) von Beispiel 13A wurden in iso-Pentylnitrit (35.87 ml) und Diiodmethan (1.16 mol, 93.71 ml) vorgelegt und und für 12 h auf 85°C erhitzt. Nach Abkühlen wurde von Feststoffen filtriert, eingeengt und anschliessend durch Chromatographie an Kieselgel gereinigt (Laufmittel: erst Cyclohexan-Dichlormethan Gradient, dann Dichlormethan-Methanol Gradient). Es wurden 5.50 g der Titelverbindung erhalten (67% d. Th.).

LC-MS (Methode 1): Rₜ = 1.19 min; MS (ESIpos): m/z = 515 (M+H)⁺

¹H-NMR (400 MHz, DMSO-d₆): δ [ppm] = 1.42 (s, 6H), 5.88 (s, 2H), 7.13-7.26 (m, 3H), 7.34-7.38 (m, 1H), 7.48 (dd, 1H), 8.69 (dd, 1H), 8.79 (dd, 1H), 11.78 (s br, 1H).

### Beispiel 56A

### 2-[5-Fluor-1-(2-fluorbenzyl)-1H-pyrazolo[3,4-b]pyridin-3-yl]-4-iod-5,5-dimethyl-5,7-dihydro-6H-pyrrolo[2,3-d]pyrimidin-6-on

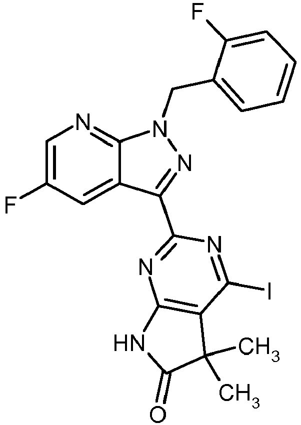

3.325 g (7.890 mmol) von Beispiel 14A wurden in Analogie zu Beispiel 55A umgesetzt. Es wurden 3.65 g der Titelverbindung erhalten (87 % d. Th., 61 % Reinheit).

LC-MS (Methode 1): Rₜ = 1.26 min; MS (ESIpos): m/z = 533 (M+H)⁺

¹H-NMR (400 MHz, DMSO-d₆): δ [ppm] = 1.42 (s, 6H), 5.87 (s, 2H), 7.14-7.26 (m, 3H), 7.37 (m, 1H), 8.48 (dd, 1H), 8.77 (dd, 1H), 11.76 (s br, 1H).

### Beispiel 57A

### 2'-[1-(2-Fluorbenzyl)-1H-pyrazolo[3,4-b]pyridin-3-yl]-4'-iod-4,5-dihydrospiro[furan-3,5'-pyrrolo[2,3-d]pyrimidin]-6'(7'H)-on

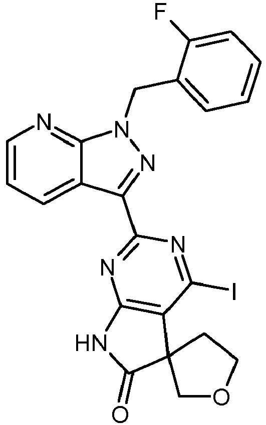

563 mg (1.305 mmol) der unter Beispiel 15A erhaltenen Verbindung wurden in 1,2-Dimethoxyethan (7.5 ml) vorgelegt und anschliessend mit 339 mg (1.305 mmol) Cäsiumiodid, 165 mg (0.652 mmol) Iod und 74 mg (0.391 mmol) Kupfer-(I)-iodid versetzt. Danach wurde iso-Pentylnitrit (1.04 ml) zugegeben und für 2 Tage auf 60°C erhitzt. Nach Abkühlen wurde filtriert, der Filterkuchen mit Essigsäureethylester gewaschen und das Filtrat zweimal mit 5%iger wässriger NatriumthiosulfatLösung und einmal mit gesättigter wässriger Natriumchlorid-Lösung gewaschen. Anschliessend wurde die organische Phase über Natriumsulfat getrocknet, filtriert und bis zur Trockene eingeengt. Der Rückstand wurde mittels präparativer HPLC gereinigt (Acetonitril/Wasser (+0.05 % Ameisensäure) - Gradient). Es wurden 98 mg der Titelverbindung erhalten (14% d. Th.).

LC-MS (Methode 1): Rₜ = 1.07 min; MS (ESIpos): m/z = 543 (M+H)⁺

¹H-NMR (400 MHz, DMSO-d₆): δ [ppm] = 2.23-2.29 (m, 1H), 2.50 (1H, teilweise unter DMSO peak), 3.93 (d, 1H), 4.04 (q, 1H), 4.16 (d, 1H), 4.21-4.27 (m, 1H), 5.88 (s, 2H), 7.13-7.26 (m, 3H), 7.34-7.40 (m, 1H), 7.49 (dd, 1H), 8.69 (dd, 1H), 8.80 (dd, 1H), 11.80 (s br, 1H).

### Beispiel 58A

### 2-[1-(2-Fluorbenzyl)-1H-pyrazolo[3,4-b]pyridin-3-yl]-4-iod-5-methyl-5,8-dihydropyrido[2,3-d]pyrimidin-7(6H)-on

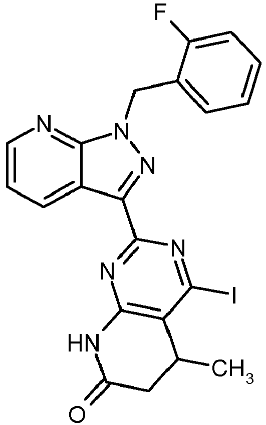

530 mg (1.314 mmol) von Beispiel 53A wurden analog zur Vorschrift unter Beispiel 57A umgesetzt. Es wurden 171 mg der Titelverbindung erhalten (25 % d. Th.).

LC-MS (Methode 1): Rₜ = 1.13 min; MS (ESIpos): m/z = 515 (M+H)⁺

¹H-NMR (400 MHz, DMSO-d₆): δ [ppm] = 1.12 (d, 3H), 2.46 (Signal teilweise unter Lösungmittel, 1H), 3.01 (dd, 1H), 3.16-3.19 (m, 1H), 5.88 (s, 2H), 7.13-7.14 (m, 2H), 7.24 (t, 1H), 7.34-7.38 (m, 1H), 7.45 (dd, 1H), 8.67 (dd, 1H), 9.09 (dd, 1H), 11.33 (s, 1H).

### Beispiel 59A

### 2-[1-(2-Fluorbenzyl)-1H-pyrazolo[3,4-b]pyridin-3-yl]-4-iod-5-(trifluormethyl)-5,8-dihydro-pyrido[2,3-d]pyrimidin-7(6H)-on

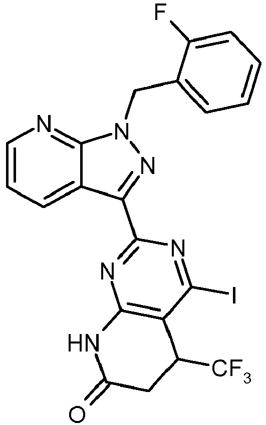

556 mg (1.216 mmol) von Beispiel 54A wurden in Analogie zu Beispiel 57A umgesetzt. Es wurden 605 mg der Titelverbindung erhalten (87 % d. Th.).

LC-MS (Methode 1): Rₜ = 1.15 min; MS (ESIpos): m/z = 569 (M+H)⁺

¹H-NMR (400 MHz, DMSO-d₆): δ [ppm] = 2.74 (d, 1H), 3.38 (dd, 1H), 4.17-4.24 (m, 1H), 5.90 (s, 2H), 7.14-7.17 (m, 2H), 7.24 (t, 1H), 7.33-7.40 (m, 1H), 7.48 (dd, 1H), 8.69 (dd, 1H), 9.01 (dd, 1H), 11.60 (s, 1H).

### Beispiel 60A

### 5-Fluor-1-(2-fluorbenzyl)-1H-pyrazolo[3,4-b]pyridin-3-carboximidohydrazid

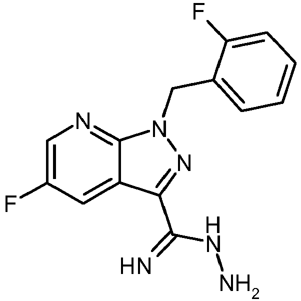

23.000 g (66.22 mmol) Beispiel 9A wurden in 322 ml Ethanol gelöst und bei 0°C mit 26.804 g (264.88 mmol) Triethylamin sowie 6.027 g (66.22 mmol) Hydrazinhydrat (55%-ige Lösung in Wasser) versetzt. Das Gemisch wurde über Nacht bei RT gerührt und anschließend auf 1.715 l einer 10%-igen wässrigen Natriumchlorid-Lösung gegeben und zweimal mit Essigsäureethylester extrahiert. Die vereinigten organischen Phasen wurden mit 10%-iger wässriger Natriumchlorid-Lösung gewaschen, über Natriumsulfat getrocknet und am Rotationsverdampfer eingeengt. Der Rückstand wurde an Kieselgel gereinigt (Eluent: Dichlormethan/Methanol, 95:5). Es wurden 15.000 g (75 % d. Th.) der Titelverbindung erhalten.

LC-MS (Methode 1): Rₜ = 0.58 min; MS (ESIpos): m/z = 303 (M+H)⁺

¹H-NMR (400 MHz, DMSO-d₆): δ [ppm] = 5.38 (s, 2H), 5.54 (s, 2H), 5.72 (s, 2H), 7.10-7.15 (m, 2H), 7.20-7.25 (m, 1H), 7.32-7.38 (m, 1H), 8.21 (dd, 1H), 8.64 (dd, 1H).

### Beispiel 61A

### Methyl-2-{3-[5-fluor-1-(2-fluorbenzyl)-1H-pyrazolo[3,4-b]pyridin-3-yl]-5-hydroxy-1,2,4-triazin-6-yl}-2-methylpropanoat

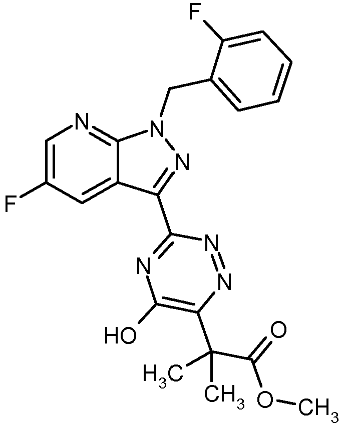

11.780 g (38.97 mmol) Beispiel 60A wurden in 353 ml Ethanol gelöst und mit 14.667 g (77.94 mmol) Dimethyl-2,2-dimethyl-3-oxobutandioat (beschrieben in J. Am. Chem. Soc. 124(14), 3680-3691; 2002) versetzt. Das Gemisch wurde über Nacht zum Rückfluß erhitzt. Nach dem Abkühlen wurde der Feststoff abgesaugt, mit wenig Ethanol gewaschen und das Filtrat eingeengt. Der Rückstand wurde an Kieselgel gereinigt (Eluent: Dichlormethan/Aceton, 95:5). Es wurden 10.000 g (58 % d. Th.) der Titelverbindung erhalten.

LC-MS (Methode 1): Rₜ = 1.07 min; MS (ESIpos): m/z = 441 (M+H)^{+ 1}H-NMR (400 MHz, DMSO-d₆): δ [ppm] = 1.45 (s, 6H), 3.56 (s, 3H), 5.91 (s, 2H), 7.16 (dt, 1H), 7.22-7.31 (m, 2H), 7.36-7.41 (m, 1H), 8.41 (dd, 1H), 8.83 (dd, 1H), 14.58 (s, 1H).

### Beispiel 62A

### 1-(2,3-Difluorbenzyl)-5-fluor-3-iod-1H-pyrazolo[3,4-b]pyridin

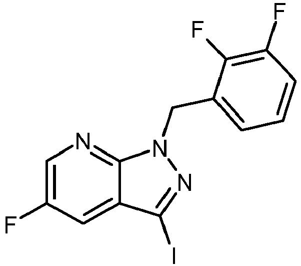

10.000 g (38.02 mmol) Beispiel 6A wurden in 270 ml Dimethylformamid gelöst und mit 8.658 g (41.82 mmol) 1-(Brommethyl)-2,3-difluorbenzol sowie 13.627 g (41.82 mmol) Cäsiumcarbonat versetzt. Es wurde 2 h bei Raumtemperatur gerührt und anschließend Essigsäureethylester sowie Wasser zugegeben. Die organische Phase wurde abgetrennt und die wässrige Phase dreimal mit Essigsäureethylester extrahiert. Die vereinigten organischen Phasen wurden über Natriumsulfat getrocknet und am Rotationsverdampfer eingeengt. Es wurden 8.460 g (56 % d. Th.) der Zielverbindung erhalten. Der Rückstand wurde ohne weitere Reinigung in der nächsten Stufe eingesetzt.

LC-MS (Methode 1): Rₜ = 1.24 min; MS (ESIpos): m/z = 390 (M+H)⁺

¹H-NMR (400 MHz, DMSO-d₆): δ [ppm] = 5.78 (s, 2H), 7.03-7.08 (m, 1H), 7.15-7.20 (m, 1H), 7.36-7.44 (m, 1H), 7.95 (dd, 1H), 8.72 (t, 1H).

### Beispiel 63A

### 1-(2,3-Difluorbenzyl)-5-fluor-1H-pyrazolo[3,4-b]pyridin-3-carbonitril

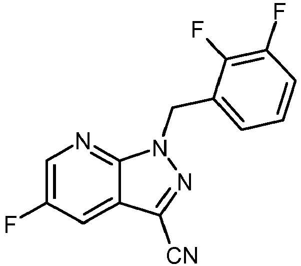

8.470 g (21.25 mmol) Beispiel 62A wurden in 59 ml Dimethylsulfoxid gelöst und mit 2.093 g (23.37 mmol) Kupfer(I)cyanid versetzt. Es wurde 1.5 h bei 150°C gerührt, anschließend mit Methanol verdünnt und über Celite filtriert. Es wurde mit Methanol nachgewaschen und das Filtrat am Rotationsverdempfer eingeengt. Der Rückstand wurde in Essigsäureethylester aufgenommen und zweimal mit einer Mischung aus gesättigter wässriger Ammoniomchlorid-Lösung und 25%-iger wässriger Ammoniaklösung (v/v = 3:1) sowie mit gesättigter wässriger Natriumchlorid-Lösung gewaschen. Die organische Phase wurde abgetrennt, über Natriumsulfat getrocknet und am Rotationsverdampfer eingeengt. Es wurden 5.750 g (89 % d. Th., Reinheit ca. 94 %) der Zielverbindung erhalten. Der Rückstand wurde ohne weitere Reinigung in der nächsten Stufe eingesetzt.

LC-MS (Methode 1): Rₜ = 1.32 min; MS (ESIpos): m/z = 289 (M+H)⁺

¹H-NMR (400 MHz, DMSO-d₆): δ [ppm] = 5.93 (s, 2H), 7.13-7.24 (m, 2H), 7.40-7.47 (m, 1H), 8.53 (dd, 1H), 8.88 (dd, 1H).

### Beispiel 64A

### 1-(2,3-Difluorbenzyl)-5-fluor-1H-pyrazolo[3,4-b]pyridin-3-carboximidamid-Acetat

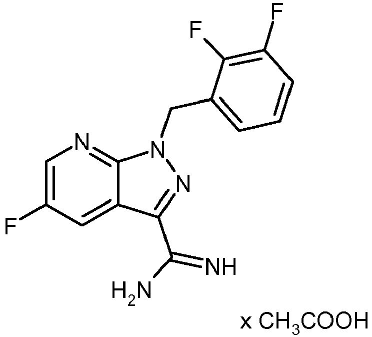

0.433 g (18.81 mmol) Natrium wurden portionsweise in 133 ml Methanol eingerührt. Nach beendeter Gasentwicklung wurden 5.750 g (18.81 mmol) Beispiel 63A portionsweise addiert und das Gemisch 2 h bei Raumtemperatur gerührt. Es wurden 1.208 g (22.57 mmol) Ammoniumchlorid und 4.394 g (73.17 mmol) Essigsäure zugegeben und das Gemisch über Nacht unter Rückfluss gekocht. Nach dem Abkühlen wurde der Ansatz am Rotationsverdampfer eingeengt und der Rückstand mit Essigsäureethylester und 1N Natronlauge versetzt, wobei sich ein Feststoff bildete. Der Feststoff wurde abgesaugt, mit Essigsäureethylester gewaschen und im Hochvakuum getrocknet. Es wurden 5.860 g (83 % d. Th.) der Zielverbindung erhalten.

LC-MS (Methode 1): Rₜ = 0.60 min; MS (ESIpos): m/z = 306 (M-C₂H₃O₂)⁺

¹H-NMR (400 MHz, DMSO-d₆): δ = 1.83 (s, 3H), 5.85 (s, 2H), 7.04 (t, 1H), 7.13 - 7.19 (m, 1H), 7.36-7.43 (m, 1H), 8.44 (dd, 1H), 8.45 (s br, 2H), 8.74 (s, 1H).

### Beispiel 65A

### 1-(2,3-Difluorbenzyl)-5-fluor-1H-pyrazolo[3,4-b]pyridin-3-carboximidohydrazid

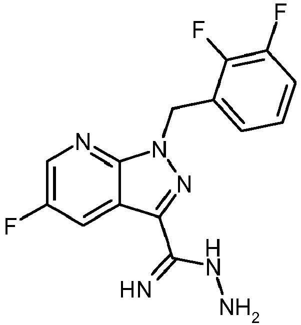

5.680 g (15.55 mmol) der Verbindung aus Beispiel 64A wurden in 76 ml Ethanol gelöst und bei 0°C mit 6.293 g (62.19 mmol) Triethylamin sowie 0.973 g (15.55 mmol) Hydrazinhydrat (80%-ige Lösung in Wasser) versetzt. Das Gemisch wurde über Nacht bei RT gerührt und anschließend Rotationsverdampfer eingeengt. Es wurden 5.850 g (99 % d. Th., Reinheit 84%) der Titelverbindung erhalten. Das Produkt wurde ohne weitere Reinigung in der nächsten Stufe eingesetzt.

LC-MS (Methode 1): Rₜ = 0.68 min; MS (ESIpos): m/z = 321 (M+H)⁺

### Beispiel 66A

### Methyl-2- {4-[1-(2,3-difluorbenzyl)-5-fluor-1H-pyrazolo[3,4-b]pyridin-3-yl]-2-hydroxyphenyl} -2-methylpropanoat

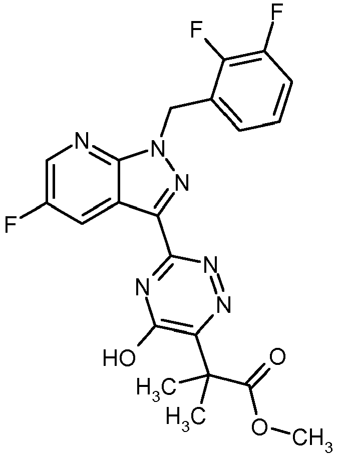

5.850 g (15.40 mmol) der Verbindung aus Beispiel 65A wurden in 140 ml Ethanol gelöst, mit 5.795 g (30.80 mmol) Dimethyl-2,2-dimethyl-3-oxobutandioat (beschrieben in J. Am. Chem. Soc. 124(14), 3680-3691; 2002) versetzt und über Nacht gerührt. Der enstandene Feststoff wurde abgesaugt, mit wenig Ethanol gewaschen und das Filtrat eingeengt. Der Rückstand wurde mit Diethylether verrührt, abgesaugt, mit wenig Diethylether gewaschen und im Hochvakuum getrocknet. Es wurden 2.060 g (29 % d. Th.) der Titelverbindung erhalten.

LC-MS (Methode 1): Rₜ = 1.03 min; MS (ESIpos): m/z = 459 (M+H)⁺

¹H-NMR (400 MHz, DMSO-d₆): δ [ppm] = 1.45 (s, 6H), 3.56 (s, 3H), 5.94 (s, 2H), 7.10-7.21 (m, 2H), 7.42 (q, 1H), 8.42 (dd, 1H), 8.83 (s, 1H), 14.53 (s br, 1H).

### Beispiel 67A

### 1-(2,4-Difluorbenzyl)-5-fluor-3-iod-1H-pyrazolo[3,4-b]pyridin

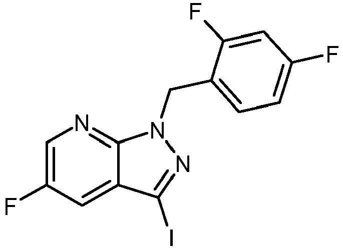

10.000 g (38.02 mmol) Beispiel 6A wurden in 270 ml Dimethylformamid gelöst und mit 8.658 g (41.82 mmol) 1-(Brommethyl)-2,4-difluorbenzol sowie 13.627 g (41.82 mmol) Cäsiumcarbonat versetzt. Es wurde 2 h bei Raumtemperatur gerührt und anschließend Essigsäureethylester sowie Wasser zugegeben. Die organische Phase wurde abgetrennt und die wässrige Phase dreimal mit Essigsäureethylester extrahiert. Die vereinigten organischen Phasen wurden über Natriumsulfat getrocknet und am Rotationsverdampfer eingeengt. Es wurden 8.340 g (53 % d. Th., Reinheit ca. 93 %) der Zielverbindung erhalten. Der Rückstand wurde ohne weitere Reinigung in der nächsten Stufe eingesetzt.

LC-MS (Methode 3): Rₜ = 1.45 min; MS (ESIpos): m/z = 390 (M+H)⁺

¹H-NMR (400 MHz, DMSO-d₆): δ [ppm] = 5.71 (s, 2H), 7.07 (dt, 1H), 7.27 (dt, 1H), 7.33-7.39 (m, 1H), 7.94 (dd, 1H), 8.72 (t, 1H).

### Beispiel 68A

### 1-(2,4-Difluorbenzyl)-5-fluor-1H-pyrazolo[3,4-b]pyridin-3-carbonitril

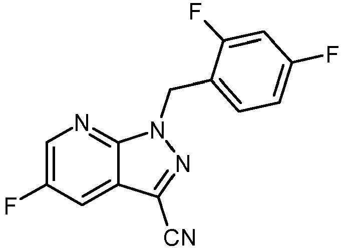

8.340 g (20.07 mmol) Beispiel 67A wurden in 56 ml Dimethylsulfoxid gelöst und mit 1.977 g (22.07 mmol) Kupfer(I)cyanid versetzt. Es wurde 1.5 h bei 150°C gerührt, anschließend mit Methanol verdünnt und über Celite filtriert. Es wurde mit Methanol nachgewaschen und das Filtrat am Rotationsverdempfer eingeengt. Der Rückstand wurde in Essigsäureethylester aufgenommen und zweimal mit einer Mischung aus gesättigter wässriger Ammoniomchlorid-Lösung und 25%-iger wässriger Ammoniaklösung (v/v = 3:1) sowie mit gesättigter wässriger Natriumchlorid-Lösung gewaschen. Die organische Phase wurde abgetrennt, über Natriumsulfat getrocknet und am Rotationsverdampfer eingeengt. Es wurden 5.270 g (83 % d. Th., Reinheit ca. 91%) der Zielverbindung erhalten. Der Rückstand wurde ohne weitere Reinigung in der nächsten Stufe eingesetzt.

LC-MS (Methode 1): Rₜ = 1.09 min; MS (ESIpos): m/z = 289 (M+H)⁺

¹H-NMR (400 MHz, DMSO-d₆): δ [ppm] = 5.85 (s, 2H), 7.10 (dt, 1H), 7.30 (dt, 1H), 7.45-7.51 (m, 1H), 8.52 (dd, 1H), 8.88 (t, 1H).

### Beispiel 69A

### 1-(2,4-Difluorbenzyl)-5-fluor-1H-pyrazolo[3,4-b]pyridin-3-carboximidamid-Acetat

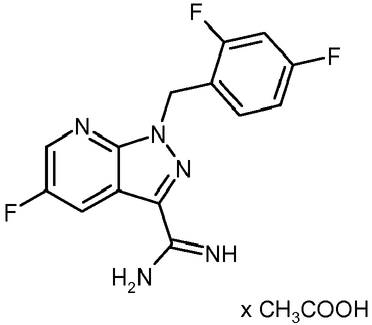

0.383 g (16.68 mmol) Natrium wurden portionsweise in 118 ml Methanol eingerührt. Nach beendeter Gasentwicklung wurden 5.270 g (ca. 16.68 mmol) Beispiel 68A portionsweise addiert und das Gemisch 2 h bei Raumtemperatur gerührt. Es wurden 1.070 g (20.01 mmol) Ammoniumchlorid und 3.895 g (64.86 mmol) Essigsäure zugegeben und das Gemisch über Nacht unter Rückfluss gekocht. Nach dem Abkühlen wurde der Ansatz am Rotationsverdampfer eingeengt und der Rückstand mit Essigsäureethylester und 1N Natronlauge versetzt, wobei sich ein Feststoff bildete. Der Feststoff wurde abgesaugt, mit Essigsäureethylester gewaschen und im Hochvakuum getrocknet. Es wurden 5.640 g (93 % d. Th.) der Zielverbindung erhalten.

LC-MS (Methode 1): Rₜ = 0.59 min; MS (ESIpos): m/z = 306 (M-C₂H₃O₂)⁺

¹H-NMR (400 MHz, DMSO-d₆): δ = 1.83 (s, 3H), 5.87 (s, 2H), 7.06 (t, 1H), 7.26 - 7.37 (m, 2H), 8.43 (dd, 1H), 8.73 (s, 1H).

### Beispiel 70A

### 1-(2,2-Difluorbenzyl)-5-fluor-1H-pyrazolo[3,4-b]pyridin-3-carboximidohydrazid

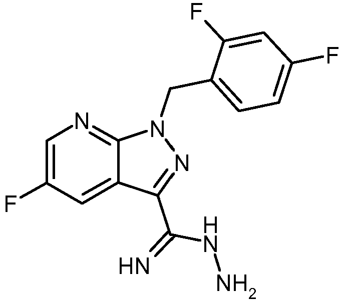

5.640 g (15.44 mmol) Beispiel 69A wurden in 76 ml Ethanol gelöst und bei 0°C mit 6.249 g (61.76 mmol) Triethylamin sowie 0.966 g (15.44 mmol) Hydrazinhydrat (80%-ige Lösung in Wasser) versetzt. Das Gemisch wurde über Nacht bei RT gerührt und anschließend Rotationsverdampfer eingeengt. Es wurden 5.30 g (100 % d. Th., Reinheit 93%) der Titelverbindung erhalten. Das Produkt wurde ohne weitere Reinigung in der nächsten Stufe eingesetzt.

LC-MS (Methode 1): Rₜ = 0.67 min; MS (ESIpos): m/z = 321 (M+H)⁺

### Beispiel 71A

### Methyl-2- {4-[1-(2,4-difluorbenzyl)-5-fluor-1H-pyrazolo[3,4-b]pyridin-3-yl]-2-hydroxyphenyl}-2-methylpropanoat

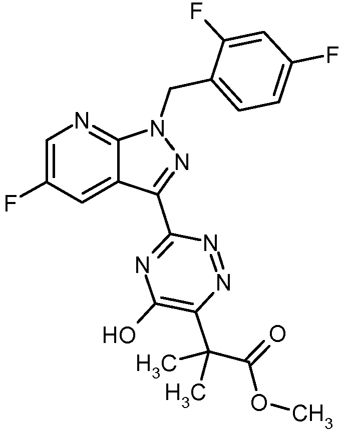

5.300 g (ca. 15.42 mmol) Beispiel 69A wurden in 140 ml Ethanol gelöst, mit 5.805 g (30.85 mmol) Dimethyl-2,2-dimethyl-3-oxobutandioat (beschrieben in J. Am. Chem. Soc. 124(14), 3680-3691; 2002) versetzt und über Nacht gerührt. Der enstandene Feststoff wurde abgesaugt, mit wenig Ethanol gewaschen und das Filtrat eingeengt. Der Rückstand wurde mit Diethylether verrührt, abgesaugt, mit wenig Diethylether gewaschen und im Hochvakuum getrocknet. Es wurden 2.290 g (32 % d. Th.) der Titelverbindung erhalten.

LC-MS (Methode 1): Rₜ = 1.03 min; MS (ESIpos): m/z = 459 (M+H)⁺

¹H-NMR (400 MHz, DMSO-d₆): δ [ppm] = 1.44 (s, 6H), 3.55 (s, 3H), 5.86 (s, 2H), 7.07 (dt, 1H), 7.30 (dt, 1H), 7.42 (q, 1H), 8.43 (dd, 1H), 8.81 (s, 1H), 14.45 (s br, 1H).

### Beispiel 72A

### 5-2-[1-(2-Fluorbenzyl)-1H-pyrazolo[3,4-b]pyridin-3-yl]-5H-pyrrolo[2,3-d]pyrimidin-5,6(7H)-dion-5-oxim

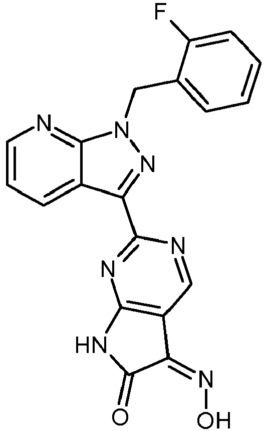

700 mg (1.943 mmol) Beispiel 4 wurden in Essigsäure (14 ml) suspendiert und mit 281 mg (4.079 mmol) Natriumnitrit und einigen Tropfen Wasser versetzt. Nach 30min bei RT wurde Wasser zugegeben und ein Niederschlag abfiltriert, mit Wasser nachgewaschen und anschliessend am Hochvakuum getrocknet. Man erhielt 756 mg der Titelverbindung (99 % d. Th.).

LC-MS (Methode 1): Rₜ = 0.90 min; MS (ESIpos): m/z = 390 (M+H)⁺

### Beispiel 73A

### 5-Amino-2-[1-(2-fluorbenzyl)-1H-pyrazolo[3,4-b]pyridin-3-yl]-5,7-dihydro-6H-pyrrolo[2,3-d]pyrimidin-6-on

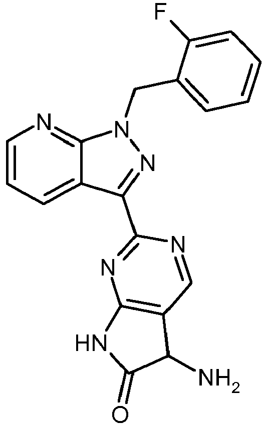

124 mg (0.319 mmol) Beispiel 72A wurden in Trifluoressigsäure (2.5 ml) vorgelegt und mit 41 mg (0.637 mmol) Zinkstaub versetzt und über Nacht bei RT gerührt. Anschliessend wurde eingeengt und das Rohmaterial (ca. 150 mg) ohne weitere Reiningung in der nächsten Stufe eingesetzt.

LC-MS (Methode 1): Rₜ = 0.69 min; MS (ESIpos): m/z = 376 (M+H)⁺

### Beispiel 74A

### 1-(2,3-Difluorbenzyl)-1H-pyrazolo[3,4-b]pyridin-3-carboximidohydrazid

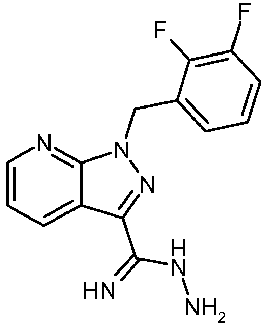

In Analogie zu Beispiel 70A wurden 10.00 g (28.791 mmol) Beispiel 36A umgesetzt. Es wurden 11.74 g der Titelverbindung in ca. 60%iger Reinheit erhalten (81 % d. Th.). Die Verbindung wurde ohne weitere Reinigung in der Folgestufe eingesetzt.

LC-MS (Methode 1): Rₜ = 0.57 min; MS (ESIpos): m/z = 303 (M+H)⁺

### Beispiel 75A

### Methyl-2-{3-[1-(2,3-difluorbenzyl)-1H-pyrazolo[3,4-b]pyridin-3-yl]-5-hydroxy-1,2,4-triazin-6-yl}-2-methylpropanoat

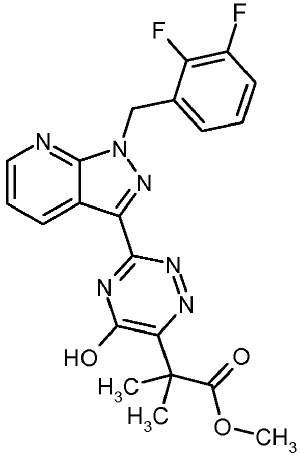

10.67 g (ca. 21.355 mmol) Beispiel 74A wurden in 300 ml Ethanol gelöst, mit 8.037 g (42.710 mmol) Dimethyl-2,2-dimethyl-3-oxobutandioat (beschrieben in J. Am. Chem. Soc. 124(14), 3680-3691; 2002) versetzt und über Nacht bei 50°C gerührt. Nach Abkühlen wurde von einem Niederschlag filtriert und dieser mit Ethanol gewaschen. Der Rückstand wurde eingeengt und anschliessend in Methanol (50 ml) und Acetonitril (50 ml) aufgenommen und mittels präparativer HPLC gereinigt (Acetonitril:Wasser - Gradient). Es wurden 0.75 g (8 % d. Th.) der Titelverbindung erhalten.

LC-MS (Methode 1): Rₜ = 0.98 min; MS (ESIpos): m/z = 441 (M+H)⁺

### Beispiel 76A

### 1-(2,4-Difluorbenzyl)-1H-pyrazolo[3,4-b]pyridin-3-carboximidohydrazid

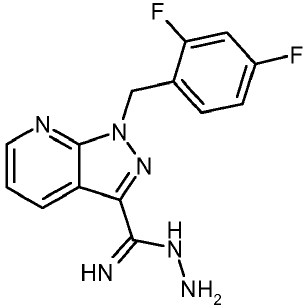

In Analogie zu Beispiel 70A wurden 10.00 g (28.791 mmol) Beispiel 31A umgesetzt. Es wurden 9.31 g der Titelverbindung in ca. 82%iger Reinheit erhalten (87 % d. Th.). Die Verbindung wurde ohne weitere Reinigung in der Folgestufe eingesetzt.

LC-MS (Methode 1): Rₜ = 0.56 min; MS (ESIpos): m/z = 303 (M+H)⁺

### Beispiel 77A

### Methyl-2- {3-[1-(2,4-difluorbenzyl)-1H-pyrazolo[3,4-b]pyridin-3-yl]-5-hydroxy-1,2,4-triazin-6-yl}-2-methylpropanoat

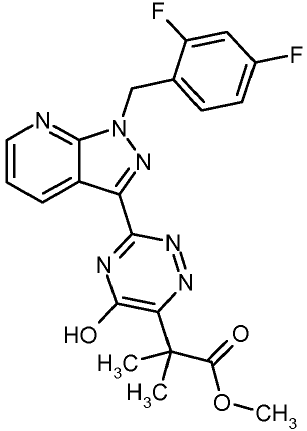

6.80 g (ca. 18.446 mmol) Beispiel 76A wurden in 272 ml Ethanol gelöst, mit 6.942 g (36.892 mmol) Dimethyl-2,2-dimethyl-3-oxobutandioat (beschrieben in J. Am. Chem. Soc. 124(14), 3680-3691; 2002) versetzt und über Nacht bei 50°C gerührt. Nach Abkühlen wurde von einem Niederschlag filtriert und dieser mit Ethanol gewaschen. Der Rückstand wurde in viel Ethanol aufgenommen, wonach sich erneut ein Feststoff bildete. Dieser wurde abfiltriert und mit Ethanol und Diethylether gewaschen. Der Feststoff wurde getrocknet und entspricht der Titelverbindung 2.21 g (26 % d. Th.). Das Filtrat wurde eingeengt und anschliessend mittels präparativer HPLC gereinigt (Acetonitril:Wasser:Wasser +1%TFA - 40:55:5). Es wurden zusätzlich 0.64 g (7 % d. Th.) der Titelverbindung erhalten. Insgesamt wurden 2.85 g (33 % d. Th.) der Titelverbindung erhalten.

LC-MS (Methode 1): Rₜ = 1.03 min; MS (ESIpos): m/z = 441 (M+H)⁺

### Beispiel 78A

### 5-Fluor-3-iod-1-(2,3,6-trifluorbenzyl)-1H-pyrazolo[3,4-b]pyridin

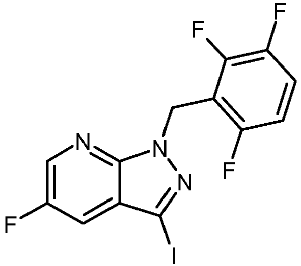

In DMF (170 ml) wurden 10.0 g (38.021 mmol) der Verbindung aus Beispiel 6A vorgelegt und anschließend 9.41 g (41.823 mmol) 2,3,6-Trifluorbenzylbromid sowie 13.62 g (41.82 mmol) Cäsiumcarbonat zugefügt. Die Mischung wurde über Nacht bei RT gerührt. Anschließend wurde die Reaktionsmischung auf Wasser (200 ml) gegeben und 15 min gerührt. Es wurde ein Niederschlag abgesaugt, welcher mit Wasser gewaschen und anschliessend im Hochvakuum getrocknet wurde. Es wurden 12.1 g der Titelverbindung erhalten (78 % d. Th.).

LC-MS (Methode 1): Rₜ = 1.24 min

MS (ESIpos): m/z = 408 (M+H)⁺

1H-NMR (400 MHz, DMSO-d₆): δ = 5.78 (s, 2H), 7.16 - 7.22 (m, 1H), 7.51 - 7.59 (m, 1H), 7.92 (dd, 1H), 8.73 (t, 1H).

### Beispiel 79A

### 5-Fluor-1-(2,3,6-trifluorbenzyl)-1H-pyrazolo[3,4-b]pyridin-3-carbonitril

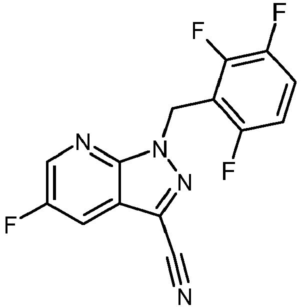

In Analogie zu Beispiel 8A wurden 12.1 g (29.72 mmol) Beispiel 78A umgesetzt. Es wurden 9.35 g der Titelverbindung in 79%iger Reinheit erhalten (81% d. Th.).

LC-MS (Methode 1): Rₜ = 1.08 min

MS (ESIpos): m/z = 307 (M+H)⁺ 1H-NMR (400 MHz, DMSO-d6): δ = 5.91 (s, 2H), 7.21 - 7.23 (m, 1H), 7.57 - 7.61 (m, 1H), 8.51 (dd, 1H), 8.89 (t, 1H).

### Beispiel 80A

### 5-Fluor-1-(2,3,6-trifluorbenzyl)-1H-pyrazolo[3,4-b]pyridin-3-carboximidamid-Acetat

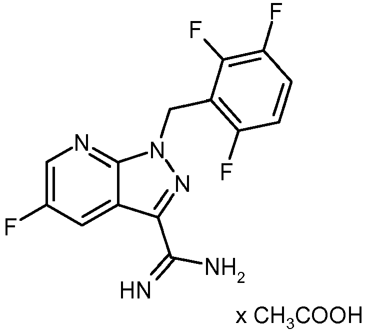

In Analogie zu Beispiel 9A wurden 9.1 g der Rohverbindung von Beispiel 79A umgesetzt. Es wurden 7.77g g der Titelverbindung erhalten (86% d. Th.).

LC-MS (Methode 1): Rₜ = 0.61 min

MS (ESIpos): m/z = 324 (M+H)⁺

### Beispiel 81A

### 5-Fluor-1-(2,3,6-trifluorbenzyl)-1H-pyrazolo[3,4-b]pyridin-3-carboximidohydrazid

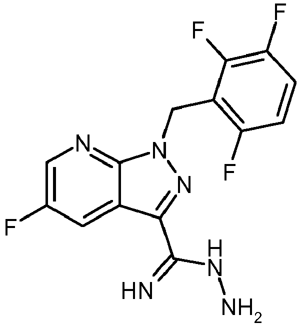

In Analogie zur Synthese von Beispiel 70A wurden 7.77 g (20.271 mmol) Beispiel 80A umgesetzt. Es wurden 6.85 g der Titelverbindung erhalten (99 % d. Th.). Die Verbindung wurde ohne weitere Reinigung in der Folgestufe eingesetzt.

LC-MS (Methode 1): Rₜ = 0.59 min; MS (ESIpos): m/z = 339 (M+H)⁺

### Beispiel 82A

### Methyl-2- {3-[5-fluor-1-(2,3,6-trifluorbenzyl)-1H-pyrazolo[3,4-b]pyridin-3-yl]-5-hydroxy-1,2,4-triazin-6-yl} -2-methylpropanoat

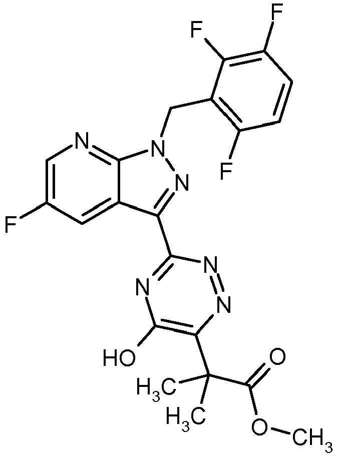

6.85 g (20.271 mmol) der Verbindung aus Beispiel 81A wurden in 300 ml Ethanol gelöst, mit 7.629 g (40.542 mmol) Dimethyl-2,2-dimethyl-3-oxobutandioat (beschrieben in J. Am. Chem. Soc. 124(14), 3680-3691; 2002) versetzt und über Nacht bei 50°C gerührt. Nach Abkühlen wurde von einem Niederschlag filtriert und dieser mit Ethanol gewaschen. Das Filtrat wurde eingeengt und anschliessend mittels präparativer HPLC gereinigt (Methanol:Wasser-75:25). Es wurden 3.30 g (33 % d. Th.) der Titelverbindung erhalten.

LC-MS (Methode 3): Rₜ = 1.24 min; MS (ESIpos): m/z = 477 (M+H)⁺

### Beispiel 83A

### Ethyl- {2-[5-fluor-1-(2-fluorbenzyl)-1H-pyrazolo[3,4-b]pyridin-3-yl]-4-hydroxypyrimidin-5-yl} acetat

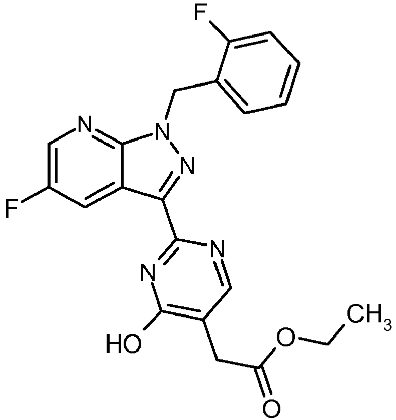

2.00 g (5.73 mmol) Beispiel 9A und 1.42 g (6.58 mmol) Diethyl-2-formylbutandioat (Synthese beschrieben in WO 2005/73234, Seite 43) wurden in 50 ml Ethanol vorgelegt. Anschließend wurden 4.27 ml Natriumethylat-Lösung (21%-ig in Ethanol, 11.5 mmol) zugetropft. Dann wurde 9h zum Rückfluss erhitzt. Nach dem Abkühlen wurde das Reaktionsgemisch mit 25 ml Wasser und danach mit 1N Salzsäure versetzt. Der sich bildende Niederschlag wurde abgesaugt und nacheinander mit Methanol (20 ml) und mit Diethylether (20 ml) gewaschen. Nach Trocknung im Hochvakuum wurden 1.53 g der Titelverbindung erhalten (63 % d. Th.).

LC-MS (Methode 1): Rₜ = 1.04 min; MS (ESIpos): m/z = 426 (M+H)⁺

¹H-NMR (400 MHz, DMSO-d₆): δ [ppm] = 1.19 (t, 3H), 3.48 (s, 2H), 4.09 (q, 2H), 5.86 (s, 2H), 7.16 (t, 1H), 7.24 (t, 1H), 7.31-7.34 (m, 2H), 8.11 (s br, 1H), 8.48 (d, 1H), 8.78 (s, 1H), 12.88 (s br, 1H).

### Beispiel 84A

### Ethyl- {4-chlor-2-[5-fluor-1-(2-fluorbenzyl)-1H-pyrazolo[3,4-b]pyridin-3-yl]pyrimidin-5-yl} acetat

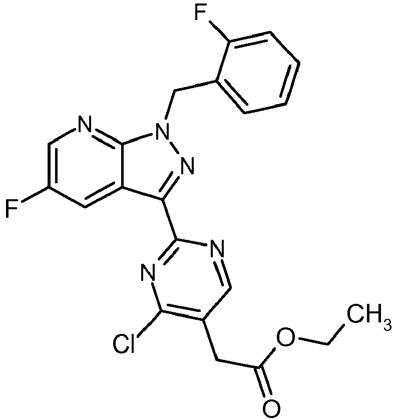

1.53 g (3.60 mmol) Beispiel 83A wurden in 3.35 ml (36.0 mmol) Phosphorylchlorid vorgelegt. Dann wurden 0.684 ml (5.40 mmol) N.N-Dimethylanilin addiert. Anschließend wurde 1h im Ölbad bei 150°C umgesetzt. Die flüchtigen Komponenten wurden am Rotationsverdampfer abgetrennt und dann wurde der Rückstand vorsichtig in 50 ml 2M wässrige Natriumcarbonat-Lösung eingerührt. Das Gemisch wurde 15 min gerührt und der Feststoff abfiltriert. Der Feststoff wurde in Essigsäureethylester aufgenommen, mit gesättigter wässriger Natriumchlorid-Lösung gewaschen und die organische Phase mit Magnesiumsulfat getrocknet. Das Lösungsmittel wurde bei vermindertem Druck destillativ abgetrennt und der Rückstand über Nacht im Hochvakuum getrocknet. So wurden 1.44 g der Titelverbindung erhalten (90 % d. Th.).

LC-MS (Methode 1): Rₜ = 1.28 min; MS (ESIpos): m/z = 444 (M+H)⁺

¹H-NMR (400 MHz, DMSO-d₆): δ [ppm] = 1.21 (t, 3H), 3.99 (s, 2H), 4.15 (q, 2H), 5.90 (s, 2H), 7.17 (dt, 1H), 7.21-7.31 (m, 2H), 7.38 (m, 1H), 8.55 (dd, 1H), 8.79 (dd, 1H), 8.96 (s, 1H).

### Beispiel 85A

### Ethyl-1-{4-chlor-2-[5-fluor-1-(2-fluorbenzyl)-1H-pyrazolo[3,4-b]pyridin-3-yl]pyrimidin-5-yl}cyclopropancarboxylat

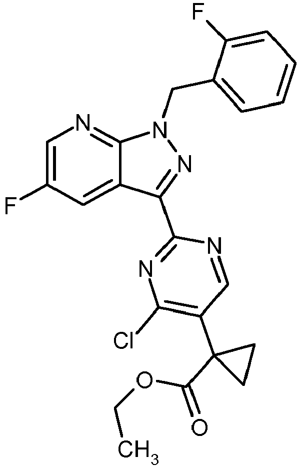

1.44 g (3.24 mmol) Beispiel 84A wurden in 40 ml THF/DMF (v/v = 1/1) vorgelegt. Dann wurden 410 mg (16.2 mmol, 95%-ig) Natriumhydrid portionsweise zugegeben. Die entstandene Lösung wurde ca. 30 min (bis zum Ausbleiben der Gasentwicklung) gerührt. Dann wurde 0.587 ml (6.81 mmol) 1,2-Dibromethan addiert und 1h bei Raumtemperatur gerührt. Das Reaktionsgemisch wurde mit 50 ml Wasser hydrolysiert und mit 50 ml Essigsäureethylester extrahiert. Dann wurde die organische Phase mit 50 ml Wasser gewaschen. Die vereinigten wässrigen Phasen wurden mit Essigsäureethylester (2x30 ml) extrahiert und anschließend die vereinigten organischen Phasen mit gesättigter wässriger Natriumchlorid-Lösung gewaschen. Nach dem Trocken über Magnesiumsulfat wurde das Lösungsmittel am Rotationsverdampfer abgetrennt. Man erhielt 1.51 g der Titelverbindung in ca. 80%-iger Reinheit (Ausbeute 79% d.Th.). Das Rohmaterial wurde ohne weitere Aufarbeitung in der Folgestufe eingesetzt.

LC-MS (Methode 1): Rₜ = 1.36 min; MS (ESIpos): m/z = 470 (M+H)⁺

¹H-NMR (400 MHz, DMSO-d₆): δ [ppm] = 1.10 (t, 3H), 1.46 (m, 2H), 1.65 (m, 2H), 4.08 (q, 2H), 5.90 (s, 2H), 7.17 (t, 1H), 7.21-7.29 (m, 2H), 7.37 (m, 1H), 8.53 (dd, 1H), 8.79 (m, 1H), 8.94 (s, 1H).

### Beispiel 86A

### Ethyl-1-{4-azido-2-[5-fluor-1-(2-fluorbenzyl)-1H-pyrazolo[3,4-b]pyridin-3-yl]pyrimidin-5-yl}cyclopropancarboxylat

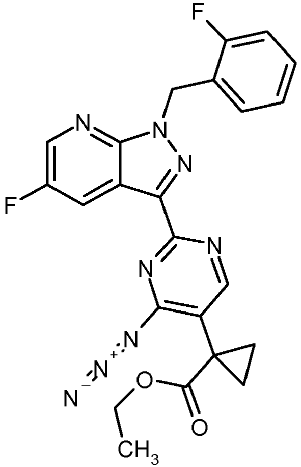

1.51 g (2.57 mmol) Beispiel 85A wurden in 30 ml DMF vorgelegt. Dann wurden 217 mg (3.34 mmol,) Natriumazid zugegeben. Anschließend wurde über Nacht bei 60°C gerührt. Nach dem Abkühlen wurde das Reaktionsgemisch mit 150 ml Wasser versetzt und mit Essigsäureethylester (3x70 ml) extrahiert. Dann wurden die vereinigten organischen Phasen mit gesättigter wässriger Natriumchlorid-Lösung gewaschen. Nach dem Trocken mit Magnesiumsulfat wurde das Lösemittel am Rotationsverdampfer abgetrennt. Man erhielt 1.15 g der Titelverbindung in ca. 68%-iger Reinheit (93% d. Th.) enthielt. Das Rohmaterial wurde ohne weitere Aufarbeitung in der Folgestufe eingesetzt.

LC-MS (Methode 1): Rₜ = 1.33 min; MS (ESIpos): m/z = 477 (M+H)⁺

### Beispiel 87A

### Ethyl-1-{4-amino-2-[5-fluor-1-(2-fluorbenzyl)-1H-pyrazolo[3,4-b]pyridin-3-yl]pyrimidin-5-yl}cyclopropancarboxylat

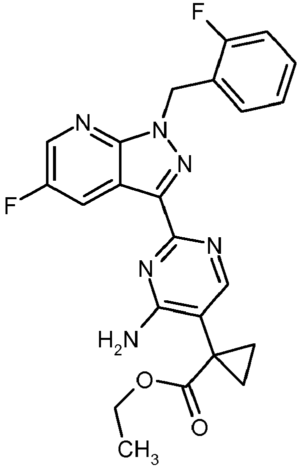

1.15 g (2.41 mmol) Beispiel 86A wurden in 30 ml DMF vorgelegt. Dann wurden 500 mg (10%-ig) Palladium auf Aktivkohle zugegeben. Anschließend wurde für 3h bei 1 Atmosphäre Wasserstoffdruck hydriert. Das Reaktionsgemisch wurde über Celite filtriert. Es wurde mit DMF nachgewaschen und die flüchtigen Komponenten am Rotationsverdampfer abgetrennt. Das so erhaltene Rohmaterial wurde am Hochvakuum über Nacht getrocknet. So wurden 1.28 g Rohmaterial erhalten, welches die Titelverbindung in ca. 90 %-iger Reinheit (LC-MS) enthielt. Das so erhaltene Material wurde ohne weitere Aufreinigung eingesetzt.

LC-MS (Methode 3): Rₜ = 1.11 min; MS (ESIpos): m/z = 451 (M+H)⁺

### Ausführungsbeispiele:

### Beispiel 1

### 2-[1-(2-Fluorbenzyl)-1H-pyrazolo[3,4-b]pyridin-3-yl]-5,5-dimethyl-5,7-dihydro-6H-pyrrolo[2,3-d]pyrimidin-6-on

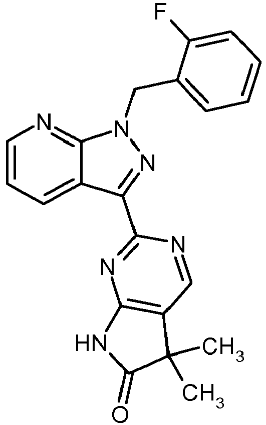

1.00 g (1.944 mmol) von Beispiel 55A wurden in in DMF (50 ml) mit 425 mg Palladium auf Kohle (10 %) versetzt und 4 h bei Wasserstoff-Normaldruck hydriert. Anschliessend wurde über Celite filtriert und bis zur Trockene eingeengt. Der Rückstand wurde mittels präparativer HPLC gereinigt (Acetonitril:Wasser (+0.05 % Ameisensäure) - Gradient). Es wurden 500 mg der Titelverbindung erhalten (66% d. Th.).

LC-MS (Methode 1): Rₜ = 1.00 min; MS (ESIpos): m/z = 389 (M+H)⁺

¹H-NMR (400 MHz, DMSO-d₆): δ [ppm] = 1.38 (s, 6H), 5.86 (s, 2H), 7.13-7.17 (m, 1H), 7.21-7.26 (m, 2H), 7.34-7.39 (m, 1H), 7.44 (dd, 1H), 8.64 (s, 1H), 8.67 (dd, 1H), 8.87 (dd, 1H), 11.61 (s br, 1H).

### Beispiel 2

### 2-[5-Fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

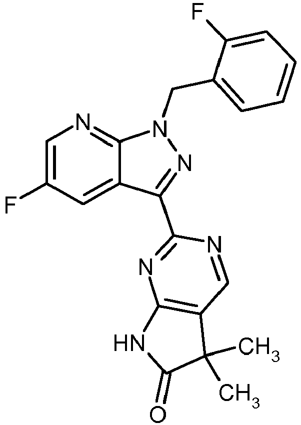

1.74 g (ca. 1.944 mmol) von Beispiel 56A wurden in Analogie zu Beispiel 1 umgesetzt. Es wurden 644 mg der Titelverbindung erhalten (78 % d. Th.).

LC-MS (Methode 1): Rₜ = 1.10 min; MS (ESIpos): m/z = 407 (M+H)⁺

¹H-NMR (400 MHz, DMSO-d₆): δ [ppm] = 1.38 (s, 6H), 5.85 (s, 2H), 7.14-7.18 (m, 1H), 7.21-7.28 (m, 2H), 7.35-7.40 (m, 1H), 8.59 (dd, 1H), 8.63 (s, 1H), 8.75 (dd, 1H), 11.58 (s br, 1H).

### Beispiel 3

### 2'-[1-(2-Fluorbenzyl)-1H-pyrazolo[3,4-b]pyridin-3-yl]-4,5-dihydrospiro[furan-3,5'-pyrrolo[2,3-d]pyrimidin]-6'(7'H)-on

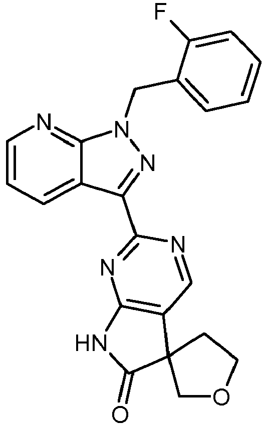

96 mg (0.177 mmol) von Beispiel 57A wurden in Analogie zu Beispiel 1 umgesetzt. Es wurden 51 mg der Titelverbindung erhalten (68 % d. Th.).

LC-MS (Methode 1): Rₜ = 0.92 min; MS (ESIpos): m/z = 417 (M+H)⁺

¹H-NMR (400 MHz, DMSO-d₆): δ [ppm] = 2.23-2.29 (m, 1H), 2.34-2.41 (m, 1H), 3.89 (d, 1H), 3.97 (d, 1H), 4.07-4.11 (m, 1H), 4.12-4.18 (m, 1H), 5.86 (s, 2H), 7.13-7.17 (t, 1H), 7.21-7.26 (m, 2H), 7.34-7.39 (m, 1H), 7.44 (dd, 1H), 8.57 (s, 1H), 8.67 (dd, 1H), 8.87 (dd, 1H), 11.72 (s br, 1H).

### Beispiel 4

### 2-[1-(2-Fluorbenzyl)-1H-pyrazolo[3,4-b]pyridin-3-yl]-5,7-dihydro-6H-pyrrolo[2,3-d]pyrimidin-6-on

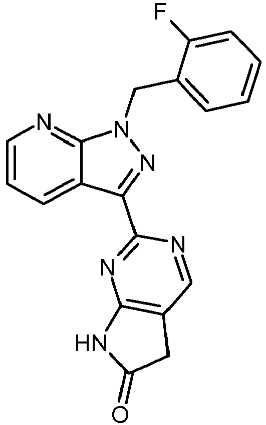

9.46 g (23.276 mmol) Beispiel 19A wurden in THF (400 ml) vorgelegt und mit 2.612 g (23.726 mmol) Kalium-tert.-butylat versetzt. Es wurde für 1 h bei RT gerührt und anschliessend mit Wasser versetzt und mit Essigsäure auf pH=5 eingestellt und danach 10 min bei RT gerührt. Im Anschluss daran wurde dreimal mit Essigsäureethylester extrahiert und die vereinigten organischen Phasen mit gesättigter wässriger Natriumchlorid-Lösung gewaschen. Danach wurde die organische Phase über Natriumsulfat getrocknet, filtriert und bis zur Trockene eingeengt. Der Rückstand wurde in Methanol aufgeschlämmt und abgesaugt. Der Filterkuchen wurde mehrmals mit Methanol gewaschen und anschliessend im Hochvakuum getrocknet. Man erhielt 6.61 g der Titelverbindung als Feststoff (78 % d. Th.).

LC-MS (Methode 1): Rₜ = 0.82 min; MS (ESIpos): m/z = 361 (M+H)⁺

¹H-NMR (400 MHz, DMSO-d₆): δ [ppm] = 3.68 (s, 2H), 5.85 (s, 2H), 7.14-7.18 (m, 1H), 7.21-7.27 (m, 2H), 7.34-7.38 (m, 1H), 7.42 (dd, 1H), 8.49 (s, 1H), 8.67 (dd, 1H), 8.88 (dd, 1H), 11.58 (s, 1H).

### Beispiel 5

### 2'-[1-(2-Fluorbenzyl)-1H-pyrazolo[3,4-b]pyridin-3-yl]spiro[cyclopropan-1,5'-pyrrolo[2,3-d]pyrimidin]-6'(7'H)-on

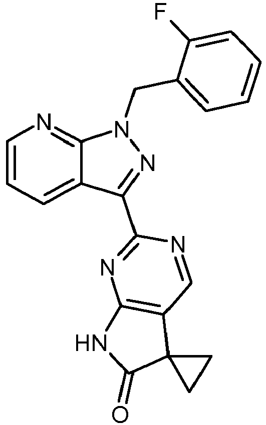

394 mg (0.913 mmol) von Beispiel 22A wurden in Analogie zur Vorschrift unter Beispiel 4 umgesetzt. Es wurden 186 mg der Titelverbindung erhalten (53 % d. Th.).

LC-MS (Methode 1): Rₜ = 0.95 min; MS (ESIpos): m/z = 387 (M+H)⁺

¹H-NMR (400 MHz, DMSO-d₆): δ [ppm] = 1.64 (ddd, 2H), 1.86 (ddd, 2H), 5.85 (s, 2H), 7.16 (t, 1H), 7.22-7.27 (m, 2H), 7.34-7.38 (m, 1H), 7.42 (dd, 1H), 8.39 (s, 1H), 8.67 (dd, 1H), 8.87 (dd, 1H), 11.78 (s br, 1H).

### Beispiel 6

### 3-[1-(2-Fluorbenzyl)-1H-pyrazolo[3,4-b]pyridin-3-yl]-5,7-dihydro-6H-pyrrolo[2,3-e][1,2,4]triazin-6-on

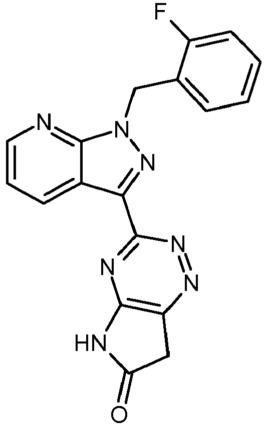

### Stufe (a): Methyl-{3-[1-(2 fluorbenzyl)-1H-pyrazolo[3,4-b]pyridin-3yl]-5-hydroxy-1,2,4-triazin-6-yl}acetat

5.950 g (14.441 mmol) der Verbindung aus Beispiel 23A wurden in 70 ml Ethanol gelöst und mit 3.351 g (20.929 mmol) Dimethyl-2-oxobutandioat (beschrieben in Synth. Commun. 9(7), 603-7; 1979) versetzt. Das Gemisch wurde über Nacht zum Rückfluß erhitzt. Nach dem Abkühlen wurde der Feststoff abgesaugt, mit wenig Ethanol gewaschen und im Hochvakuum getrocknet (Reinheit nach LC/MS = 43%).

### Stufe (b): Methyl-{5-amino-3-[1-(2-fluorbenzyl)-1H-pyrazolo[3,4-b]pyridin-3-yl]-1,2,4-triazin-6-yl)acetat

1.100 g (1.199 mmol) des Zwischenprodukts aus Stufe a) wurden mit 14 ml Phosphorylchlorid versetzt und 1.75 h bei 100°C gerührt. Nach dem Abkühlen wurde das Reaktionsgemisch in 100 ml konzentrierte Ammoniak-Lösung eingerührt. Es wurde 20 Min. bei RT nachgerührt. Der entstandene Niederschlag wurde abfiltriert und mit wenig Wasser gewaschen und im Hochvakuum getrocknet.

### Stufe (c): 3-[1-(2-Fluorbenzyl}-1H-pyrazolo[3,4-b]pyridin-3-yl]-5,7-dihydro-6H pyrrolo[2,3-e][1,2,4]triazin-6-on

Das Zwischenprodukt aus Stufe b) wurde in 350 ml Methanol gelöst und mit 20 Tropfen einer 1 N Natronlauge versetzt (pH-Wert = 6). Das Gemisch wurde über Nacht bei RT gerührt und am Rotationsverdampfer eingeengt. Der Rückstand wurde an Kieselgel gereinigt (Eluent: Dichlormethan/Methanol, 96:4). Es wurden 67 mg (15% d. Th.) der Titelverbindung erhalten.

LC-MS (Methode 1): Rₜ = 0.83 min; MS (EIpos): m/z = 362 [M+H]⁺.

¹H-NMR (400 MHz, DMSO-d₆): δ [ppm] = 5.03 (s, 1H), 5.82 (s, 2H), 7.14-7.18 (m, 1H), 7.21-7.25 (m, 2H), 7.34-7.42 (m, 2H), 8.65-8.67 (m, 2H), 11.53 (s br, 1H), 13.55 (s br, 1H).

### Beispiel 7

### 4-(Difluormethyl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

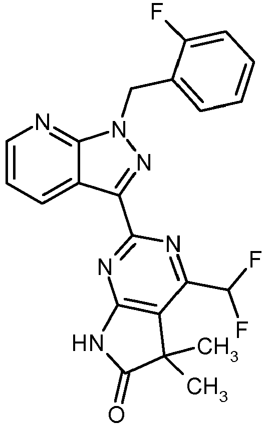

Das Rohprodukt aus Beispiel 28A (1.24 g, ca. 1.312 mmol) wurde analog der Vorschrift unter Beispiel 19A hydriert. Danach wurde über Celite filtriert und eingeengt. Der Rückstand wurde mittels präparativer HPLC gereinigt (Eluent: Acetonitril/Wasser mit 0.1% Ameisensäure). Es wurden 101 mg der Titelverbindung erhalten (17% d. Th.).

LC-MS (Methode 1): Rₜ = 1.07 min; MS (EIpos): m/z = 439 [M+H]⁺.

H-NMR (400 MHz, DMSO-d₆): δ [ppm] = 1.44 (s, 6H), 5.89 (s, 2H), 7.08-7.40 (m, 5H), 7.47 (dd, 1H), 8.69 (dd, 1H), 8.92 (dd, 1H), 12.00 (s br, 1H).

### Beispiel 8

### 2'-[1-(2,4-Difluorbenzyl)-1H-pyrazolo[3,4-b]pyridin-3-yl]spiro[cyclopropan-1,5'-pyrrolo[2,3-d]pyrimidin]-6'(7'H)-on

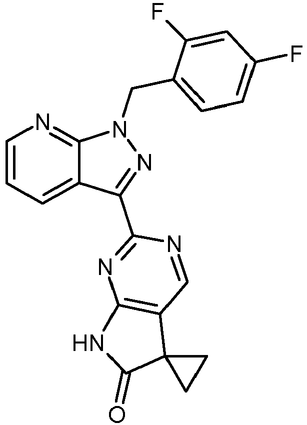

### Stufe (a): Ethyl-1-{4-chlor-2-[1-(2,4-difluorbenzyl)-1H-pyrazolo[3,4-b]pyridin-3-yl]pyrimidin-5-yl}cyclopropancarboxylat

0.80 g (1.802 mmol) von Beispiel 33A wurden in 15 ml DMF unter Argon gelöst, mit 360 mg (9.012 mmol) Natriumhydrid (60%ige Suspension in Mineralöl) versetzt und bei RT 15 Min. gerührt. Anschließend wurden 1.015 g (5.407 mmol) 1,2-Dibromethan addiert und 2 h bei RT gerührt. Es wurde Wasser sowie gesättigte wässrige Natriumchlorid-Lösung addiert und das Gemisch drei mal mit Essigsäureethylesster extrahiert. Die vereinigten organischen Phasen wurden über Natriumsulfat getrocknet und am Rotationsverdampfer eingeengt. Es wurden 848 mg (Reinheit 54%, 54% d. Th.) des Zwischenprodukts erhalten

### Stufe (b): Ethyl-1-{4-amino-2-[1-(2,4-difluorbenzyl}-1H-pyrazolo[3,4-b]pyridin-3-yl]pyrimidin-5-yl}cyclopropancarboxylat

Das Zwischenprodukt aus Stufe a) wurde in 13 ml DMF gelöst und mit 95 mg (1.461 mmol) Natriumazid versetzt. Das Reaktionsgemisch wurde für 7 h bei 60°C gerührt und nach dem Abkühlen auf Wasser gegeben. Es wurde drei mal mit Essigsäureethylester extrahiert. Die vereinigten organischen Phasen wurden mit gesättigter wässriger Natriumchloridlösung gewaschen, über Natriumsulfat getrocknet, mit 10 ml DMF versetzt und der Essigsäureethylester bei 80 mbar am Rotationsverdampfer entfernt. Die produkthaltige DMF-Lösung wurde mit 20 ml DMF versetzt und mit 250 mg Palladium (10 % auf Kohle) versetzt und bei Normaldruck über Nacht hydriert. Die Reaktionslösung wurde durch Celite filtiert und der Filterkuchen mit DMF gewaschen. Die vereinigten organischen Phasen wurden am Rotationsverdampfer eingeengt.

### Stufe (c) : 2'-[1-(2,4-Difluorbenzyl)-1H-pyrazolo[3,4-b]pyridin-3-yl]spiro[cyclopropan-1,5'-pyrrolo[2,3-d]pyrimidin]-6'(7'H)-on

Das Zwischenprodukt aus Stufe b) wurde in 20 ml THF aufgenommen, unter einer Argonatmosphäre mit 109 mg (0.974 mmol) Kalium-tert.-butylat versetzt und für 2 h bei RT gerührt. Das Reaktionsgemisch wurde mit Wasser versetzt und mit Essigsäure auf pH = 5 gestellt. Es wurde 10 Min. bei RT gerührt und das Gemisch mit Essigsäureethylester extrahiert. Die vereinigten oranischen Phasen wurden über Natriumsulfat getrocknet und am Rotationsverdampfer eingeengt. Der Rückstand wurde mittels präparativer HPLC gereinigt (Eluent: Acetonitril/Wasser mit 0.1% Ameisensäure, Gradient 50:50 → 70:30). Es wurden 101 mg der Titelverbindung erhalten (25% d. Th.).

LC-MS (Methode 1): Rₜ = 0.93 min; MS (EIpos): m/z = 405 [M+H]⁺.

¹H-NMR (400 MHz, DMSO-d₆): δ [ppm] = 1.62-1.65 (m, 2H), 1.85-1.88 (m, 2H), 5.82 (s, 2H), 7.07 (dt, 1H), 7.29 (dt, 1H), 7.34-7.44 (m, 2H), 8.39 (s, 1H), 8.67 (d, 1H), 8.87 (d, 1H), 11.77 (s br, 1H).

### Beispiel 9

### 3-[1-(2-Fluorbenzyl)-1H-pyrazolo[3,4-b]pyridin-3-yl]-7,7-dimethyl-5,7-dihydro-6H-pyrrolo[2,3-e][1,2,4]triazin-6-on

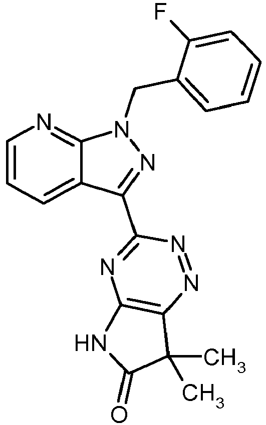

### Stufe (a) : Methyl-2-{3-[1-(2-fluorbenzyl)-1H-pyrazolo[3,4-b]pyridin-3-yl]-5-hydroxy-1,2,4-triazin-6-yl}-2-methylpropanoat

6.00 g (14.562 mmol) der Verbindung aus Beispiel 23A wurden in 70 ml Ethanol gelöst und mit 2.740 g (14.562 mmol) Dimethyl-2,2-dimethyl-3-oxobutandioat (beschrieben in J. Am. Chem. Soc. 124(14), 3680-3691; 2002) versetzt. Das Gemisch wurde über Nacht zum Rückfluß erhitzt. Nach dem Abkühlen wurde der Feststoff abgesaugt, mit wenig Ethanol gewaschen, das Filtrat eingeengt und im Hochvakuum getrocknet.

### Stufe (b): 3-[1-(2-Fluorbenzyl)-1H-pyrazolo[3,4-b]pyridin-3-yl]-7,7-dimethyl-5,7-dihydro-6H pyrrolo[2,3-e][1,2,4]triazin-6-on

Das Zwischenprodukt aus Stufe a) wurde mit 35 ml Phosphorylchlorid versetzt und über Nacht gerührt. Das Reaktionsgemisch wurde in 500 ml Acetonitril gelöst und unter Eiskühlung in 300 ml konzentrierte Ammoniak-Lösung eingerührt. Es wurde 2 h bei RT nachgerührt und das Reaktionsgemisch am Rotationsverdampfer eingeengt. Der Rückstand wurde mit Wasser sowie Essigsäureethylester verrührt und die wässrige Phase mit Essigsäureethylester extrahiert. Die vereinigten organischen Phasen wurden über Natriumsulfat getrocknet und am Rotationsverdampfer eingeengt. Der Rückstand wurde mittels präparativer HPLC gereinigt (Eluent: Methanol/Wasser, Gradient 30:70 → 90:10). Es wurden 701 mg (25% d. Th.) der Titelverbindung erhalten.

LC-MS (Methode 1): Rₜ = 0.96 min; MS (EIpos): m/z = 405 [M+H]⁺.

¹H-NMR (400 MHz, DMSO-d₆): δ [ppm] = 1.45 (s, 6H), 5.89 (s, 2H), 7.15 (dt, 1H), 7.21-7.27 (m, 2H), 7.34-7.40 (m, 1H), 7.48 (dd, 1H), 8.71 (dd, 1H), 8.86 (dd, 1H), 12.16 (s, 1H).

### Beispiel 10

### 2'-[1-(2,3-Difluorbenzyl)-1H-pyrazolo[3,4-b]pyridin-3-yl]spiro[cyclopropan-1,5'-pyrrolo[2,3-d]pyrimidin]-6'(7'H)-on

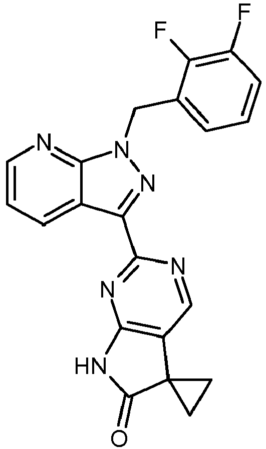

### Stufe (a): Ethyl-1-{4-chlor-2-[1-(2,3-difluorbenzyl)-1H-pyrazolo[3,4-b]pyridin-3-yl]pyrimidin-5-yl}cyclopropancarboxylat:

0.80 g (1.802 mmol) der unter Beispiel 38A erhaltenen Verbindung wurden in 15 ml DMF unter Argon gelöst, mit 360 mg (9.012 mmol) Natriumhydrid (60%ige Suspension in Öl) versetzt und bei RT 15 Min. gerührt. Anschließend wurden 1.015 g (5.407 mmol) 1,2-Dibromethan addiert und 2 h bei RT gerührt. Es wurde Wasser sowie gesättigte wässrige Natriumchlorid-Lösung addiert und das Gemisch drei mal mit Essigsäureethylesster extrahiert. Die vereinigten organischen Phasen wurden über Natriumsulfat getrocknet und am Rotationsverdampfer eingeengt. Es wurden 902 mg (Reinheit 70%, 75% d. Th.) des Zwischenprodukts erhalten.

### Stufe (b): Ethyl-1-{4-amino-2-[1-(2,3-difluorbenzyl)-1H-pyrazolo[3,4-b]pyridin-3-yl]pyrimidin-5-yl}cyclopropancarboxylat:

Das Zwischenprodukt aus Stufe a) wurde in 13 ml DMF gelöst und mit 131 mg (2.020 mmol) Natriumazid versetzt. Das Reaktionsgemisch wurde für 7 h bei 60°C gerührt und nach dem Abkühlen auf Wasser gegeben. Es wurde drei mal mit Essigsäureethylester extrahiert. Die vereinigten organischen Phasen wurden mit gesättigter wässriger Natriumchloridlösung gewaschen, über Natriumsulfat getrocknet, mit 10 ml DMF versetzt und der Essigsäureethylester bei 80 mbar am Rotationsverdampfer entfernt. Die produkthaltige DMF-Lösung wurde mit 20 ml DMF versetzt und mit 250 mg Palladium (10 % auf Kohle) versetzt und bei Normaldruck für 2 h hydriert. Die Reaktionslösung wurde durch Celite filtiert und der Filterkuchen mit DMF gewaschen. Die vereinigten organischen Phasen wurden am Rotationsverdampfer eingeengt.

### Stufe (c) : 2 '-[1-(2,3-Difluorbenzyl)-1H-pyrazolo[3,4-b]pyridin-3-yl]spiro[cyclopropan-1,5'-pyrrolo[2,3-d]pyrimidin]-6'(7'H)-on

Der Rückstand wurde in 25 ml THF aufgenommen, unter einer Argonatmosphäre mit 151 mg (1.346 mmol) Kalium-tert.-butylat versetzt und für 2 h bei RT gerührt. Das Reaktionsgemisch wurde mit Wasser versetzt und mit Essigsäure auf pH = 5 gestellt. Es wurde 10 min bei RT gerührt und das Gemisch mit Essigsäureethylester extrahiert. Die vereinigten oranischen Phasen wurden über Natriumsulfat getrocknet und am Rotationsverdampfer eingeengt. Der Rückstand wurde mittels präparativer HPLC gereinigt (Eluent: Acetonitril/Wasser mit 0.1% Ameisensäure, Gradient 50:50 → 70:30). Es wurden 227 mg der Titelverbindung erhalten (42% d. Th.).

LC-MS (Methode 1): Rₜ = 0.93 min; MS (EIpos): m/z = 405 [M+H]⁺.

¹H-NMR (400 MHz, DMSO-d₆): δ [ppm] = 1.62-1.65 (m, 2H), 1.85-1.88 (m, 2H), 5.90 (s, 2H), 7.06-7.10 (m, 1H), 7.15-7.21 (m, 1H), 7.37-7.46 (m, 2H), 8.39 (s, 1H), 8.68 (dd, 1H), 8.88 (dd, 1H), 11.78 (s br, 1H).

### Beispiel 11

### 2-[1-(2,3-Difluorbenzyl)-1H-pyrazolo[3,4-b]pyridin-3-yl]-5,5-dimethyl-5,7-dihydro-6H-pyrrolo[2,3-d]pyrimidin-6-on

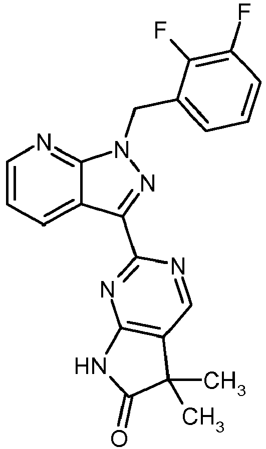

0.86 g (ca. 1.800 mmol) der unter Beispiel 41A erhaltenen Verbindung wurden in Analogie zur Vorschrift unter Beispiel 4 umgesetzt. Es wurden 331 mg der Titelverbindung erhalten (45% d. Th.).

LC-MS (Methode 1): Rₜ = 0.98 min; MS (EIpos): m/z = 407 [M+H]⁺.

¹H-NMR (400 MHz, DMSO-d₆): δ [ppm] = 1.38 (s, 6H), 5.91 (s, 2H), 7.05 (t, 1H), 7.14-7.20 (m, 1H), 7.36-7.41 (m, 1H), 7.44 (dd, 1H), 8.65 (s, 1H), 8.68 (dd, 1H), 8.88 (dd, 1H), 11.59 (s br, 1H).

### Beispiel 12

### 4-Cyclopropyl-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

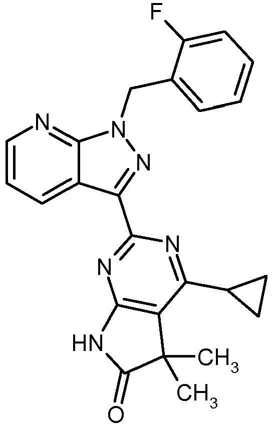

Die unter Beispiel 47A erhaltene Lösung wurde in Analogie zu Beispiel 19A hydriert. Nach Reinigung mittels präparativer HPLC (Eluent: Acetonitril/Wasser mit 0.1% Ameisensäure) wurden 48 mg der Titelverbindung erhalten (7% d. Th.).

LC-MS (Methode 1): Rₜ = 1.14 min; MS (EIpos): m/z = 429 [M+H]⁺.

¹H-NMR (400 MHz, DMSO-d₆): δ [ppm] = 1.14-1.18 (m, 2H), 1.27-1.30 (m, 2H), 1.48 (s, 6H), 2.23-2.27 (m, 1H), 5.84 (s, 2H), 7.12-7.25 (m, 3H), 7.33-7.38 (m, 1H), 7.45 (dd, 1H), 8.39 (s, 1H), 8.66 (dd, 1H), 8.69 (dd, 1H), 11.52 (s br, 1H).

### Beispiel 13

### 2-[1-(2,4-Difluorbenzyl)-1H-pyrazolo[3,4-b]pyridin-3-yl]-5,5-dimethyl-5,7-dihydro-6H-pyrrolo[2,3-d]pyrimidin-6-on

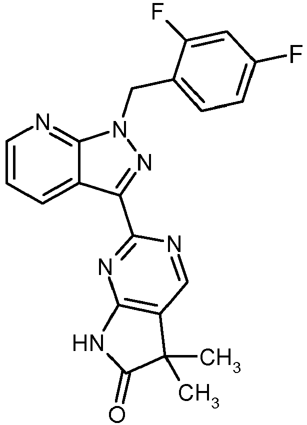

0.75 g (ca. 1.269 mmol) der unter Beispiel 50A erhaltenen Verbindung wurden in Analogie zur Vorschrift unter Beispiel 4 umgesetzt. Es wurden 193 mg der Titelverbindung erhalten (37% d. Th.).

LC-MS (Methode 1): Rₜ = 0.97 min; MS (EIpos): m/z = 407 [M+H]⁺.

¹H-NMR (400 MHz, DMSO-d₆): δ [ppm] = 1.38 (s, 6H), 5.83 (s, 2H), 7.06 (ddd, 1H), 7.26-7.38 (m, 2H), 7.43 (dd, 1H), 8.64 (s, 1H), 8.68 (dd, 1H), 8.87 (dd, 1H), 11.59 (s br, 1H).

### Beispiel 14

### 2-[1-(2-Fluorbenzyl)-1H-pyrazolo[3,4-b]pyridin-3-yl]-5-methyl-5,8-dihydropyrido[2,3-d]pyrimidin-7(6H)-on

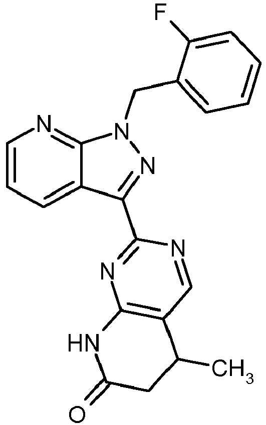

168 mg (0.327 mmol) von Beispiel 58A wurden in Analogie zu Beispiel 1 umgesetzt. Es wurden 71 mg der Titelverbindung erhalten (56 % d. Th.).

LC-MS (Methode 1): Rₜ = 0.95 min; MS (ESIpos): m/z = 389 (M+H)⁺

¹H-NMR (400 MHz, DMSO-d₆): δ [ppm] = 1.27 (d, 3H), 2.43 (dd, 1H), 2.76 (dd, 1H), 3.21-3.27 (m, 1H), 5.85 (s, 2H), 7.12-7.26 (m, 3H), 7.33-7.43(m, 2H), 8.61 (s, 1H), 8.66 (dd, 1H), 9.04 (dd, 1H), 11.20 (s, 1H).

### Beispiel 15

### 2-[1-(2-Fluorbenzyl)-1H-pyrazolo[3,4-b]pyridin-3-yl]-5-(trifluormethyl)-5,8-dihydropyrido[2,3-d]pyrimidin-7(6H)-on

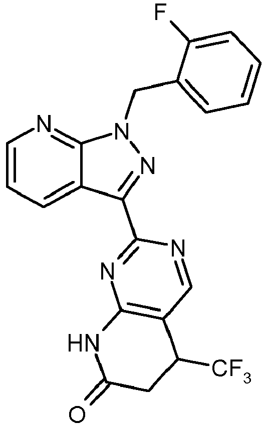

603 mg (1.061 mmol) von Beispiel 59A erhaltenen Verbindung wurden in Analogie zu Beispiel 1 umgesetzt. Es wurden 174 mg der Titelverbindung erhalten (37 % d. Th.).

LC-MS (Methode 1): Rₜ = 1.01 min; MS (ESIpos): m/z = 443 (M+H)⁺

¹H-NMR (400 MHz, DMSO-d₆): δ [ppm] = 2.75 (d, 1H), 3.29 (Signal teilweise unter Wassersignal, 1H), 4.32-4.40 (m, 1H), 5.87 (s, 2H), 7.13-7.26 (m, 3H), 7.34-7.40 (m, 1H), 7.44 (dd, 1H), 8.68 (dd, 1H), 8.77 (s, 1H), 9.06 (dd, 1H), 11.55 (s, 1H).

### Beispiel 16

### 2-[1-(2-Fluorbenzyl)-1H-pyrazolo[3,4-b]pyridin-3-yl]-5H-pyrrolo[2,3-d]pyrimidin-5,6(7H)-dion

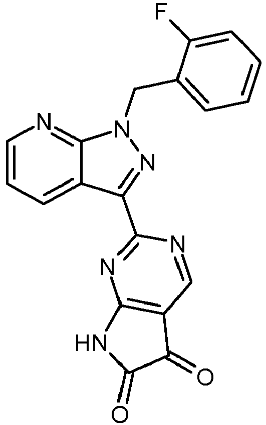

2.00 g (5.550 mmol) Beispiel 4 wurden in Dioxan (200 ml) vorgelegt und mit 3.079 g (27.751 mmol) Selendioxid versetzt und 2 h zum Rückfluss erhitzt. Nach Abkühlen wurde filtriert und das Filtrat wurde eingeengt und mittels Chromatographie an Kieselgel (Laufmittel: Cyclohexan/ Essigsäureethylester 1:1) gereinigt. Es wurden 890 mg der Titelverbindung erhalten (42 % d. Th.).

LC-MS (Methode 1): Rₜ = 0.93 min; MS (ESIpos): m/z = 375 (M+H)⁺

1H-NMR (400 MHz, DMSO-d6): δ [ppm] = 5.91 (s, 2H), 7.17 (ddd, 1H), 7.21-7.26 (m, 1H), 7.27-7.31 (ddd, 1H), 7.35-7.41 (m, 1H), 7.51 (dd, 1H), 8.72 (dd, 1H), 8.87 (s, 1H), 8.89 (dd, 1H), 12.21 (s, 1H).

### Beispiel 17

### 2-[1-(2-Fluorbenzyl)-1H-pyrazolo[3,4-b]pyridin-3-yl]-5-hydroxy-5-methyl-5,7-dihydro-6H-pyrrolo[2,3-d]pyrimidin-6-on

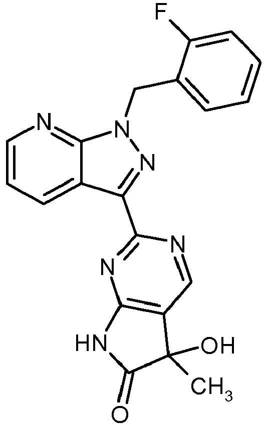

200 mg (0.534 mmol) von Beispiel 16 wurden in THF (10 ml) bei 0°C vorgelegt und mit 0.356 ml (1.069 mmol) Methylmagnesiumbromid (3M-Lösung in Diethylether) versetzt. Nach 15 min bei 0°C wurde für 1 h auf RT erwärmt. Anschliessend wurde der Ansatz auf gesättigte wässrige Ammoniumchlorid-Lösung gegeben und dreimal mit Essigsäureethylester extrahiert. Die organischen Phasen wurden vereinigt, einmal mit Wasser und einmal mit gesättigter wässriger Natriumchlorid-Lösung gewaschen. Danach wurde über Natriumsulfat getrockent, filtriert und eingeengt. Der Rückstand wurde mittels präparativer HPLC gereinigt (Acetonitril:Wasser (+0.05 % Ameisensäure) - Gradient). Es wurden 55 mg der Titelverbindung erhalten (53% d. Th.).

LC-MS (Methode 1): Rₜ = 0.81 min; MS (ESIpos): m/z = 391 (M+H)⁺

1H-NMR (400 MHz, DMSO-d6): δ [ppm] = 1.50 (s, 3H), 5.86 (s, 2H), 6.29 (s, 1H), 7.16 (ddd, 1H), 7.21-7.26 (m, 2H), 7.34-7.39 (m, 1H), 7.44 (dd, 1H), 8.61 (s, 1H), 8.68 (dd, 1H), 8.87 (dd, 1H), 11.53 (s, 1H).

### Beispiel 18

### 2-[1-(2-Fluorbenzyl)-1H-pyrazolo[3,4-b]pyridin-3-yl]-5-hydroxy-5-(trifluormethyl)-5,7-dihydro-6H-pyrrolo[2,3-d]pyrimidin-6-on

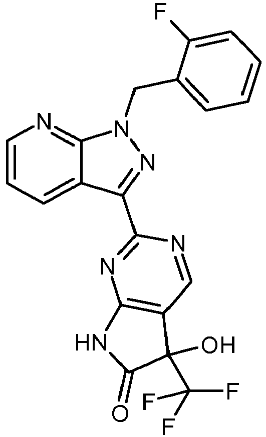

150 mg (0.401 mmol) Beispiel 16 wurden in 1,2-Dimethoxyethan (3 ml) bei RT vorgelegt und mit 6 mg (0.04 mmol) Cäsiumfluorid versetzt. Anschliessend wurden 177 µl (1.202 mmol) (Trifluormethyl)-trimethylsilan zugetropft und der Ansatz über Nacht bei RT gerührt. Danach wurden erneut die folgenden Reagenzien zugegeben: 1,2-Dimethoxyethan (2 ml), Cäsiumfluorid (20 mg) und (Trifluormethyl)-trimethylsilan (177 µl). Nach einer weiteren Nacht wurden erneut Cäsiumfluorid (20 mg) und (Trifluormethyl)-trimethylsilan (2.404 ml, 0.5M in THF) zugegeben. Der Ansatz wurde für weitere 2 Tage gerührt und stand anschliessend für 2 weitere Tage ohne Rühren bei RT. Danach wurde eingeengt und der Rückstand wurde mittels präparativer HPLC gereinigt (Acetonitril:Wasser (+0.05 % Ameisensäure) - Gradient). Es wurden 8 mg der Titelverbindung erhalten (4% d. Th.).

LC-MS (Methode 1): Rₜ = 1.01 min; MS (ESIpos): m/z = 445 (M+H)⁺

1H-NMR (400 MHz, DMSO-d6): δ [ppm] = 5.88 (s, 2H), 7.16 (t, 1H), 7.22-7.29 (m, 2H), 7.35-7.41 (m, 1H), 7.47 (dd, 1H), 8.61 (s, 1H), 8.15 (s, 1H), 8.71 (dd, 1H), 8.76 (s, 1H), 8.87 (dd, 1H), 12.28 (s, 1H).

### Beispiel 19

### 3-[1-(2-Fluorbenzyl)-1H-pyrazolo[3,4-b]pyridin-3-yl]-7,7-dimethyl-5,7-dihydro-6H-pyrrolo[2,3-e][1,2,4]triazin-6-on

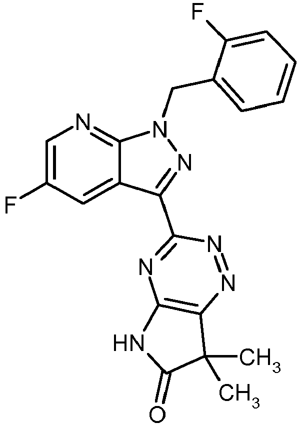

9.700 g (22.025 mmol) der Verbindung aus Beispiel 61A wurde mit 50 ml Phosphorylchlorid versetzt und über Nacht bei RT gerührt. Das Reaktionsgemisch wurde in 150 ml Acetonitril gelöst und unter Eiskühlung in eine Mischung aus 1337 ml konzentrierter wässriger Ammoniak-Lösung (35%-ig) und 1300 ml Acetonitril eingerührt. Es wurde 2 Tage bei Raumtemperatur gerührt. Anschliessend wurden 300g Natriumchlorid zugegeben. Es bildeten sich 2 Phasen. Die organische Phase wurde abgetrennt und bis zur Trockene eingeengt. Der Rückstand wurde in Essigsäureethylester (150 ml) aufgenommen. Es bildete sich ein Niederschlag, welcher über eine Fritte abgesaugt wurde. Es wurde dreimal mit Wasser und anschliessend dreimal mit Essigsäureethylester (5 ml) nachgewaschen. Der Rückstand wurde im Hochvakuum getrocknet. Es wurden 4.58 g (51% d. Th.) der Titelverbindung erhalten.

LC-MS (Methode 1): Rₜ = 0.99 min; MS (EIpos): m/z = 408 [M+H]⁺.

¹H-NMR (400 MHz, DMSO-d₆): δ [ppm] = 1.45 (s, 6H), 5.88 (s, 2H), 7.15-7.30 (m, 3H), 7.35-7.41 (m, 1H), 8.56 (dd, 1H), 8.79 (dd, 1H), 12.18 (s br, 1H).

### Beispiel 20

### 3-[1-(2,3-Difluorbenzyl)-5-fluor-1H-pyrazolo[3,4-b]pyridin-3-yl]-7,7-dimethyl-5,7-dihydro-6H-pyrrolo[2,3-e][1,2,4]triazin-6-on

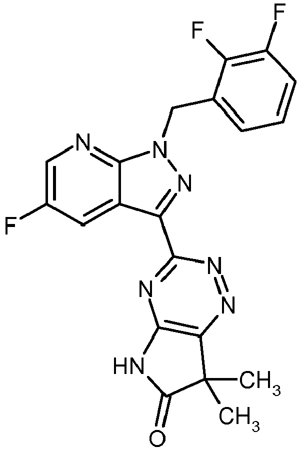

2.060 g (4.49 mmol) der Verbindung aus Beispiel 66A wurde mit 22 ml Phosphorylchlorid versetzt und über Nacht bei RT gerührt. Das Reaktionsgemisch wurde in 100 ml Acetonitril gelöst und unter Eiskühlung in 290 ml konzentrierte wässrige Ammoniak-Lösung (35%-ig) eingerührt. Es wurde 2 h bei Raumtemperatur, dann 7 h bei 50°C gerührt und das Reaktionsgemisch anschließend am Rotationsverdampfer eingeengt. Der Rückstand wurde mit Wasser sowie Essigsäureethylester verrührt und die wässrige Phase mit Essigsäureethylester extrahiert. Die vereinigten organischen Phasen wurden über Natriumsulfat getrocknet und am Rotationsverdampfer eingeengt. Der Rückstand wurde mit Diethylether verrührt, abgesaugt, mit wenig Diethylether gewaschen und im Hochvakuum getrocknet. Es wurden 748 mg (37% d. Th.) der Titelverbindung erhalten.

LC-MS (Methode 3): Rₜ = 1.23 min; MS (EIpos): m/z = 426 [M+H]⁺.

¹H-NMR (400 MHz, DMSO-d₆): δ [ppm] = 1.45 (s, 6H), 5.94 (s, 2H), 7.09-7.21 (m, 2H), 7.41 (q, 1H), 8.57 (dd, 1H), 8.80 (s, 1H), 12.18 (s br, 1H).

### Beispiel 21

### 3-[1-(2,4-Difluorbenzyl)-5-fluor-1H-pyrazolo[3,4-b]pyridin-3-yl]-7,7-dimethyl-5,7-dihydro-6H-pyrrolo[2,3-e][1,2,4]triazin-6-on

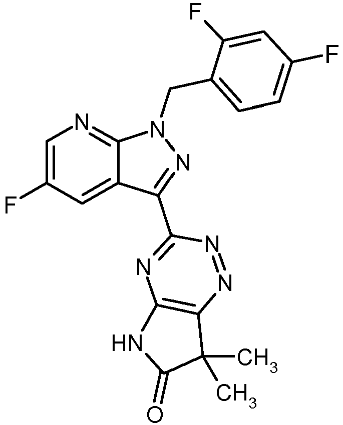

2.290 g (5.000 mmol) der Verbindung aus Beispiel 71A wurde mit 25 ml Phosphorylchlorid versetzt und über Nacht bei RT gerührt. Das Reaktionsgemisch wurde in 120 ml Acetonitril gelöst und unter Eiskühlung in 350 ml konzentrierte wässrige Ammoniak-Lösung (35%-ig) eingerührt. Es wurde 2 h bei Raumtemperatur, dann 6 h bei 50°C gerührt und das Reaktionsgemisch anschließend am Rotationsverdampfer eingeengt. Der Rückstand wurde mit Wasser sowie Essigsäureethylester verrührt und die wässrige Phase mit Essigsäureethylester extrahiert. Die vereinigten organischen Phasen wurden über Natriumsulfat getrocknet und am Rotationsverdampfer eingeengt. Der Rückstand wurde mit Diethylether verrührt, abgesaugt, mit wenig Diethylether gewaschen und im Hochvakuum getrocknet. Es wurden 1.020 g (47% d. Th) der Titelverbindung erhalten.

LC-MS (Methode 4): Rₜ = 1.06 min; MS (EIpos): m/z = 426 [M+H]⁺.

¹H-NMR (400 MHz, DMSO-d₆): δ [ppm] = 1.45 (s, 6H), 5.86 (s, 2H), 7.09 (dt, 1H), 7.29 (dt, 1H), 7.41 (q, 1H), 8.55 (dd, 1H), 8.79 (s, 1H), 12.19 (s br, 1H).

### Beispiel 22

### 2-[1-(2-Fluorbenzyl)-1H-pyrazolo[3,4-b]pyridin-3-yl]-5-hydroxy-5-phenyl-5,7-dihydro-6H-pyrrolo[2,3-d]pyrimidin-6-on

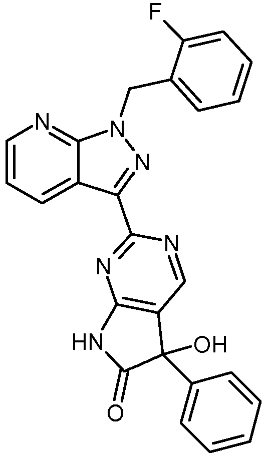

75 mg (0.200 mmol) Beispiel 16 wurden in THF (5 ml) bei 0°C vorgelegt und mit 0.134 ml einer 3M Lösung von Phenylmagnesiumbromid in Diethylether (0.401 mmol) versetzt. Nach 15 min bei 0°C wurde für 1 h auf RT erwärmt. Anschliessend wurde der Ansatz auf gesättigte wässrige Ammoniumchlorid-Lösung gegeben und dreimal mit Essigsäureethylester extrahiert. Die organischen Phasen wurden vereinigt, einmal mit Wasser und einmal mit gesättigter wässriger Natriumchlorid-Lösung gewaschen. Danach wurde über Natriumsulfat getrocknet, filtriert und eingeengt. Der Rückstand wurde mittels präparativer HPLC gereinigt (Acetonitril:Wasser (+0.05 % Ameisensäure) - Gradient). Es wurden 38 mg der Titelverbindung erhalten (42% d. Th.).

LC-MS (Methode 1): Rₜ = 1.03 min; MS (ESIpos): m/z = 453 (M+H)⁺

¹H-NMR (400 MHz, DMSO-d₆): δ [ppm] = 5.87 (d, 2H), 7.08 (s, 1H), 7.17 (dd, 1H), 7.22-7.29 (m, 2H), 7.33-7.47 (m, 7H), 8.48 (s, 1H), 8.69 (dd, 1H), 8.88 (dd, 1H), 11.76 (br s, 1H).

### Beispiel 23

### 5-Fluor-2-[1-(2-fluorbenzyl)-1H-pyrazolo[3,4-b]pyridin-3-yl]-5-methyl-5,7-dihydro-6H-pyrrolo[2,3-d]pyrimidin-6-on

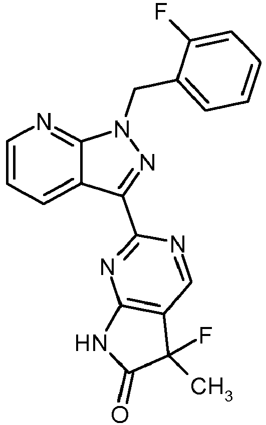

100 mg (0.256 mmol) Beispiel 17 wurden in Dichlormethan (4.795 ml) bei -78°C vorgelegt und mit 40.61 µl (0.307 mmol) Diethylaminoschwefeltrifluorid versetzt. Anschliessend wurde über Nacht langsam auf RT erwärmt. Danach wurde abermals 40.61 µl (0.307 mmol) Diethylaminoschwefeltrifluorid zugegeben und eine weitere Nacht bei RT gerührt. Der Ansatz wurde danach mit Dichlormethan verdünnt und mit gesättigter wässriger Natriumhydrogencarbonatlösung extrahiert. Die organische Phase wurde über Natriumsulfat getrockent, filtriert und eingeengt. Der Rückstand wurde mittels präparativer HPLC gereinigt (Acetonitril:Wasser (+0.05 % Ameisensäure) - Gradient). Es wurden 42 mg der Titelverbindung erhalten (42% d. Th.).

LC-MS (Methode 1): Rₜ = 0.96 min; MS (ESIpos): m/z = 393 (M+H)⁺

¹H-NMR (400 MHz, DMSO-d₆): δ [ppm] = 1.82 (s, 3H), 5.88 (s, 2H), 7.16 (ddd, 1H), 7.21-7.28 (m, 2H), 7.34-7.40 (m, 1H), 7.46 (dd, 1H), 8.70 (dd, 1H), 8.86-8.89 (m, 2H), 12.01 (s, 1H).

### Beispiel 24

### tert.-Butyl- {2-[1-(2-fluorbenzyl)-1H-pyrazolo[3,4-b]pyridin-3-yl]-5-hydroxy-6-oxo-6,7-dihydro-5H-pyrrolo[2,3-d]pyrimidin-5-yl} acetat

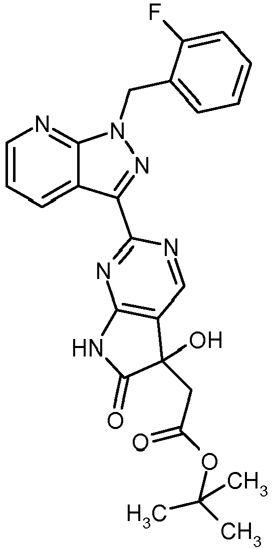

100 mg (0.267 mmol) Beispiel 16 wurden in THF (5 ml) bei -45°C vorgelegt und mit 1.087 ml einer 1M Lösung von Bis(trimethylsilyl)lithiumamid in THF versetzt und für 30min bei -45°C gerührt. Anschliessend wurde 126 mg (1.087 mmol) Essigsäure-tert.-butylester (in 5 ml THF gelöst) zugetropft. Es wurde auf RT erwärmt und über Nacht bei dieser Temperatur gerührt. Der Ansatz wurde mit Wasser versetzt und mit Essigsäure auf pH=4 eingestellt. Danach wurde dreimal mit Essigsäureethylester extrahiert. Die vereinigten organischen Phasen wurden mit Wasser und gesättigter wässriger Natriumchloridlösung gewaschen, über Natriumsulfat getrocknet, filtriert und eingeengt. Der Rückstand wurde mittels präparativer HPLC gereinigt (Acetonitril:Wasser (+0.05 % Ameisensäure) - Gradient). Es wurden 73 mg der Titelverbindung erhalten (55% d. Th.).

LC-MS (Methode 1): Rₜ = 0.96 min; MS (ESIpos): m/z = 491 (M+H)⁺

¹H-NMR (400 MHz, DMSO-d₆): δ [ppm] = 1.17 (s, 9H), 3.05 (q, 2H), 5.87 (s, 2H), 6.59 (s, 1H), 7.16 (t, 1H), 7.22-7.28 (m, 2H), 7.35-7.40 (m, 1H), 7.45 (dd, 1H), 8.66 (s, 1H), 8.68 (dd, 1H), 8.87 (dd, 1H), 11.64 (s, 1H).

### Beispiel 25

### {2-[1-(2-Fluorbenzyl)-1H-pyrazolo[3,4-b]pyridin-3-yl]-5-hydroxy-6-oxo-6,7-dihydro-5H-pyrrolo[2,3-d]pyrimidin-5-yl} essigsäure

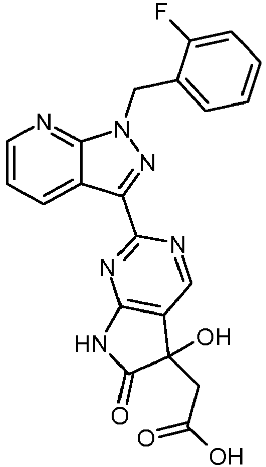

60 mg (0.122 mmol) von Beispiel 24 wurden in Dichlormethan (1 ml) und Trifluoressigsäure (1 ml) für 30 min bei RT gerührt. Danach wurde eingeengt und der Rückstand in Acetonitril aufgenommen und mit Wasser versetzt. Es wurde ein Niederschlag abfiltriert, der mit Acetonitril gewaschen wurde und am Hochvakuum getrocknet wurde. Es wurden so 42 mg der Titelverbindung erhalten (80% d. Th.).

LC-MS (Methode 1): Rₜ = 0.80 min; MS (ESIpos): m/z = 435 (M+H)⁺

¹H-NMR (400 MHz, DMSO-d₆): δ [ppm] = 3.02 (d, 1H), 3.17 (d, 1H), 5.87 (s, 2H), 6.53 (s, 1H), 7.16 (t, 1H), 7.21-7.26 (m, 2H), 7.34-7.39 (m, 1H), 7.44 (dd, 1H), 8.66 (s, 1H), 8.68 (dd, 1H), 8.88 (dd, 1H), 11.56 (s, 1H).

### Beispiel 26

### Methyl- {2-[1-(2-fluorbenzyl)-1H-pyrazolo[3,4-b]pyridin-3-yl]-6-oxo-6,7-dihydro-5H-pyrrolo[2,3-d]pyrimidin-5-yl} carbamat

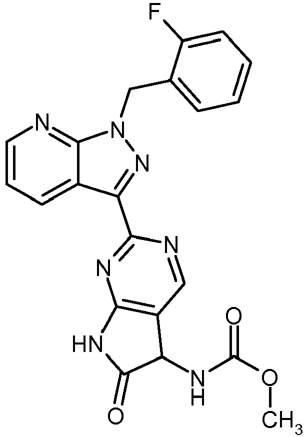

150 mg (ca. 0.319 mmol) Beispiel 73A wurden in Pyridin (2 ml) vorgelegt und anschliessend mit Chlorameisensäuremethylester (24 µl in 1 ml Dichlormethan) versetzt bis ein vollständiger Umsatz erreicht wurde. Danach wurde eingeengt und der Rückstand wurde mittels präparativer HPLC gereinigt (Acetonitril:Wasser (+0.05 % Ameisensäure) - Gradient). Es wurden 11 mg der Titelverbindung in 90%iger Reinheit erhalten (7% d. Th.).

LC-MS (Methode 1): Rₜ = 0.87 min; MS (ESIpos): m/z = 434 (M+H)⁺

¹H-NMR (400 MHz, DMSO-d₆): δ [ppm] = 3.55 (s, 3H), 5.10 (d, 1H), 5.86 (s, 2H), 7.16 (t, 1H), 7.21-7.26 (m, 2H), 7.35-7.38 (m, 1H), 7.44 (dd, 1H), 8.21 (d, 1H), 8.46 (s, 1H), 8.68 (dd, 1H), 8.87 (dd, 1H), 11.66 (s, 1H).

### Beispiel 27

### 3-[1-(2,3-Difluorbenzyl)-1H-pyrazolo[3,4-b]pyridin-3-yl]-7,7-dimethyl-5,7-dihydro-6H-pyrrolo[2,3-e][1,2,4]triazin-6-on

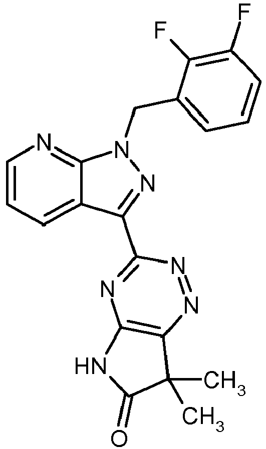

0.75 g (1.703 mmol) der Verbindung aus Beispiel 75A wurde mit 8 ml Phosphorylchlorid versetzt und über Nacht bei RT gerührt. Das Reaktionsgemisch wurde dann in 120 ml Acetonitril aufgenommen und unter Eiskühlung in 72 ml konzentrierte Ammoniak-Lösung (33 % in Wasser) eingerührt. Es wurde 3 Tage bei Raumtemperatur gerührt und das Reaktionsgemisch anschließend am Rotationsverdampfer eingeengt. Der Rückstand wurde mit Wasser sowie Essigsäureethylester verrührt und die wässrige Phase zweimal mit Essigsäureethylester extrahiert. Die vereinigten organischen Phasen wurden mit gesättigter wässriger Natriumchloridlösung gewaschen und danach über Natriumsulfat getrocknet, filtriert und am Rotationsverdampfer eingeengt. Der Rückstand wurde mittels präparativer HPLC gereinigt (Acetonitril/Wasser/Wasser + 1% TFA - 40:55:5)Es wurden 134 mg der Titelverbindung in erhalten (19% d. Th.).

LC-MS (Methode 4): Rₜ = 0.98 min; MS (EIpos): m/z = 408 [M+H]⁺.

¹H-NMR (400 MHz, DMSO-d₆): δ [ppm] = 1.46 (s, 6H), 5.94 (s, 2H), 7.07 (t, 1H), 7.15-7.19 (m, 1H), 7.37-7.42 (m, 1H), 7.49 (dd, 1H), 8.72 (dd, 1H), 8.86 (dd, 1H), 12.19 (s br, 1H).

### Beispiel 28

### 3-[1-(2,4-Difluorbenzyl)-1H-pyrazolo[3,4-b]pyridin-3-yl]-7,7-dimethyl-5,7-dihydro-6H-pyrrolo[2,3-e] [1,2,4]triazin-6-on

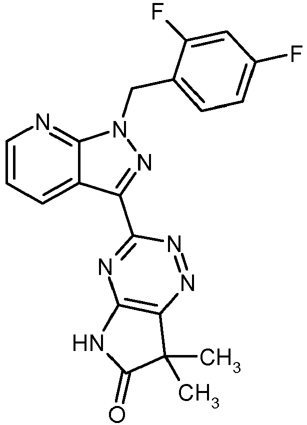

2.74 g (6.221 mmol) der Verbindung aus Beispiel 77A wurde mit 29 ml Phosphorylchlorid versetzt und über Nacht bei RT gerührt. Das Reaktionsgemisch wurde dann in 430 ml Acetonitril aufgenommen und unter Eiskühlung in 260 ml konzentrierte Ammoniak-Lösung (33 % in Wasser) eingerührt. Es wurde über Nacht bei Raumtemperatur gerührt und das Reaktionsgemisch anschließend am Rotationsverdampfer eingeengt. Der Rückstand wurde mit Wasser sowie Essigsäureethylester verrührt und die wässrige Phase zweimal mit Essigsäureethylester extrahiert. Die vereinigten organischen Phasen wurden mit gesättigter wässriger Natriumchloridlösung gewaschen und danach über Natriumsulfat getrocknet, filtriert und am Rotationsverdampfer eingeengt. Der Rückstand wurde mit Essigsäureethylester und Diethylether verrührt. Es wurde ein Feststoff abgesaugt, der anschliessend mit Diethylether und Essigsäureethylester gewaschen wurde. Nach Trocknung am Hochvakuum wurden 2.13 g der Titelverbindung in 94%iger Reinheit erhalten (79% d. Th.).

LC-MS (Methode 1): Rₜ = 0.93 min; MS (EIpos): m/z = 408 [M+H]⁺.

¹H-NMR (400 MHz, DMSO-d₆): δ [ppm] = 1.45 (s, 6H), 5.86 (s, 2H), 7.05-7.10 (m, 1H), 7.26-7.32 (ddd, 1H), 7.34-7.40 (m, 1H), 7.48 (dd, 1H), 8.71 (dd, 1H), 8.85 (dd, 1H), 12.19 (s br, 1H).

### Beispiel 29

### 3-[5-Fluor-1-(2,3,6-trifluorbenzyl)-1H-pyrazolo[3,4-b]pyridin-3-yl]-7,7-dimethyl-5,7-dihydro-6H-pyrrolo[2,3-e][1,2,4]triazin-6-on

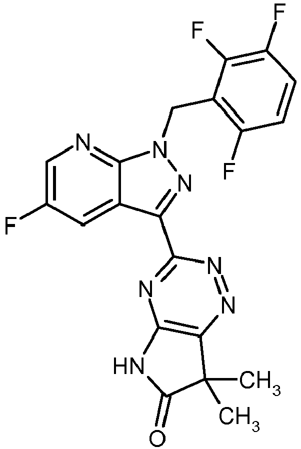

1.50 g (3.149 mmol) der Verbindung aus Beispiel 82A wurde mit 15 ml Phosphorylchlorid versetzt und über Nacht gerührt. Das Reaktionsgemisch wurde dann in 222 ml Acetonitril aufgenommen und unter Eiskühlung in 133 ml konzentrierte Ammoniak-Lösung (33 % in Wasser) eingerührt. Es wurde über Nacht bei Raumtemperatur gerührt und das Reaktionsgemisch anschließend am Rotationsverdampfer eingeengt. Der Rückstand wurde mit Ethanol und Wasser versetzt. Es bildete sich ein Niederschlag, welcher abfiltriert wurde und anschliessend mit Diethylether gewaschen wurde. Nach Trocknung am Hochvakuum wurden 982 mg der Titelverbindung erhalten (70% d. Th.).

LC-MS (Methode 1): Rₜ = 1.01 min; MS (EIpos): m/z = 444 [M+H]⁺.

¹H-NMR (400 MHz, DMSO-d₆): δ [ppm] = 1.44 (s, 6H), 5.92 (s, 2H), 7.21 (m, 1H), 7.57 (m, 1H), 8.54 (dd, 1H), 8.81 (dd, 1H), 12.19 (s br, 1H).

### Beispiel 30

### 2'-[5-Fluor-1-(2-fluorbenzyl)-1H-pyrazolo[3,4-b]pyridin-3-yl]spiro[cyclopropan-1,5'-pyrrolo[2,3-d]pyrimidin]-6'(7'H)-on

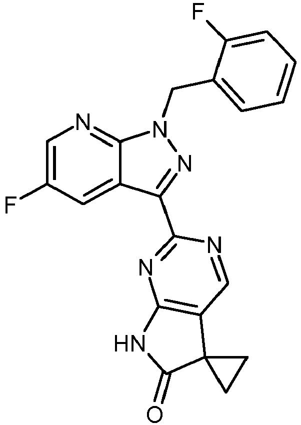

1.28 g (2.84 mmol) von Beispiel 87A wurden in Analogie zur Vorschrift unter Beispiel 4 mit 478 mg (4.26 mmol) Kalium-tert.-butylat in 60 ml THF umgesetzt. Es wurden 426 mg der Titelverbindung erhalten (36 % d. Th.).

LC-MS (Methode 4): Rₜ = 1.04 min; MS (ESIpos): m/z = 405 (M+H)⁺

¹H-NMR (400 MHz, DMSO-d₆): δ [ppm] = 1.63 (dd, 2H), 1.87 (dd, 2H), 5.85 (s, 2H), 7.17 (dt, 1H), 7.20-7.31 (m, 2H), 7.38 (m, 1H), 8.38 (s, 1H), 8.59 (dd, 1H), 8.75 (dd, 1H), 11.77 (s br, 1H).

### Beispiel 31

### 2-[1-(2-Fluorbenzyl)-1H-pyrazolo[3,4-b]pyridin-3-yl]-5-(propan-2-yliden)-5,7-dihydro-6H-pyrrolo[2,3-d]pyrimidin-6-on

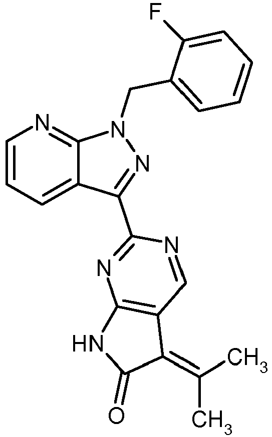

500 mg (1.388 mmol) Beispiel 4 wurden in Aceton (50 ml) vorgelegt, mit 0.274 ml (2.775 mmol) Piperidin versetzt und 1h zum Rückfluss erhitzt. Nach Abkühlen wurde von einem Niederschlag filtriert, dieser mit Aceton nachgewaschen und das Filtrat im Vakuum eingeengt. Dieser Rückstand wurde anschliessend mittels Chromatographie an Kieselgel (Laufinittel: Cyclohexan/Essigsäure-ethylester - Gradient) gereinigt. Das so erhaltene Material wurde abermals in Essigsäureethylester aufgeschlämmt, abfiltriert, mit Essigsäureethylester gewaschen und im Hochvakuum getrocknet. Man erhielt 101 mg der Titelverbindung als Feststoff (18 % d. Th.).

LC-MS (Methode 4): Rₜ = 1.06 min; MS (ESIpos): m/z = 401 (M+H)⁺

¹H-NMR (400 MHz, DMSO-d₆): δ [ppm] = 2.41 (s, 3H), 5.86 (s, 2H), 7.14-7.18 (m, 1H), 7.22-7.28 (m, 2H), 7.33-7.41 (m, 1H), 7.44 (dd, 1H), 8.67 (dd, 1H), 8.83 (s, 1H), 8.90 (dd, 1H), 11.67 (s, 1H).

### Beispiel 32

### 2'-[1-(2-Fluorbenzyl)-1H-pyrazolo[3,4-b]pyridin-3-yl]-2,2-dimethylspiro[cyclopropan-1,5'-pyrrolo[2,3-d]pyrimidin]-6'(7'H)-on

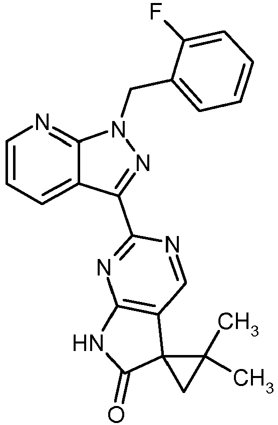

19.97 mg (0.499 mmol) Natriumhydrid (60 % in Mineralöl) und 164 mg (0.749 mmol) Trimethylsulfoxoniumiodid wurden unter Argon vorgelegt und mit 1.9 ml DMSO versetzt. Es wurde 1h bei RT gerührt und danach wurden 100 mg (0.250 mmol) Beispiel 31 in DMSO (4.6 ml) gelöst zugetropft. Nach 1h bei RT wurde das Gemisch für 6h auf 50°C erhitzt. Das Rohgemisch wurde mittels präparativer HPLC gereinigt (Acetonitril:Wasser (+ 0.05 % Ameisensäure) - Gradient). Es wurden 45 mg der Titelverbindung in erhalten (42% d. Th.).

LC-MS (Methode 1): Rₜ = 1.07 min; MS (ESIpos): m/z = 415 (M+H)⁺

1H-NMR (400 MHz, DMSO-d₆): δ [ppm] = 1.41 (s, 3H), 1.44 (s, 3H), 1.73 (d, 1H), 1.92 (d, 1H), 5.85 (s, 2H), 7.14-7.18 (m, 1H), 7.22-7.26 (m, 2H), 7.34-7.38 (m, 1H), 7.43 (dd, 1H), 8.47 (s, 1H), 8.67 (dd, 1H), 8.88 (dd, 1H), 11.69 (s, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontroll-zyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 1 | 12 |
| 2 | 12 |
| 7 | 45 |
| 8 | 13 |
| 10 | 6 |
| 11 | 4 |
| 14 | 65 |
| 17 | 13 |
| 18 | 93 |
| 19 | 157 |
| 20 | 45 |
| 23 | 15 |
| 24 | 269 |
| 26 | 840 |
| 27 | 54 |
| 28 | 103 |
| 30 | 97 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 1 | 0.01 |
| 2 | 0.01 |
| 3 | 0.03 |
| 4 | 0.03 |
| 5 | 0.03 |
| 6 | 0.03 |
| 7 | 0.03 |
| 8 | 0.03 |
| 9 | 0.03 |
| 10 | 0.03 |
| 11 | 0.03 |
| 12 | 0.1 |
| 13 | 0.1 |
| 14 | 0.1 |
| 15 | 0.3 |
| 16 | 1.0 |
| 17 | 0.1 |
| 18 | 0.03 |
| 19 | 0.03 |
| 20 | 0.03 |
| 21 | 0.1 |
| 22 | 0.03 |
| 23 | 0.03 |
| 24 | 0.3 |
| 25 | 1.0 |
| 26 | 0.3 |
| 27 | 0.03 |
| 28 | 0.3 |
| 29 | 0.1 |
| 30 | 0.03 |
| 77A | 1.0 |
| 82A | 0.3 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen der Formel (I) werden in männlichen CD-1-Mäusen, männlichen Wister-Ratte und/oder weiblichen Beagle-Hunden bestimmt. Das Applikationsvolumen beträgt bei Mäusen 5 mL/kg, bei Ratten 5 mL/kg und bei Hunden 0.5 mL/kg. Die intravenöse Gabe erfolgt bei Mäusen und Ratten mittels einer speziesspezifischen Plasma/DMSO-Formulierung (99/1) und bei Hunden mittels Wasser/PEG400/Ethanol (50/40/10 bzw. 30/60/10). Den Ratten wird zur vereinfachten Blutabnahme vor der Substanzgabe ein Silikonkatheter in die rechte *Vena jugularis externa* gelegt. Die Operation erfolgt einen Tag vor dem Versuch unter Isofluran-Narkose und unter Gabe eines Analgetikums (Atropin/Rimadyl (3/1) 0.1 mL s.c.). Die Substanzgabe wird bei Mäusen und Ratten i.v. bolus und bei Hunden mittels 15-minütiger Infusion durchgeführt. Die Blutabnahme erfolgt bei Ratten nach 0.033, 0.083, 0.17, 0.5, 1, 2, 3, 4, 6, 7 und 24 Stunden, bei Mäusen nach 0.033, 0.083, 0.17, 0.5, 1, 2, 3, 4, 6, 7, 24, 48 und 72 Stunden sowie bei Hunden nach 0.083, 0.25, 0.28 0.33, 0.42, 0.75, 1, 2, 3, 4, 6, 7 und 24 Stunden. Die orale Gabe der gelösten Substanz mittels Schlundsonde wird in allen Spezies basierend auf einer Wasser/PEG400/Ethanol-Formulierung (50/40/10) durchgeführt. Die Blutabnahme in Ratten erfolgt hier nach 0.083, 0.17, 0.5, 0.75, 1, 2, 3, 4, 6, 7 und 24 Stunden und in Hunden nach 0.083, 0.17, 0.5, 0.75, 1, 2, 3, 4, 6, 7, 24, 30 und 48 Stunden. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen der Formel (I), Kalibrierproben und QC's wird ein interner Standard zugesetzt (ZK 228859) und es folgt eine Proteinfällung mittels Acetonitril im Überschuss. Nach Zugabe eines Ammoniumacetat-Puffers (0.01 M, pH 6.8) und folgendem Vortexen wird bei 1000 g zentrifugiert und der Überstand mittels LC-MS/MS (API 4000, AB Sciex) vermessen. Die chromatographische Trennung wird auf einer 1100-HPLC von Agilent durchgeführt. Das Injektionsvolumen beträgt 10 µL. Als Trennsäule wird eine auf 40°C temperierte Luna 5µ C8(2) 100A 50x2mm der Fa. Phenomenex verwendet. Es wird ein binärer Laufmittelgradient bei 500 µL/min gefahren (A: 0.01M Ammoniumacetat-Puffer pH 6.8, B: 0.1 % Ameisensäure in Acetonitril): 0 Min. (90 % A), 1 Min. (90 % A), 3 Min. (15 % A), 4 Min. (15 % A), 4.50 Min. (90 % A), 6 Min. (90 % A). Die Temperatur der Turbo V Ionenquelle beträgt 500°C. Folgende MS-Geräteparameter werden benutzt: Curtain Gas 15 units, Ionsprayvoltage 4.8 kV, Gas 1 45 units, Gas 235 units, CAD Gas 40 units. Die Quantifizierung der Substanzen erfolgt anhand der Peakhöhen oder -flächen aus extrahierten Ionenchromatogrammen spezifischer MRM-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels des validierten pharmakokinetischen Rechenprogramms KinEx (Vers. 2.5 und 3) berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (s.o.) und durch Quotientenbildung der C_{Blut}/C_{Plasma}-Wert ermittelt.

Nach intravenöser Gabe von 0.3 mg/kg von Beispiel 1, 18, 19 und 27 in Ratten wurden folgende Werte erhoben:

| Beispiel | 1 | 18 | 19 | 27 |
|---|---|---|---|---|
| CL _{Blut} [L/h/kg] | 1.1 | 0.46 | 0.32 | 0.35 |
| Terminale Halbwertszeit [h] | 0.9 | 1.4 | 3.0 | 5.6 |
| Mean Residence Time [h] | 1.2 | 1.9 | 4.0 | 7.3 |

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 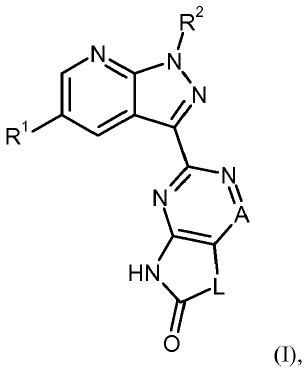 in welcher
A für CR³ steht,
wobei
R³ für Wasserstoff, Deuterium, Fluor, Difluormethyl, Trifluormethyl, (C₁-C₄)-Alkyl, Cyclopropyl oder Cyclobutyl steht,
L für eine Gruppe *-CR^{4A}R^{4B}-(CR^{5A}R5^{B})ₚ-# steht,
wobei
* für die Anknüpfstelle an die Carbonylgruppe steht,
# für die Anknüpfstelle an den Pyrimidin- bzw. Triazinring steht,
p für eine Zahl 0, 1 oder 2 steht,
R^{4A} für Wasserstoff, Fluor, (C₁-C₄)-Alkyl oder Hydroxy steht,
R^{4B} für Wasserstoff, Fluor, (C₁-C₄)-Alkyl, Trifluormethyl, (C₁-C₄)-Alkoxy-carbonylamino oder Phenyl steht,
worin (C₁-C₄)-Alkyl mit 1 oder 2 Substituenten unabhängig voneinander ausgewählt aus der Gruppe Fluor, Trifluormethyl, Hydroxy, Hydroxycarbonyl und (C₁-C₄)-Alkoxycarbonyl substituiert sein kann,
oder
R^{4A} und R^{4B} zusammen mit dem Kohlenstoffatom, an das sie gebunden sind, eine Oxo-Gruppe, einen 3- bis 6-gliedrigen Carbocyclus oder einen 4-bis 6-gliedrigen Heterocyclus bilden,
worin der 3- bis 6-gliedrigen Carbocyclus und der 4- bis 6-gliedrigen Heterocyclus mit 1 oder 2 Substituenten unabhängig voneinander ausgewählt aus der Gruppe Fluor und (C₁-C₄)-Alkyl substituiert sein können,
oder
R^{4A} und R^{4B} zusammen mit dem Kohlenstoffatom, an das sie gebunden sind, eine (C₂-C₄)-Alkenyl-Gruppe bilden,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
R¹ für Wasserstoff oder Fluor steht,
R² für Benzyl steht,
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

2. Verbindung der Formel (I) nach Anspruch 1, in welcher
A für CR³ steht,
wobei
R³ für Wasserstoff, Fluor, Difluormethyl, Trifluormethyl, Methyl, Ethyl oder Cyclopropyl steht,
L für eine Gruppe *-CR^{4A}R^{4B}-(CR^{5A}R5^{B})ₚ-# steht,
wobei
* für die Anknüpfstelle an die Carbonylgruppe steht,
# für die Anknüpfstelle an den Pyrimidin- bzw. Triazinring steht,
p für eine Zahl 0 oder 1 steht,
R^{4A} für Wasserstoff, Fluor, Methyl, Ethyl oder Hydroxy steht,
R^{4B} für Wasserstoff, Fluor, Methyl, Ethyl, Trifluormethyl, Methoxycarbonyl-amino oder Phenyl steht,
worin Methyl und Ethyl mit 1 oder 2 Substituenten unabhängig voneinander ausgewählt aus der Gruppe Fluor, Trifluormethyl und Hydroxy substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
worin der Cyclopropyl-, Cyclobutyl-, Cyclopentyl-, Azetidinyl-, Pyrrolidinyl-, Tetrahydrofuranyl-, Piperidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
R¹ für Wasserstoff oder Fluor steht,
R² für Benzyl steht,
wobei Benzyl mit 1 bis 3 Substituenten Fluor substituiert ist,
sowie ihre Salze, Solvate und Solvate der Salze.

3. Verbindung der Formel (I) nach Anspruch 1 oder 2, in welcher
A für CR³ steht,
wobei
R³ für Wasserstoff steht,
L für eine Gruppe *-CR^{4A}R^{4B}-(CR^{5A}R5^{B})ₚ-# steht,
wobei
* für die Anknüpfstelle an die Carbonylgruppe steht,
# für die Anknüpfstelle an den Pyrimidinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
R¹ für Wasserstoff oder Fluor steht,
R² für Benzyl steht,
wobei Benzyl mit 1 oder 2 Substituenten Fluor substituiert ist,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man eine Verbindung der Formel (II) 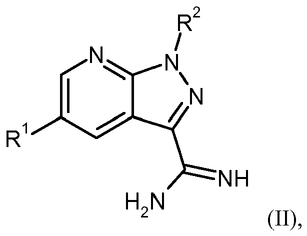 in welcher R¹ und R² jeweils die in den Ansprüchen 1 bis 3 angegebenen Bedeutungen haben,
[A] in einem inerten Lösungsmittel in Gegenwart einer geeigneten Base mit einer Verbindung der Formel (III) 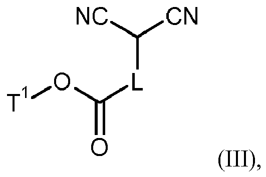 in welcher L die in den Ansprüchen 1 bis 3 angegebene Bedeutung hat und
T¹ für (C₁-C₄)-Alkyl steht,
zu einer Verbindung der Formel (IV) 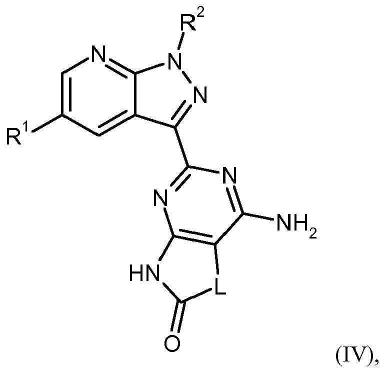 in welcher L, R¹ und R² jeweils die in den Ansprüchen 1 bis 3 angegebenen Bedeutungen haben,
umsetzt, diese dann mit iso-Pentylnitrit und einem Iod-Äquivalent in eine Verbindung der Formel (V) 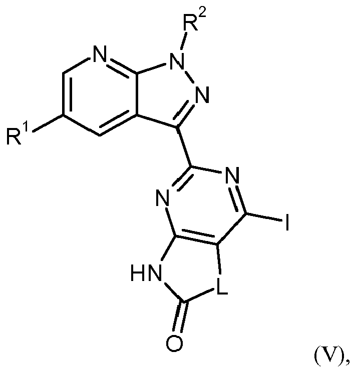 in welcher L, R¹ und R² jeweils die in den Ansprüchen 1 bis 3 angegebenen Bedeutungen haben,
überführt, und diese im Anschluss in einen inerten Lösungsmittel in Gegenwart eines geeigneten Übergangsmetallkatalysators zu einer Verbindung der Formel (I-A) 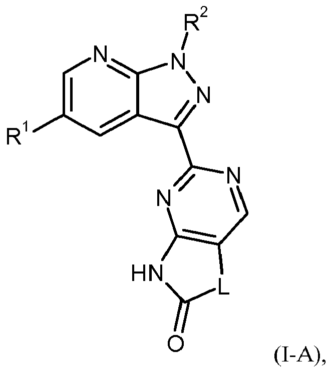 in welcher L, R¹ und R² jeweils die in den Ansprüchen 1 bis 3 angegebenen Bedeutungen haben,
umsetzt,
oder
[B] in einem inerten Lösungsmittel in Gegenwart einer geeigneten Base mit einer Verbindung der Formel (VI) 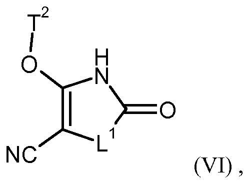 in welcher
L¹ für eine Gruppe *-CR^{4A}R^{4B}-(CR^{5A}R5^{B})ₚ-# steht,
wobei
* für die Anknüpfstelle an die Carbonylgruppe steht,
# für die Anknüpfstelle an den Pyrimidin- bzw. Triazinring steht,
p für eine Zahl 1 oder 2 steht,
R^{4A} für Wasserstoff, Fluor, (C₁-C₄)-Alkyl oder Hydroxy steht,
R^{4B} für Wasserstoff, Fluor, (C₁-C₄)-Alkyl oder Trifluormethyl steht,
oder
R^{4A} und R^{4B} zusammen mit dem Kohlenstoffatom, an das sie gebunden sind, eine Oxo-Gruppe, einen 3- bis 6-gliedrigen Carbocyclus oder einen 4- bis 6-gliedrigen Heterocyclus bilden,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
und
T² für (C₁-C₄)-Alkyl steht,
zu einer Verbindung der Formel (IV-B) 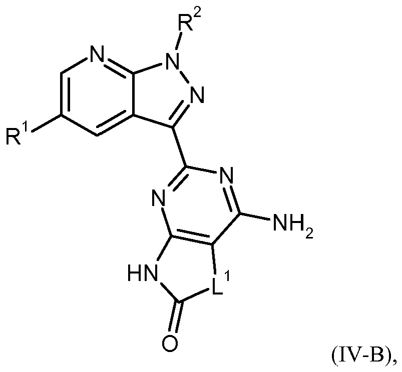 in welcher L¹, R¹ und R² jeweils die in den Ansprüchen 1 bis 3 angegebenen Bedeutungen haben,
umsetzt, und diese dann analog zum Verfahren [A] weiter zu einer Verbindung der Formel (I-B) 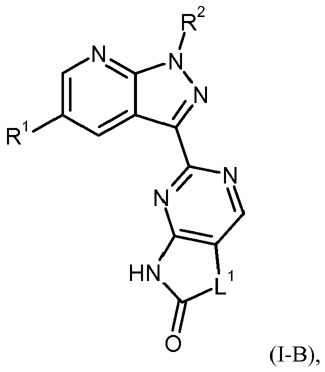 in welcher L¹, R¹ und R² jeweils die in den Ansprüchen 1 bis 3 angegebenen Bedeutungen haben,
umsetzt,
oder
[C] in einem inerten Lösungsmittel in Gegenwart einer geeigneten Base mit einer Verbindung der Formel (VII) 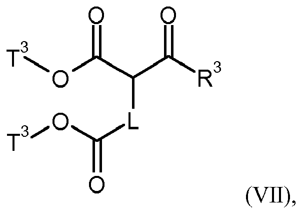 in welcher L und R³ jeweils die in den Ansprüchen 1 bis 3 angegebenen Bedeutungen haben und
T³ für (C₁-C₄)-Alkyl steht,
zu einer Verbindung der Formel (VIII) 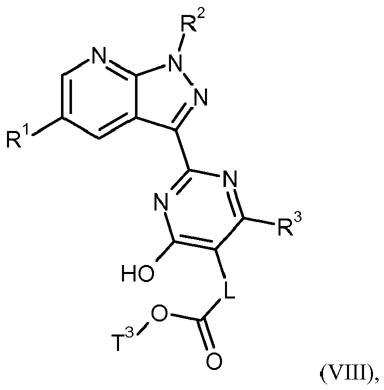 in welcher L, R¹, R², R³ und T³ jeweils die in den Ansprüchen 1 bis 3 angegebenen Bedeutungen haben,
umsetzt, diese anschliessend mit Phosphorylchlorid in eine Verbindung der Formel (IX) 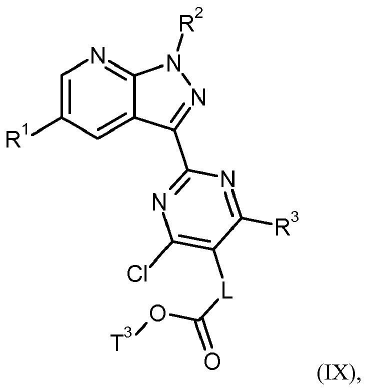 in welcher L, R¹, R², R³ und T³ jeweils die in den Ansprüchen 1 bis 3 angegebenen Bedeutungen haben,
überführt, diese im folgenden in einem inerten Lösungsmittel in eine entsprechende Azid-Verbindung überführt und diese direkt zu einer Verbindung der Formel (X) 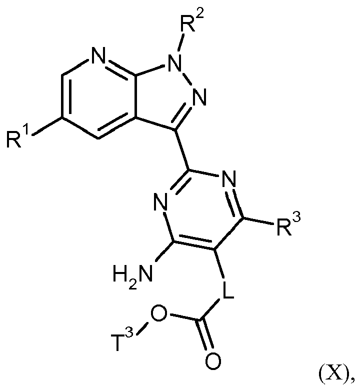 in welcher L, R¹, R², R³ und T³ jeweils die in den Ansprüchen 1 bis 3 angegebenen Bedeutungen haben,
reduziert, und diese dann in einem inerten Lösungsmittel in Gegenwart einer geeigneten Base zu einer Verbindung der Formel (I-C) 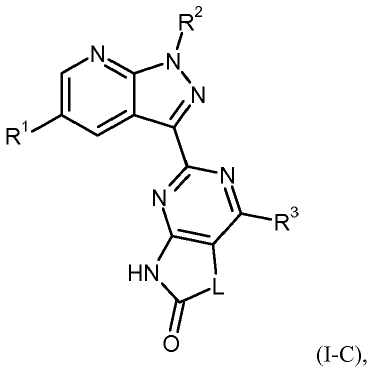 in welcher L, R¹, R² und R³ jeweils die in den Ansprüchen 1 bis 3 angegebenen Bedeutungen haben,
umsetzt,
und gegebenenfalls die resultierenden Verbindungen der Formeln (I-A), (I-B) und (I-C) gegebenenfalls mit den entsprechenden (i) Lösungsmitteln und/oder (ii) Säuren oder Basen in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

7. Verbindung der Formel (I), wie in einem der Ansprüche 1 bis 3 definiert, zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

11. Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose bei Menschen und Tieren unter Verwendung einer wirksamen Menge mindestens einer Verbindung der Formel (I), wie in einem der Ansprüche 1 bis 3 definiert, oder eines Arzneimittels, wie in einem der Ansprüche 8 bis 10 definiert.
